(19) Europäisches Patentamt / European Patent Office / Office européen des brevets

(11) EP 1 330 442 B1

(12) **EUROPEAN PATENT SPECIFICATION**

(45) Date of publication and mention of the grant of the patent:
**19.01.2011 Bulletin 2011/03**

(51) Int Cl.:
***C07D 237/32** (2006.01)* ***A61K 31/502** (2006.01)*
***A61K 31/50** (2006.01)* ***A61P 31/20** (2006.01)*

(21) Application number: **01976521.3**

(22) Date of filing: **25.10.2001**

(86) International application number:
**PCT/GB2001/004729**

(87) International publication number:
**WO 2002/036576 (10.05.2002 Gazette 2002/19)**

(54) **PHTHALAZINONE DERIVATIVES**

PHTALAZINON DERIVATE

DERIVES DE PHTALAZINONE

(84) Designated Contracting States:
**AT BE CH CY DE DK ES FI FR GB GR IE IT LI LU MC NL PT SE TR**

(30) Priority: **30.10.2000 GB 0026505**
**12.03.2001 US 275066 P**

(43) Date of publication of application:
**30.07.2003 Bulletin 2003/31**

(73) Proprietors:
- **Kudos Pharmaceuticals Limited**
  **2 Kingdom Street**
  **London W2 6BD (GB)**
- **Maybridge Limited**
  **Tintagel,**
  **Cornwall PL34 0HW (GB)**

(72) Inventors:
- **MARTIN, Niall, Morrison B.,**
  **c/o KuDOS Pharma. Ltd.**
  **Cambridge, Cambridgeshire CB4 4WG (GB)**
- **SMITH, Graeme, Cameron M.,**
  **c/o KuDOS Pharma. Ltd.**
  **Cambridge, Cambridgeshire CB4 4WG (GB)**
- **WHITE, Charles, Richard**
  **Carlisle, Cumbria CA3 9DF (GB)**
- **NEWTON, Roger, Frank,**
  **c/o Maybridge plc**
  **Tintagel, Cornwall PL34 0HW (GB)**
- **DOUGLAS, Diane, Gillian,**
  **"Niwen"**
  **Cornwall PL33 9DL (GB)**
- **EVERSLEY, Penny, Jane,**
  **c/o Maybridge plc**
  **Tintagel, Cornwall PL34 0HW (GB)**
- **VILE, Julia,**
  **c/o Maybridge plc**
  **Tintagel, Cornwall PL34 0HW (GB)**

(74) Representative: **Watson, Robert James et al**
**Mewburn Ellis LLP**
**33 Gutter Lane**
**London**
**EC2V 8AS (GB)**

(56) References cited:
**WO-A-98/43477**

- **CHEMICAL ABSTRACTS, vol. 125, no. 23, 1996 Columbus, Ohio, US; abstract no. 300924j, EL-TAMATY: "SYNTHESIS A. BIOLOGICAL ACTIVITY OF SOME 4-BENZYL-1 (2H)-PHTALAZINONE DERIVATIVES." page 1010; column 1; XP002186208 & INDIAN J. CHEM., SECT.B:ORG.CHEM. INCL. MED. CHEM., vol. 35, no. B10, 1996, pages 1067-1072,**
- **SHIMIZU,T.: "INHIBITORY EFFECTS OF AZELASTINE" PROSTAGLANDINS LEUKOTRIENS AND ESSENTIAL FATTY ACIDS, vol. 53, no. 5, 1995, pages 355-8, XP001042184 ENGL.**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 330 442 B1

## Description

The present invention relates to phthalazinone derivatives, and their use as pharmaceuticals. In particular, the present invention relates to the use of these compounds to inhibit the activity of the enzyme poly (ADP-ribose)polymerase, also known as poly(ADP-ribose)synthase and poly ADP-ribosyltransferase, and commonly referred to as PARP.

The mammalian enzyme PARP (a 113-kDa multidomain protein) has been implicated in the signalling of DNA damage through its ability to recognize and rapidly bind to DNA single or double strand breaks (D'Amours et al, 1999, Biochem. J. 342: 249-268).

Several observations have led to the conclusion that PARP participates in a variety of DNA-related functions including gene amplification, cell division, differentiation, apoptosis, DNA base excision repair and also effects on telomere length and chromosome stability (d'Adda di Fagagna et al, 1999, Nature Gen., 23(1 ): 76-80).

Studies on the mechanism by which PARP modulates DNA repair and other processes has identified its importance in the formation of poly (ADP-ribose) chains within the cellular nucleus (Althaus, F.R. and Richter, C., 1987, ADP-Ribosylation of Proteins: Enzymology and Biological Significance, Springer-Verlag, Berlin). The DNA-bound, activated PARP utilizes NAD to synthesize poly (ADP-ribose) on a variety of nuclear target proteins, including topoisomerase, histones and PARP itself (Rhun et al, 1998, Biochem. Biophys. Res. Commun., 245: 1-10)

Poly (ADP-ribosyl)ation has also been associated with malignant transformation. For example, PARP activity is higher in the isolated nuclei of SV40-transformed fibroblasts, while both leukemic cells and colon cancer cells show higher enzyme activity than the equivalent normal leukocytes and colon mucosa (Miwa et al, 1977, Arch. Biochem. Biophys. 181: 313-321; Burzio et al, 1975, Proc. Soc. Exp. Bioi. Med. 149: 933-938; and Hirai et al, 1983, Cancer Res. 43: 3441-3446).

A number of low-molecular-weight inhibitors of PARP have been used to elucidate the functional role of poly (ADP-ribosyl)ation in DNA repair. In cells treated with alkylating agents, the inhibition of PARP leads to a marked increase in DNA-strand breakage and cell killing (Durkacz et al, 1980, Nature 283: 593-596; Berger, N.A., 1985, Radiation Research, 101: 4-14).

Subsequently, such inhibitors have been shown to enhance the effects of radiation response by suppressing the repair of potentially lethal damage (Ben-Hur et al, 1984, British Journal of Cancer, 49 (Suppl. VI): 34-42; Schlicker et al, 1999, Int. J. Radiat. Bioi., 75: 91-100). PARP inhibitors have been reported to be effective in radio sensitising hypoxic tumour cells (US 5,032,617; US 5,215,738 and US 5,041,653).

Furthermore, PARP knockout (PARP -/-) animals exhibit genomic instability in response to alkylating agents and γ-irradiation (Wang et al, 1995, Genes Dev., 9: 509-520; Menissier de Murcia et al, 1997, Proc. Natl. Acad. Sci. USA, 94: 7303-7307).

A role for PARP has also been demonstrated in certain vascular diseases, septic shock, ischaemic injury and neurotoxicity (Cantoni et al, 1989, Biochim. Biophys. Acta, 1014: 1-7; Szabo, et al, 1997, J. Clin.Invest., 100: 723-735). Oxygen radical DNA damage that leads to strand breaks in DNA, which are subsequently recognised by PARP, is a major contributing factor to such disease states as shown by PARP inhibitor studies (Cosi et al, 1994, J. Neurosci. Res., 39: 38-46; Said et al, 1996, Proc. Natl. Acad. Sci. U.S.A., 93: 4688-4692). More recently, PARP has been demonstrated to play a role in the pathogenesis of haemorrhagic shock (Liaudet et al, 2000, Proc. Natl. Acad. Sci. U.S.A., 97(3): 10203-10208).

It has also been demonstrated that efficient retroviral infection of mammalian cells is blocked by the inhibition of PARP activity. Such inhibition of recombinant retroviral vector infections was shown to occur in various different cell types (Gaken et al, 1996, J. Virology, 70(6): 3992-4000). Inhibitors of PARP have thus been developed for the use in anti-viral therapies and in cancer treatment (WO91/18591).

Moreover, PARP inhibition has been speculated to delay the onset of aging characteristics in human fibroblasts (Rattan and Clark, 1994, Biochem. Biophys. Res. Comm., 201 (2): 665-672). This may be related to the role that PARP plays in controlling telomere function (d'Adda di Fagagna et al, 1999, Nature Gen., 23(1 ): 76-80).

US 5,874,444 discloses a number of PARP inhibitors, amongst which is 1(2*H*)-phthalazinone **(100):**

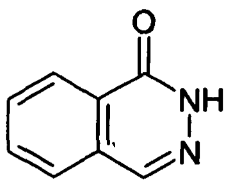

(100)

The present inventors have now discovered that certain derivatives of 1(2*H*)-phthalazinone and related compounds exhibit inhibition of the activity of PARP.

Accordingly, the first aspect of the present invention provides for the use of compounds of the formula:

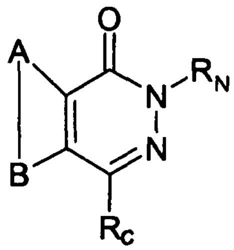

or isomers, salts, solvates and chemically protected forms thereof, in the preparation of a medicament for inhibiting the activity of PARP, wherein:

A and B together represent a fused aromatic ring, optionally substituted with one or more substituent groups selected from halo, nitro, hydroxy, ether, thiol, thioether, amino, $C_{1-7}$ alkyl, $C_{3-20}$ heterocyclyl and $C_{5-20}$ aryl;
$R_C$ is $-CH_2-R_L$, where $R_L$ is a $C_{5-20}$ aryl group, optionally substituted with one or more substituent groups selected from $C_{1-7}$ alkyl, $C_{5-20}$ aryl, $C_{3-20}$ heterocyclyl, halo, hydroxy, ether, nitro, cyano, acyl, carboxy, ester, amido, amino, sulfonamido, acylamido, ureido, acyloxy, thiol, thioether, sulfoxide and sulfone; and
$R_N$ is hydrogen.

In particular, the first aspect relates to the preparation of a medicament for the treatment of: vascular disease; septic shock; ischaemic injury; neurotoxicity; haemorraghic shock; viral infection; or diseases ameliorated by the inhibition of the activity of PARP.

A second aspect of the present invention relates to the use of a compound as described in the first aspect of the invention in the preparation of a medicament for use as an adjunct in cancer therapy or for potentiating tumour cells for treatment with ionizing radiation or chemotherapeutic agents.

A third aspect of the present invention relates to a pharmaceutical composition comprising a compound of the formula:

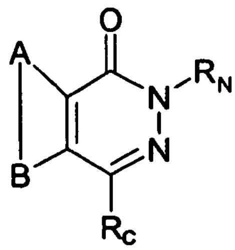

or isomers, salts, solvates and chemically protected forms thereof, wherein:

A and B together represent a fused aromatic ring, optionally substituted with one or more substituent groups selected from halo, nitro, hydroxy, ether, thiol, thioether, amino, $C_{1-7}$ alkyl, $C_{3-20}$ heterocyclyl and $C_{5-20}$ aryl;
$R_C$ is $-CH_2-R_L$;
$R_L$ is phenyl optionally substituted with one or more substituent groups selected from $C_{1-7}$ alkyl, $C_{5-20}$ aryl, $C_{3-20}$ heterocyclyl, halo, hydroxy, ether, nitro, cyano, carboxy, ester, amido, amino, sulfonamido, acylamido, ureido, acyloxy, thiol, thioether, sulfoxide and sulfone; and $R_N$ is hydrogen,
and a pharmaceutically acceptable carrier or diluent.

A fourth aspect of the present invention relates to the use of a compound as described in the third aspect in a method of treatment of the human or animal body.

A fifth aspect of the present invention relates to a compound of the formula:

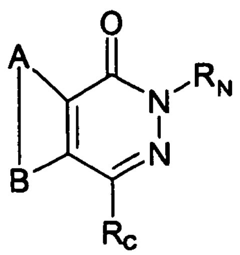

or an isomers, salt, solvate and chemically protected forms thereof, wherein:

A and B together represent a fused aromatic ring, optionally substituted with one substituent group selected from halo, nitro, hydroxy, ether, thiol, thioether, amino, $C_{1-7}$ alkyl, $C_{3-20}$ heterocyclyl and $C_{5-20}$ aryl;
$R_C$ is $-CH_2-R_L$;
$R_L$ is phenyl substituted by a substituent selected from the group consisting of: $C_{3-20}$ heterocyclyl; amido; ureido; sulfonamido; and acyloxy and optionally further substituted by a group selected from $C_{1-7}$ alkyl, $C_{5-20}$ aryl, $C_{3-20}$ heterocyclyl, halo, hydroxy, ether, nitro, cyano, carboxy, ester, amido, amino, sulfonamide, acylamido, ureido, acyloxy, thiol, thioether, sulfoxide and sulfone; and $R_N$ is hydrogen.

Brief Description of the Drawings

Figures 1 to 18 show compounds according to the present invention.

***Definitions***

The term "aromatic ring" is used herein in the conventional sense to refer to a cyclic aromatic structure, that is, a cyclic structure having delocalised $\pi$-electron orbitals.

The aromatic ring fused to the main core, i.e. that formed by -A-B-, may bear further fused aromatic rings (resulting in, e.g. naphthyl or anthracenyl groups). The aromatic ring(s) may comprise solely carbon atoms, or may comprise carbon atoms and one or more heteroatoms, including but not limited to, nitrogen, oxygen, and sulfur atoms. The aromatic ring(s) preferably have five or six ring atoms.

The aromatic ring(s) may optionally be substituted. If a substituent itself comprises an aryl group, this aryl group is not considered to be a part of the aryl group to which it is attached. For example, the group biphenyl is considered herein to be a phenyl group (an aryl group comprising a single aromatic ring) substituted with a phenyl group. Similarly, the group benzylphenyl is considered to be a phenyl group (an aryl group comprising a single aromatic ring) substituted with a benzyl group.

In one group of preferred embodiments, the aromatic group comprises a single aromatic ring, which has five or six ring atoms, which ring atoms are selected from carbon, nitrogen, oxygen, and sulfur, and which ring is optionally substituted. Examples of these groups include benzene, pyrazine, pyrrole, thiazole, isoxazole, and oxazole. 2-Pyrone can also be considered to be an aromatic ring, but is less preferred.

If the aromatic ring has six atoms, then preferably at least four, or even five or all, of the ring atoms are carbon. The other ring atoms are selected from nitrogen, oxygen and sulphur, with nitrogen and oxygen being preferred. Suitable groups include a ring with: no hetero atoms (benzene); one nitrogen ring atom (pyridine); two nitrogen ring atoms (pyrazine, pyrimidine and pyridazine); one oxygen ring atom (pyrone); and one oxygen and one nitrogen ring atom (oxazine).

If the aromatic ring has five ring atoms, then preferably at least three of the ring atoms are carbon. The remaining ring atoms are selected from nitrogen, oxygen and sulphur. Suitable rings include a ring with: one nitrogen ring atom (pyrrole); two nitrogen ring atoms (imidazole, pyrazole); one oxygen ring atom (furan); one sulphur ring atom (thiophene); one nitrogen and one sulphur ring atom

(thiazole); and one nitrogen and one oxygen ring atom (isoxazole or oxazole).

The aromatic ring may bear one or more substituent groups at any available ring position. These substituents are selected from halo, nitro, hydroxy, ether, thiol, thioether, amino, $C_{1-7}$ alkyl, $C_{3-20}$ heterocyclyl and $C_{5-20}$ aryl. The aromatic ring may also bear one or more substituent groups which together form a ring. In particular these may be of formula $-(CH_2)_m-$ or $-O-(CH_2)_p-O-$, where m is 2, 3, 4 or 5 and p is 1, 2 or 3.

$C_{1-7}$ alkyl: The term "$C_{1-7}$ alkyl" as used herein, pertains to a monovalent moiety obtained by removing a hydrogen atom from a $C_{1-7}$hydrocarbon compound having from 1 to 7 carbon atoms, which may be aliphatic or alicyclic,

or a combination thereof, and which may be saturated, partially unsaturated, or fully unsaturated.

Examples of (unsubstituted) saturated linear $C_{1-7}$ alkyl groups include, but are not limited to, methyl, ethyl, *n*-propyl, *n*-butyl, and *n*-pentyl (amyl).

Examples of (unsubstituted) saturated branched $C_{1-7}$ alkyl groups include, but are not limited to, *iso*-propyl, *iso*-butyl, *sec*-butyl, *tert*-butyl, and *neo*-pentyl.

Examples of saturated alicyclic (carbocyclic) $C_{1-7}$ alkyl groups (also referred to as "$C_{3-7}$ cycloalkyl" groups) include, but are not limited to, unsubstituted groups such as cyclopropyl, cyclobutyl, cyclopentyl, and cyclohexyl, as well as substituted groups (e.g., groups which comprise such groups), such as methylcyclopropyl, dimethylcyclopropyl, methylcyclobutyl, dimethylcyclobutyl, methylcyclopentyl, dimethylcyclopentyl, methylcyclohexyl, dimethylcyclohexyl, cyclopropylmethyl and cyclohexylmethyl.

Examples of (unsubstituted) unsaturated $C_{1-7}$ alkyl groups which have one or more carbon-carbon double bonds (also referred to as "$C_{2-7}$ alkenyl" groups) include, but are not limited to, ethenyl (vinyl, $-CH=CH_2$), 2-propenyl (allyl, $-CH_2-CH=CH_2$), isopropenyl ($-C(CH_3)=CH_2$), butenyl, pentenyl, and hexenyl.

Examples of (unsubstituted) unsaturated $C_{1-7}$ alkyl groups which have one or more carbon-carbon triple bonds (also referred to as "$C_{2-7}$ alkynyl" groups) include, but are not limited to, ethynyl (ethinyl) and 2-propynyl (propargyl).

Examples of unsaturated alicyclic (carbocyclic) $C_{1-7}$ alkyl groups which have one or more carbon-carbon double bonds (also referred to as "$C_{3-7}$ cycloalkenyl" groups) include, but are not limited to, unsubstituted groups such as cyclopropenyl, cyclobutenyl, cyclopentenyl, and cyclohexenyl, as well as substituted groups (e.g., groups which comprise such groups) such as cyclopropenylmethyl and cyclohexenylmethyl.

$C_{3-20}$ heterocyclyl: The term "$C_{3-20}$ heterocyclyl" as used herein, pertains to a monovalent moiety obtained by removing a hydrogen atom from a ring atom of a non-aromatic $C_{3-20}$ heterocyclic compound, said compound having one ring, or two or more rings (e.g., spiro, fused, bridged), and having from 3 to 20 ring atoms, atoms, of which from 1 to 10 are ring heteroatoms, and wherein at least one of said ring(s) is a heterocyclic ring. Preferably, each ring has from 3 to 7 ring atoms, of which from 1 to 4 are ring heteroatoms.

"$C_{3-20}$" denotes ring atoms, whether carbon atoms or heteroatoms.

Examples of $C_{3-20}$ heterocyclyl groups having one nitrogen ring atom include, but are not limited to, those derived from aziridine, azetidine, azetine, pyrrolidine, pyrroline, piperidine, dihydropyridine, tetrahydropyridine, and dihydropyrrole (azoline).

Examples of $C_{3-20}$ heterocyclyl groups having one oxygen ring atom include, but are not limited to, those derived from oxirane, oxetane, oxolane (tetrahydrofuran), oxole (dihydrofuran), oxane (tetrahydropyran), dihydropyran, and pyran. Examples of substituted $C_{3-20}$ heterocyclyl groups include sugars, in cyclic form, for example, furanoses and pyranoses, including, for example, ribose, lyxose, xylose, galactose, sucrose, fructose, and arabinose.

Examples of $C_{3-20}$ heterocyclyl groups having one sulfur ring atom include, but are not limited to, those derived from thiolane (tetrahydrothiophene, thiane) and tetrahydrothiopyran.

Examples of $C_{3-20}$ heterocyclyl groups having two oxygen ring atoms include, but are not limited to, those derived from dioxane, for example 1,3-dioxane and 1,4-dioxane.

Examples of $C_{3-20}$ heterocyclyl groups having two nitrogen ring atoms include, but are not limited to, those derived from diazolidine (pyrazolidine), pyrazoline, imidazolidine, imidazoline, and piperazine.

Examples of $C_{3-20}$ heterocyclyl groups having one nitrogen ring atom and one oxygen ring atom include, but are not limited to, those derived from tetrahydrooxazole, dihydrooxazole, tetrahydroisoxazole, dihydroiosoxazole, morpholine, tetrahydrooxazine, dihydrooxazine, and oxazine.

Examples of $C_{3-20}$ heterocyclyl groups having one oxygen ring atom and one sulfur ring atom include, but are not limited to, those derived from oxathiolane and oxathiane.

Examples of $C_{3-20}$ heterocyclyl groups having one nitrogen ring atom and one sulfur ring atom include, but are not limited to, those derived from thiazoline, thiazolidine, and thiomorpholine.

Other examples of $C_{3-20}$ heterocyclyl groups include, but are not limited to, oxadiazine.

If the $C_{3-20}$ heterocyclyl is substituted, the substituents are on carbon, or nitrogen (if present), atoms.

$C_{5-20}$ aryl: The term "$C_{5-20}$ aryl" as used herein, pertains to a monovalent moiety obtained by removing a hydrogen atom from an aromatic ring atom of a $C_{5-20}$ aromatic compound, said compound having one ring, or two or more rings (e.g., fused), and having from 5 to 20 ring atoms, and wherein at least one of said ring(s) is an aromatic ring. Preferably, each ring has from 5 to 7 ring atoms.

The ring atoms may be all carbon atoms, as in "carboaryl groups" in which case the group may conveniently be referred to as a "$C_{5-20}$ carboaryl" group.

Examples of $C_{5-20}$ aryl groups which do not have ring heteroatoms (i.e., $C_{5-20}$ carboaryl groups) include, but are not limited to, those derived from benzene (i.e., phenyl)

($C_6$), naphthalene ($C_{10}$), anthracene ($C_{14}$), phenanthrene ($C_{14}$), and pyrene ($C_{16}$).

Alternatively, the ring atoms may include one or more heteroatoms, including but not limited to oxygen, nitrogen, and sulfur, as in "heteroaryl groups". In this case, the group may conveniently be referred to as a "$C_{5-20}$ heteroaryl"

group, wherein "$C_{5-20}$" denotes ring atoms, whether carbon atoms or heteroatoms. Preferably, each ring has from 5 to 7 ring atoms, of which from 0 to 4 are ring heteroatoms.

Examples of $C_{5-20}$ heteroaryl groups include, but are not limited to, $C_5$ heteroaryl groups derived from furan (oxole), thiophene (thiole), pyrrole (azole), imidazole (1,3-diazole), pyrazole (1,2-diazole), triazole, oxazole, isoxazole, thiazole, isothiazole, oxadiazole, and oxatriazole; and $C_6$ heteroaryl groups derived from isoxazine, pyridine (azine), pyridazine (1,2-diazine), pyrimidine (1,3-diazine; e.g., cytosine, thymine, uracil), pyrazine (1,4-diazine), triazine, tetrazole, and oxadiazole (furazan).

The heteroaryl group may be bonded via a carbon or hetero ring atom.

Examples of $C_{5-20}$ heteroaryl groups which comprise fused rings, include, but are not limited to, $C_9$ heteroaryl groups derived from benzofuran, isobenzofuran, benzothiophene, indole, isoindole; $C_{10}$ heteroaryl groups derived from quinoline, isoquinoline, benzodiazine, pyridopyridine; $C_{14}$ heteroaryl groups derived from acridine and xanthene.

The above $C_{1-7}$ alkyl, $C_{3-20}$ heterocyclyl, and $C_{5-20}$ aryl groups, whether alone or part of another substituent, may themselves optionally be substituted with one or more groups selected from themselves and the additional substituents listed below

Halo: -F, -Cl, -Br, and -I.

Hydroxy: -OH.

Ether: -OR, wherein R is an ether substituent, for example, a $C_{1-7}$ alkyl group (also referred to as a $C_{1-7}$ alkoxy group), a $C_{3-20}$ heterocyclyl group (also referred to as a $C_{3-20}$ heterocyclyloxy group), or a $C_{5-20}$ aryl group (also referred to as a $C_{5-20}$ aryloxy group), preferably a $C_{1-7}$ alkyl group.

Nitro: $-NO_2$.

Cyano (nitrile, carbonitrile): -CN.

Acyl (keto): -C(=O)R, wherein R is an acyl substituent, for example, a $C_{1-7}$ alkyl group (also referred to as $C_{1-7}$ alkylacyl or $C_{1-7}$ alkanoyl), a $C_{3-20}$ heterocyclyl group (also referred to as $C_{3-20}$ heterocyclylacyl), or a $C_{5-20}$ aryl group (also referred to as $C_{5-20}$ arylacyl), preferably a $C_{1-7}$ alkyl group. Examples of acyl groups include, but are not limited to, $-C(=O)CH_3$ (acetyl), $-C(=O)CH_2CH_3$ (propionyl), $-C(=O)C(CH_3)_3$ (butyryl), and -C(=O)Ph (benzoyl, phenone).

Carboxy (carboxylic acid): -COOH.

Ester (carboxylate, carboxylic acid ester, oxycarbonyl): -C(=O)OR, wherein R is an ester substituent, for example, a $C_{1-7}$ alkyl group, a $C_{3-20}$ heterocyclyl group, or a $C_{5-20}$ aryl group, preferably a $C_{1-7}$ alkyl group. Examples of ester groups include, but are not limited to, $-C(=O)OCH_3$, $-C(=O)OCH_2CH_3$, $-C(=O)OC(CH_3)_3$, and -C(=O)OPh.

Amido (carbamoyl, carbamyl, aminocarbonyl, carboxamide): $-C(=O)NR^1R^2$, wherein $R^1$ and $R^2$ are independently amino substituents, as defined for amino groups. Examples of amido groups include, but are not limited to, $-C(=O)NH_2$, $-C(=O)NHCH_3$, $-C(=O)N(CH_3)_2$, $-C(=O)NHCH_2CH_3$, and $-C(=O)N(CH_2CH_3)_2$, as well as amido groups in which $R^1$ and $R^2$, together with the nitrogen atom to which they are attached, form a heterocyclic structure as in, for example, piperidinocarbonyl, morpholinocarbonyl, thiomorpholinocarbonyl, and piperazinocarbonyl.

Amino: $-NR^1R^2$, wherein $R^1$ and $R^2$ are independently amino substituents, for example, hydrogen, a $C_{1-7}$ alkyl group (also referred to as $C_{1-7}$ alkylamino or di-$C_{1-7}$ alkylamino), a $C_{3-20}$ heterocyclyl group, or a $C_{5-20}$ aryl group, preferably H or a $C_{1-7}$ alkyl group, or, in the case of a "cyclic" amino group, $R^1$ and $R^2$, taken together with the nitrogen atom to which they are attached, form a heterocyclic ring having from 4 to 8 ring atoms. Examples of amino groups include, but are not limited to, $-NH_2$, $-NHCH_3$, $-NHCH(CH_3)_2$, $-N(CH_3)_2$, $-N(CH_2CH_3)_2$, and -NHPh. Examples of cyclic amino groups include, but are not limited to, aziridino, azetidino, pyrrolidino, piperidino, piperazino, perhydrodiazepino, morpholino, and thiomorpholino. The cylic amino groups may be substituted on their ring by any of the substituents defined here, for example carboxy, carboxylate and amido. A particular form of amino group is where one of $R^1$ and $R^2$ is a sulfone ($-S(=O)_2R$), where R is a sulfone substituent, and this group can be termed a sulfonamido group. Examples of sulfonamido groups include, but are not limited to, $-NHS(=O)_2CH_3$, $-NHS(=O)_2Ph$ and $-NHS(=O)_2C_6H_4F$.

Acylamido (acylamino): $-NR^1C(=O)R^2$, wherein $R^1$ is an amide substituent, for example, hydrogen, a $C_{1-7}$ alkyl group, a $C_{3-20}$ heterocyclyl group, or a $C_{5-20}$ aryl group, preferably H or a $C_{1-7}$ alkyl group, most preferably H, and $R^2$ is an acyl substituent, for example, a $C_{1-7}$ alkyl group, a $C_{3-20}$ heterocyclyl group, or a $C_{5-20}$ aryl group, preferably a $C_{1-7}$ alkyl group. Examples of acylamide groups include, but are not limited to, $-NHC(=O)CH_3$ , $-NHC(=O)CH_2CH_3$, and -NHC(=O)Ph.

One particular form of acylamido group is where $R^2$ is an amino group ($-NR^3R^4$), where $R^3$ and $R^4$ are independently amino substituents, as this group can be termed an ureido group. Example of ureido groups include, but are not limited to $-NHC(=O)NHCH_3$, $-NHC(=O)NHCH_2CH_3$, and -NHC(=O)NHPh.

Acyloxy (reverse ester): -OC(=O)R, wherein R is an acyloxy substituent, for example, a $C_{1-7}$ alkyl group, a $C_{3-20}$ heterocyclyl group, or a $C_{5-20}$ aryl group, preferably a $C_{1-7}$ alkyl group. Examples of acyloxy groups include, but are not limited to, $-OC(=O)CH_3$ (acetoxy), $-OC(=O)CH_2CH_3$, $-OC(=O)C(CH_3)_3$, -OC(=O)Ph, $-OC(=O)C_6H_4F$, and $-OC(=O)CH_2Ph$.

Thiol : -SH.

Thioether (sulfide): -SR, wherein R is a thioether substituent, for example, a $C_{1-7}$ alkyl group (also referred to

as a $C_{1-7}$ alkylthio group), a $C_{3-20}$ heterocyclyl group, or a $C_{5-20}$ aryl group, preferably a $C_{1-7}$ alkyl group. Examples of $C_{1-7}$ alkylthio groups include, but are not limited to, $-SCH_3$ and $-SCH_2CH_3$.

Sulfoxide (sulfinyl): -S(=O)R, wherein R is a sulfoxide substituent, for example, a $C_{1-7}$ alkyl group, a $C_{3-20}$ heterocyclyl group, or a $C_{5-20}$ aryl group, preferably a $C_{1-7}$ alkyl group. Examples of sulfoxide groups include, but are not limited to, $-S(=O)CH_3$ and $-S(=O)CH_2CH_3$.

Sulfone (sulfonyl): $-S(=O)_2R$, wherein R is a sulfone substituent, for example, a $C_{1-7}$ alkyl group, a $C_{3-20}$ heterocyclyl group, or a $C_{5-20}$ aryl group, preferably a $C_{1-7}$ alkyl group. Examples of sulfone groups include, but are not limited to, $-S(=O)_2CH_3$ (methanesulfonyl, mesyl), $-S(=O)_2CF_3$, $-S(=O)_2CH_2CH_3$, and 4-methylphenylsulfonyl (tosyl).

As mentioned above, the groups that form the above listed substituent groups, e.g. $C_{1-7}$ alkyl, $C_{3-20}$ heterocyclyl and $C_{5-20}$ aryl, may themselves be substituted. Thus, the above definitions cover substituent groups which are substituted.

Further Preferences

The following preferences can apply to each aspect of the present invention, where applicable.

In the present invention, the fused aromatic ring(s) represented by -A-B- preferably consist of solely carbon ring atoms, and thus may be benzene, naphthalene, and is more preferably benzene. As described above, these rings may be substituted, but in some embodiments are preferably unsubstituted.

$R_N$ is preferably selected from hydrogen, and amido. In one embodiment, $R_N$ is preferably amido, where one amido substituent is phenyl, optionally substituted by fluorine, preferably in the para position. In another embodiment, $R_N$ is preferably H.

$R_L$ is preferably a benzene ring, naphthalene, pyridine or 1,3-benzodioxole, and more preferably a benzene ring.

When $R_L$ is a benzene ring, it is preferably substituted. The one or more substituents may be selected from: $C_{1-7}$ alkyl, more preferably methyl, $CF_3$; $C_{5-20}$ aryl; $C_{3-20}$ heterocyclyl; halo, more preferably fluoro; hydroxy; ether, more preferably methoxy, phenoxy, benzyloxy, and cyclopentoxy; nitro; cyano; carbonyl groups, such as carboxy, ester and amido; amino (including sulfonamido), more preferably $-NH_2$, -NHPh, and cycloamino groups, such as morpholino; acylamido, including ureido groups, where the acyl or amino substituent is preferably phenyl, which itself is optionally fluorinated; acyloxy; thiol; thioether; sulfoxide; sulfone.

In one group of embodiments, fluoro is particularly preferred as a substituent, along with substituents containing a phenyl, or fluorinated phenyl, component.

Preferred substituents of the benzene ring, when $R_L$ is phenyl, include:

(i) acylamido, wherein the amide substituent is selected from $C_{1-7}$ alkyl, $C_{3-20}$ heterocyclyl, and $C_{5-20}$ aryl, more preferably $C_{1-7}$ alkyl and $C_{5-20}$ aryl, which groups are optionally further substituted. The optional substituents may be selected from any of those listed above, but those of particular interest include $C_{1-7}$ alkyl and $C_{5-20}$ aryl groups, halo, ether, thioether and sulfone groups;

(ii) ureido, where one amine substituent is preferably hydrogen, and the other is selected from $C_{1-7}$ alkyl, $C_{3-20}$ heterocyclyl, and $C_{5-20}$ aryl, more preferably $C_{1-7}$ alkyl and $C_{5-20}$ aryl, which groups are optionally further substituted. The optional substituents may be selected from any of those listed above, but those of particular interest include $C_{1-7}$ alkyl, $C_{3-20}$ heterocyclyl and $C_{5-20}$ aryl groups, halo and ether groups;

(iii)sulfonamino, wherein the amine substituent is preferably hydrogen and the sulfone substituent is selected from $C_{1-7}$ alkyl, $C_{3-20}$ heterocyclyl, and $C_{5-20}$ aryl, more preferably $C_{1-7}$ alkyl and $C_{5-20}$ aryl, which groups are optionally further substituted. The optional substituents may be selected from any of those listed above, but those of particular interest include $C_{5-20}$ aryl groups and acylamido groups;

(iv) acyloxy, wherein the acyloxy substituent is selected from $C_{1-7}$ alkyl, $C_{3-20}$ heterocyclyl, and $C_{5-20}$ aryl, more preferably $C_{1-7}$ alkyl and $C_{5-20}$ aryl, which groups are optionally further substituted. The optional substituents may be selected from any of those listed above, but those of particular interest include $C_{1-7}$ alkyl and $C_{5-20}$ aryl groups, halo, ether, thioether, sulfone and nitro groups.

If A and B together represent a substituted fused aromatic ring, substituents in the five position are particularly preferred.

Where appropriate, the above preferences may be taken in combination with each other.

Includes Other Forms

Included in the above are the well known ionic, salt, solvate, and protected forms of these substituents. For example, a reference to carboxylic acid (-COOH) also includes the anionic (carboxylate) form ($-COO^-$), a salt or solvate thereof, as well as conventional protected forms. Similarly, a reference to an amino group includes the protonated form ($-N^+HR^1R^2$), a salt or solvate of the amino group, for example, a hydrochloride salt, as well as conventional protected

forms of an amino group. Similarly, a reference to a hydroxyl group also includes the anionic form ($-O^-$), a salt or solvate thereof, as well as conventional protected forms of a hydroxyl group.

Isomers, Salts, Solvates and Protected Forms

Certain compounds may exist in one or more particular geometric, optical, enantiomeric, diasteriomeric, epimeric, stereoisomeric, tautomeric, conformational, or anomeric forms, including but not limited to, *cis*- and *trans*-forms; *E*- and *Z*-forms; *c*-, *t*-, and *r*-forms; *endo*- and *exo*-forms; *R*-, *S*-, and *meso*-forms; *D*- and *L*-forms; *d*- and *l*-forms; (+) and (-) forms; keto-, enol-, and enolate-forms; syn- and anti-forms; synclinal- and anticlinal-forms; α- and β-forms; axial and equatorial forms; boat-; chair-, twist-, envelope-, and halfchair-forms; and combinations thereof, hereinafter collectively referred to as "isomers" (or "isomeric forms").

If the compound is in crystalline form, it may exist in a number of different polymorphic forms.

Note that, except as discussed below for tautomeric forms, specifically excluded from the term "isomers", as used herein, are structural (or constitutional) isomers (i.e. isomers which differ in the connections between atoms rather than merely by the position of atoms in space). For example, a reference to a methoxy group, $-OCH_3$, is not to be construed as a reference to its structural isomer, a hydroxymethyl group, $-CH_2OH$. Similarly, a reference to ortho-chlorophenyl is not to be construed as a reference to its structural isomer, meta-chlorophenyl. However, a reference to a class of structures may well include structurally isomeric forms falling within that class (e.g., $C_{1-7}$ alkyl includes *n*-propyl and *iso*-propyl; butyl includes *n-, iso-, sec*-, and *tert*-butyl; methoxyphenyl includes *ortho-, meta-,* and para-methoxyphenyl).

The above exclusion does not pertain to tautomeric forms, for example, keto-, enol-, and enolate-forms, as in, for example, the following tautomeric pairs: keto/enol, imine/enamine, amide/imino alcohol, amidine/amidine, nitroso/oxime, thioketone/enethiol, *N*-nitroso/hyroxyazo, and nitro/aci-nitro.

Particularly relevant to the present invention is the tautomeric pair that exists when $R_N$ is H, illustrated below:

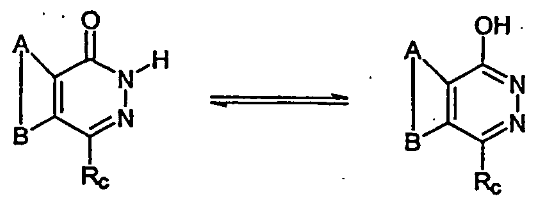

Note that specifically included in the term "isomer" are compounds with one or more isotopic substitutions. For example, H may be in any isotopic form, including $^1H$, $^2H$ (D), and $^3H$ (T); C may be in any isotopic form, including $^{12}C$, $^{13}C$, and $^{14}C$; O may be in any isotopic form, including $^{16}O$ and $^{18}O$; and the like.

Unless otherwise specified, a reference to a particular compound includes all such isomeric forms, including (wholly or partially) racemic and other mixtures thereof. Methods for the preparation (e.g. asymmetric synthesis) and separation (e.g. fractional crystallisation and chromatographic means) of such isomeric forms are either known in the art or are readily obtained by adapting the methods taught herein, or known methods, in a known manner.

Unless otherwise specified, a reference to a particular compound also includes ionic, salt, solvate, and protected forms of thereof, for example, as discussed below, as well as its different polymorphic forms.

It may be convenient or desirable to prepare, purify, and/or handle a corresponding salt of the active compound, for example, a pharmaceutically-acceptable salt. Examples of pharmaceutically acceptable salts are discussed in Berge et al., 1977, "Pharmaceutically Acceptable Salts," J. Pharm. Sci., Vol. 66, pp. 1-19.

For example, if the compound is anionic, or has a functional group which may be anionic (e.g., $-COOH$ may be $-COO^-$), then a salt may be formed with a suitable cation. Examples of suitable inorganic cations include, but are not limited to, alkali metal ions such as $Na^+$ and $K^+$, alkaline earth cations such as $Ca^{2+}$ and $Mg^{2+}$, and other cations such as $Al^{3+}$. Examples of suitable organic cations include, but are not limited to, ammonium ion (i.e., $NH_4^+$) and substituted ammonium ions (e.g., $NH_3R^+$, $NH_2R_2^+$, $NHR_3^+$, $NR_4^+$). Examples of some suitable substituted ammonium ions are those derived from: ethylamine, diethylamine, dicyclohexylamine, triethylamine, butylamine, ethylenediamine, ethanolamine, diethanolamine, piperazine, benzylamine, phenylbenzylamine, choline, meglumine, and tromethamine, as well as amino acids, such as lysine and arginine. An example of a common quaternary ammonium ion is $N(CH_3)_4^+$.

If the compound is cationic, or has a functional group which may be cationic (e.g., $-NH_2$ may be $-NH_3^+$), then a salt may be formed with a suitable anion. Examples of suitable inorganic anions include, but are not limited to, those derived from the following inorganic acids: hydrochloric, hydrobromic, hydroiodic, sulfuric, sulfurous, nitric, nitrous, phosphoric, and phosphorous. Examples of suitable organic anions include, but are not limited to, those derived from the

following organic acids: acetic, propionic, succinic, gycolic, stearic, palmitic, lactic, malic, pamoic, tartaric, citric, gluconic, ascorbic, maleic, hydroxymaleic, phenylacetic, glutamic, aspartic, benzoic, cinnamic, pyruvic, salicyclic, sulfanilic, 2-acetyoxybenzoic, fumaric, toluenesulfonic, methanesulfonic, ethanesulfonic, ethane disulfonic, oxalic, isethionic, valeric, and gluconic. Examples of suitable polymeric anions include, but are not limited to, those derived from the following polymeric acids: tannic acid, carboxymethyl cellulose.

It may be convenient or desirable to prepare, purify, and/or handle a corresponding solvate of the active compound. The term "solvate" is used herein in the conventional sense to refer to a complex of solute (e.g., active compound, salt of active compound) and solvent. If the solvent is water, the solvate may be conveniently referred to as a hydrate, for example, a mono-hydrate, a di-hydrate, a tri-hydrate, etc. It may be convenient or desirable to prepare, purify, and/or handle the active compound in a chemically protected form. The term "chemically protected form," as used herein, pertains to a compound in which one or more reactive functional groups are protected from undesirable chemical reactions, that is, are in the form of a protected or protecting group (also known as a masked or masking group or a blocked or blocking group). By protecting a reactive functional group, reactions involving other unprotected reactive functional groups can be performed, without affecting the protected group; the protecting group may be removed, usually in a subsequent step, without substantially affecting the remainder of the molecule. See, for example, Protective Groups in Organic Synthesis (T. Green and P. Wuts, Wiley, 1991).

For example, a hydroxy group may be protected as an ether (-OR) or an ester (-OC(=O)R), for example, as: a t-butyl ether; a benzyl, benzhydryl (diphenylmethyl), or trityl (triphenylmethyl) ether; a trimethylsilyl or t-butyldimethylsilyl ether; or an acetyl ester (-OC(=O)$CH_3$, -OAc).

For example, an aldehyde or ketone group may be protected as an acetal or ketal, respectively, in which the carbonyl group (>C=O) is converted to a diether (>C$(OR)_2$), by reaction with, for example, a primary alcohol. The aldehyde or ketone group is readily regenerated by hydrolysis using a large excess of water in the presence of acid.

For example, an amine group may be protected, for example, as an amide or a urethane, for example, as: a methyl amide (-NHCO-$CH_3$) ; a benzyloxy amide (-NHCO-$OCH_2C_6H_5$, -NH-Cbz) ; as a t-butoxy amide (-NHCO-OC$(CH_3)_3$, -NH-Boc); a 2-biphenyl-2-propoxy amide (-NHCO-OC$(CH_3)_2C_6H_4C_6H_5$, -NH-Bpoc), as a 9-fluorenylmethoxy amide (-NH-Fmoc), as a 6-nitroveratryloxy amide (-NH-Nvoc), as a 2-trimethylsilylethyloxy amide (-NH-Teoc), as a 2,2,2-trichloroethyloxy amide (-NH-Troc), as an allyloxy amide (-NH-Alloc), as a 2(-phenylsulphonyl)ethyloxy amide (-NH-Psec); or, in suitable cases, as an *N*-oxide (>NO•).

For example, a carboxylic acid group may be protected as an ester for example, as: an $C_{1-7}$ alkyl ester (e.g. a methyl ester; a t-butyl ester); a $C_{1-7}$ haloalkyl ester (e.g. a $C_{1-7}$ trihaloalkyl ester) ; a tri$C_{1-7}$ alkylsilyl-$C_{1-7}$ alkyl ester; or a $C_{5-20}$ aryl-$C_{1-7}$ alkyl ester (e.g. a benzyl ester; a nitrobenzyl ester); or as an amide, for example, as a methyl amide.

For example, a thiol group may be protected as a thioether (-SR), for example, as: a benzyl thioether; an acetamidomethyl ether (-S-$CH_2NHC(=O)CH_3$).

Acronyms

For convenience, many chemical moieties are represented using well known abbreviations, including but not limited to, methyl (Me), ethyl (Et), *n*-propyl (nPr), *iso*-propyl (iPr), *n*-butyl (nBu), *tert*-butyl (tBu), *n*-hexyl (nHex), cyclohexyl (cHex), phenyl (Ph), biphenyl (biPh), benzyl (Bn), naphthyl (naph), methoxy (MeO), ethoxy (EtO), benzoyl (Bz), and acetyl (Ac).

For convenience, many chemical compounds are represented using well known abbreviations, including but not limited to, methanol (MeOH), ethanol (EtOH), iso-propanol (i-PrOH), methyl ethyl ketone (MEK), ether or diethyl ether ($Et_2O$), acetic acid (AcOH), dichloromethane (methylene chloride, DCM), trifluoroacetic acid (TFA), dimethylformamide (DMF), tetrahydrofuran (THF), and dimethylsulfoxide (DMSO).

Synthesis

Compounds of the invention can be synthesised by a number of methods, examples of which are given below. Some synthesis routes are shown in Yamaguchi, et al., J. Med. Chem. 1993, 36, 4502 - 4068.

In general, a key step in the synthesis of these compounds is the addition/insertion of hydrazine, thus providing the adjacent nitrogen ring atoms in the central moiety. This addition of hydrazine is accomplished in particular by a ring insertion step in the routes exemplified below.

The formed aromatic ring (represented by -A-B-) is usually derivatised before the routes shown below, and starting materials with the desired structure and substituent pattern are either commercially available or readily synthesised. Route 2 exemplifies a strategy where the aromatic ring is already substituted at the beginning of the synthesis route.

The route illustrated leads to compounds where $R_N$ is H. The possible substituents at this position can be added by the use of an appropriate electrophile with suitable reaction conditions.

Further derivatisation of the groups on $R_C$ can be carried out using a variety of conventional methods, some

of which are illustrated in 'Further derivatisation steps'.

Route 1

Part 1: Synthesis of 2-arylindan-1,3-diones

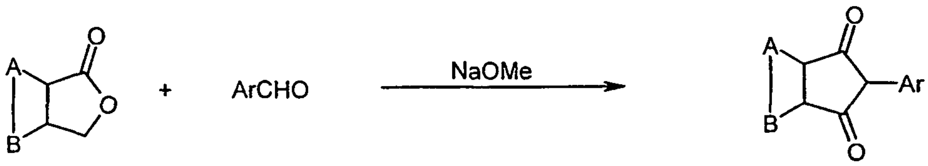


To an ice-cooled solution of phthalide or equivalent, (13.41 g; 0.1 mol) and the aromatic aldehyde (0.1 mol) in a mixture of methanol (50 ml) and ethylpropionate (50 ml) was added a solution of sodium methoxide in methanol [sodium (9.20 g; 0.4 mol) in methanol (50 ml)] with the temperature kept below 10°C. The solution was then heated to gentle reflux for 3 h, cooled to room temperature and poured onto water (500 ml). The mixture was washed with ether (5 x 100 ml) and the aqueous layer acidified with acetic acid and the solid filtered off. This was then used crude in the next stage.

Part 2: Reaction of 2-arylindan-1,3-ones with hydrazine hydrate

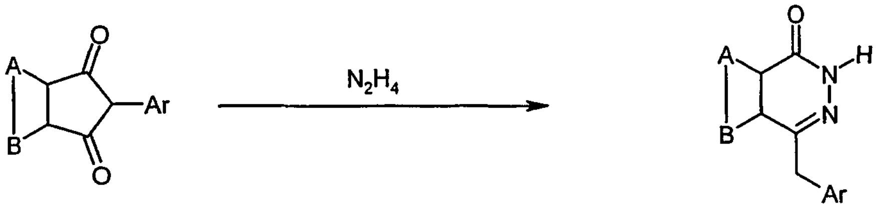


A suspension of 2-arylindan-1,3-dione (20 mmol) in hydrazine monohydrate (40 ml) was heated to reflux for 4 h, cooled and the product filtered off. The solid was washed with ethanol.

Route 2

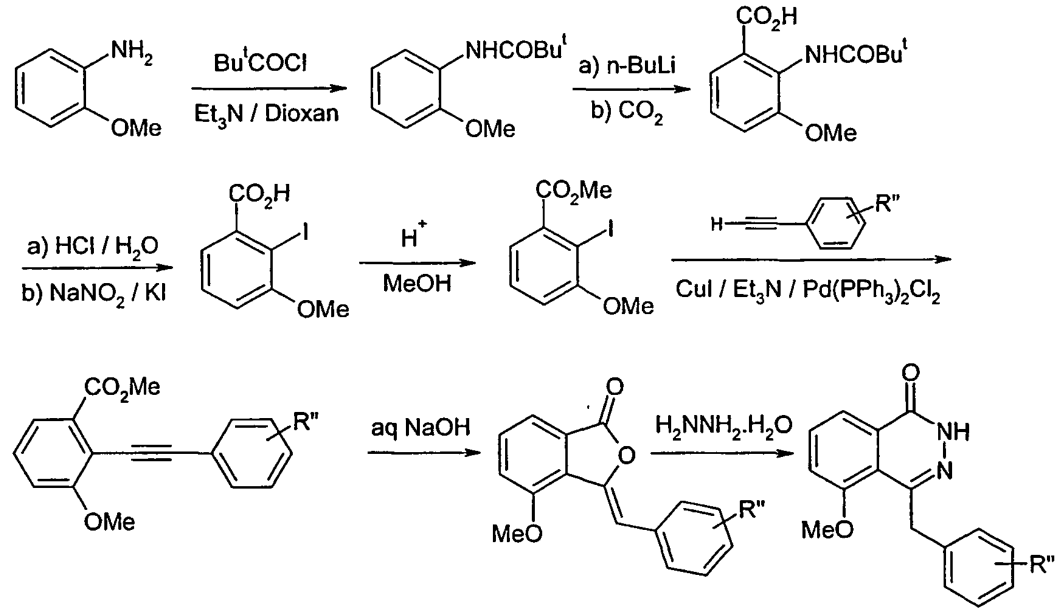


Pivaloyl chloride (120g, 1mol) was added dropwise at ambient temperature to a stirred solution of 2-methoxyaniline (123g, 1mol) and triethylamine (111g, 1.1mol) in 1,4-dioxane (1200ml), the mixture was stirred at ambient temperature for 2 hours, then it was poured into water (3000ml). The product was extracted into ethyl acetate (3 x 300ml), then the combined extracts were dried ($MgSO_4$) and the solvents were removed *in vacuo* to give *N*-(2-methoxyphenyl) pivalamide (198g; 96%) as a low-melting solid which was used without further purification.

*n*-Butyllithium (1.6M solution in hexane; 200ml, 0.32mol) was added dropwise under nitrogen at -10°C to a stirred solution of *N*-(2-methoxyphenyl)pivalamide (27.6g, 0.133mol) in tetrahydrofuran (600ml), the mixture was allowed to warm to ambient temperature, stirred for a further 2 hours, then it was added to a large excess of crushed solid carbon dioxide. The mixture was allowed to warm to ambient temperature, 3M hydrochloric acid (200ml) was added and the tetrahydrofuran was removed *in vacuo.* The resulting solid was collected by filtration and crystallised from acetonitrile to give 3-methoxy-2-pivalamidobenzoic acid (21g; 63%) as a solid, m.pt. 117-120°C. Concentration of the liquor yielded a second crop (2.6g; 8%).

A stirred mixture of 3-methoxy-2-pivalamidobenzoic acid (20g, 0.08mol) and 7M hydrochloric acid (280ml) was heated under reflux for 2 hours then cooled to 0°C. A solution of sodium nitrite (5.8g, 0.09mol) in water (46ml) was added dropwise at <5°C, the mixture was stirred at 0-5°C for 2 hours, then a solution of potassium iodide (17.8g, 0.11mol) in water (39ml) was added dropwise at 0-5°C. The stirred mixture was heated at 70-80°C for 2 hours then cooled in ice. The product was extracted into ethyl acetate (3 x 300ml), the combined extracts were washed with 20% aqueous sodium thiosulphate solution (3 x 300ml), then they were dried ($MgSO_4$) and the solvent was removed *in vacuo* to leave 2-iodo-3-methoxybenzoic acid (13g, 58%) as a solid, m.pt. 142-146°C.

A stirred mixture of 2-iodo-3-methoxybenzoic acid (20g, 0.07mol; prepared in a manner similar to that described above), methanol (300ml) and concentrated sulphuric acid (3.5ml) was heated under reflux for 8 hours, cooled to ambient temperature and added to water (1500ml). The product was extracted into dichloromethane (3 x 500ml), the combined extracts were washed with 5% aqueous sodium hydroxide solution (3 x 500ml), then they were dried ($MgSO_4$) and the solvent was removed *in vacuo* to give methyl 2-iodo-3-methoxybenzoate (18.5g, 90%) as a solid, m.pt. 58-61°C.

The appropriately substituted phenylacetylene (0.063mol) was added at ambient temperature under nitrogen to a stirred solution of copper(I) iodide (0.1g, 6.3mmol), *bis*(triphenylphosphine)palladium(II) dichloride (0.43g, 6.3mmol), and methyl 2-iodo-3-methoxybenzoate (18.5g, 0.063mol) in triethylamine (375ml), the mixture was stirred at ambient temperature for 72 hours, then it was added to 5M hydrochloric acid (1000ml). The product was extracted into ethyl acetate (3 x 400ml), the combined extracts were dried ($MgSO_4$) and the solvent was removed *in vacuo* to give the methyl 3-methoxy-2-(substituted phenylethynyl)benzoate which was used without purification.

A mixture of the crude 3-methoxy-2-(substituted phenylethynyl)benzoate and 30% aqueous sodium hydroxide solution (302ml) was stirred and heated under reflux for 4 hours, cooled to ambient temperature and acidified by the addition of concentrated sulphuric acid. The product was extracted into ether (3 x 500ml), the combined extracts were washed with 10% aqueous sodium carbonate solution (2000ml), then they were dried ($MgSO_4$) and the solvent was removed *in vacuo* to give 4-methoxy-3-(substituted benzylidene)phthalide which was used without purification.

A mixture of the crude 4-methoxy-3-(substituted benzylidene)phthalide (14g) and hydrazine hydrate (83 ml) was heated under reflux for 5 hours then cooled to ambient temperature. The resulting solid was collected by filtration, washed well with ethanol and dried *in vacuo* to give the desired compound.

The substitution of the aromatic ring in the starting material can be altered as appropriate.

Further Derivatisation

*(a) Demethylation of 4-(methoxybenzyl)-1(2H)-phthalazinones*

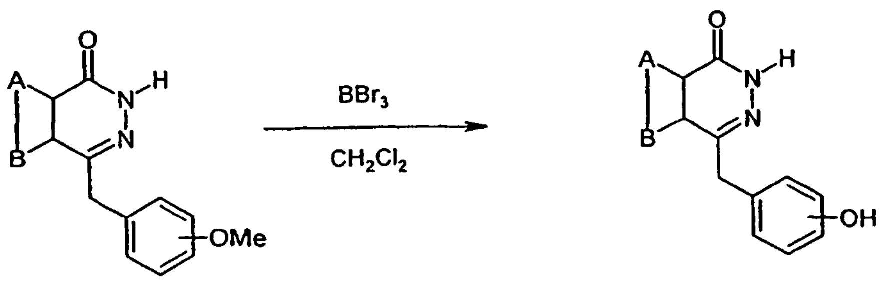


To a suspension of the methoxybenzylphthalazinone or equivalent (0.7 g; 2.65 mmol) in dichloromethane (5 ml) under nitrogen at room temperature was added a solution of boron tribromide in dichloromethane (1M; 6 ml; 6.0 mmol). The mixture was heated to reflux for 24 h, cooled and poured into sodium hydroxide (10%; 25 ml). The organic layer was removed and the aqueous layer acidified (HCl) and the solid filtered off.

(b) *Derivatisation of 4-(aminobenzyl)-1(2H)-phthalazinones*

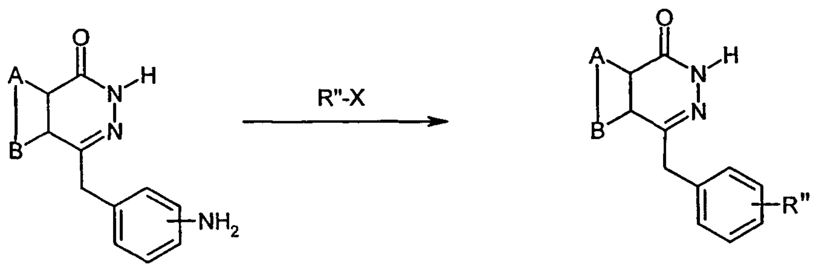


To a stirred solution of the aminobenzylphthalazinone or equivalent (0.6 g; 2.4 mmol) and triethylamine (0.24 g; 2.4 mmol) in 1,4-dioxan (50 ml) was added dropwise the electrophile (2.4 mmol). The mixture was then heated to reflux for 2 h, cooled and poured onto water (100 ml). The solid was then filtered of and washed with water and ethanol before drying *in vacuo.*

(c) *Acylation of hydroxybenzylphthalazinones*

The acylation is generally carried out by the addition of the appropriate acid chloride to the hydroxybenzylphthalazinone under suitable conditions. Examples of this are given below:

(i)

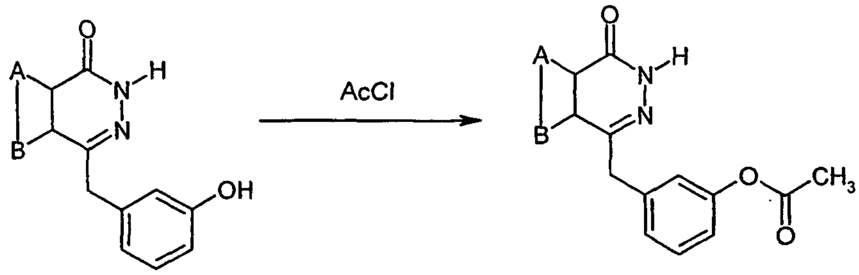


To a stirred solution of the hydroxybenzylphthalazinone or equivalent (e.g. **164**) (0.7 g; 2.79 mmol) and triethylamine (0.28 g; 2.79 mmol) in 1,4-dioxan (40 ml) was added dropwise acetyl chloride (0.2 ml; 2.79 mmol). The mixture was then heated to reflux for 2 h, cooled and poured onto water (100 ml). The solid was then filtered of and washed with water and ethanol before drying *in vacuo.*

(ii)

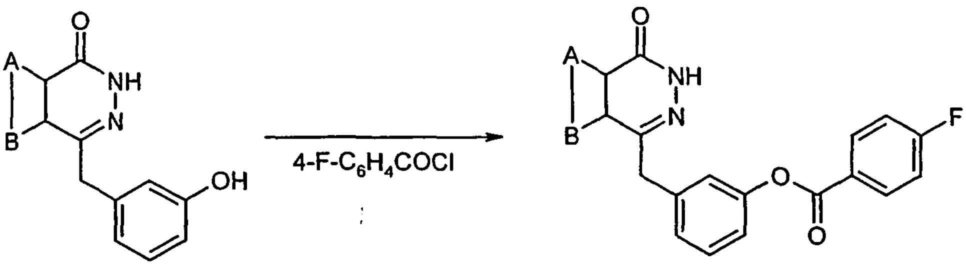


A stirred suspension of the hydroxybenzylphthalazinone (1.36 g; 5.41 mmol), triethylamine (0.61 g; 6.00 mmol) and 4-fluorobenzoyl chloride (0.86 g; 5.41 mmol) in dry 1,4-dioxan (50 ml) was heated under reflux, with the exclusion of moisture ($CaCl_2$), for 2 h and cooled to room temperature. The reaction mixture was then poured onto water (250 ml) and the solid filtered off. The crude solid was then boiled in cyclohexane (10 ml), cooled and filtered off.

(iii)

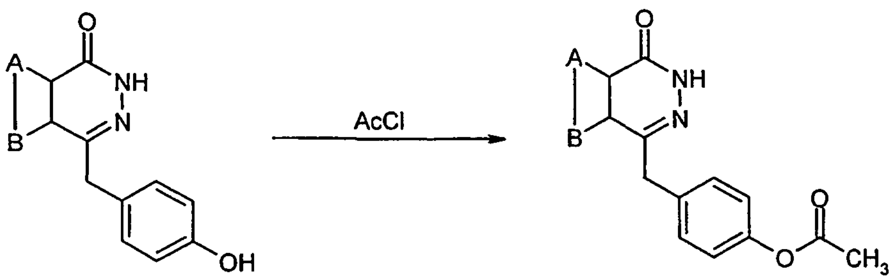


A stirred suspension of hydroxybenzylphthalazinone (0.70 g; 2.79 mmol) or equivalent, triethylamine (0.4 ml; 2.79 mmol) and acetylchloride (0.2 ml; 2.79 mmol) in dry 1,4-dioxan (40 ml) was heated to reflux, with the exclusion of moisture ($CaCl_2$), for 2 h and cooled to room temperature. The reaction mixture was then poured onto water (250 ml) and the solid filtered off.

(iv)

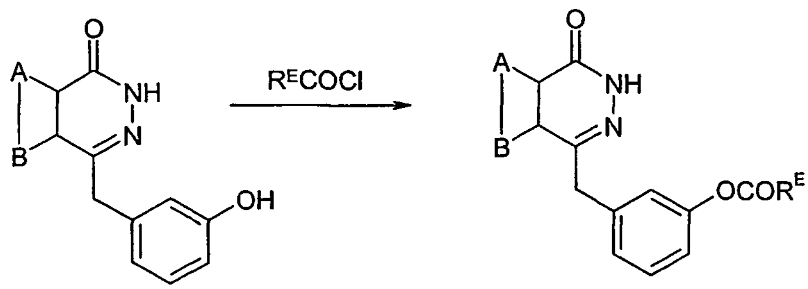


The appropriate acid chloride (0.24mmol) was added to a stirred solution of 4-(3-hydroxybenzyl)phthalazin-1(2*H*)-one (50mg, 0.2mmol) or equivalent and triethylamine (33μl) in 1,4-dioxane (0.5ml) and the mixture was stirred at ambient temperature whilst the progress of the reaction was monitored by tlc. In some cases heating under reflux was necessary to force the reaction to proceed to completion. Once the reaction was complete, the mixture was diluted with ice-water and the product was extracted into ethyl acetate. The extract was washed with saturated aqueous sodium chloride solution and dried ($MgSO_4$), then the solvent was removed *in vacuo.* The residue was purified to give the required ester. The purification was carried out via preparative scale HPLC on a Gilson LC using a Jones Chromatography Genesis 4μ C18 column using gradient elution between aqueous trifluoroacetic acid and acetonitrile as eluents.

(d) *Derivatisation of 3-(aminobenzyl)-1(2H)phthalazinones*

(i)

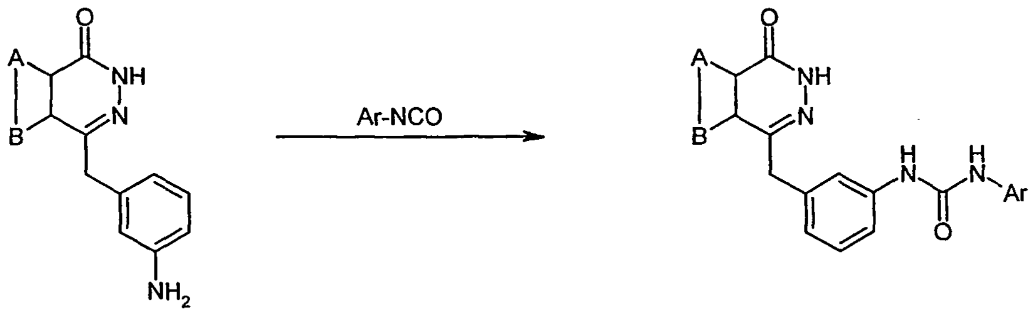


To a stirred solution of the aminobenzylphthalazinone (160) (1.00 g; 40 mmol) or equivalent in dry 1,4-dioxan (25 ml) at 40°C was added the appropriate isocyanate (40 mmol). The mixture was stirred for a further 2 h, cooled to room temperature and the solid filtered off.

(ii)

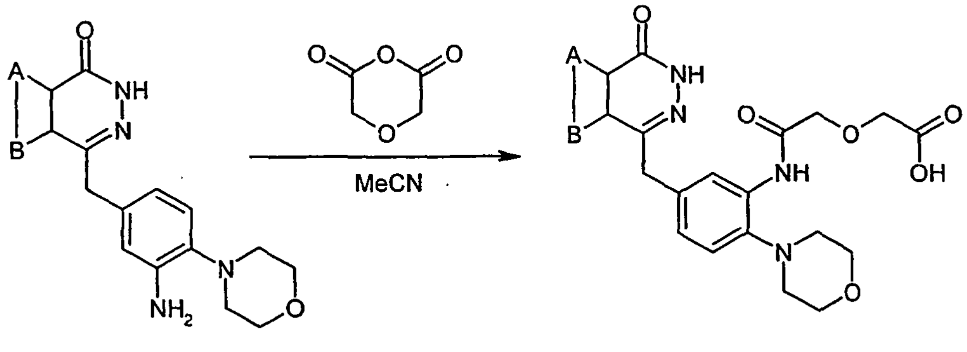


To a stirred solution of the aminophthalazinone (197)(1.00 g; 2.97 mmol) or equivalent in acetonitrile (25 ml) was added diglycolic anhydride (0.35 g; 3.00 mmol). The mixture was stirred at room temperature for 1 h, and the solid filtered off. The crude solid was dissolved in NaOH (10%; 20 ml) and filtered through Celite. The aqueous was then acidified and the solid filtered off.

(iii)

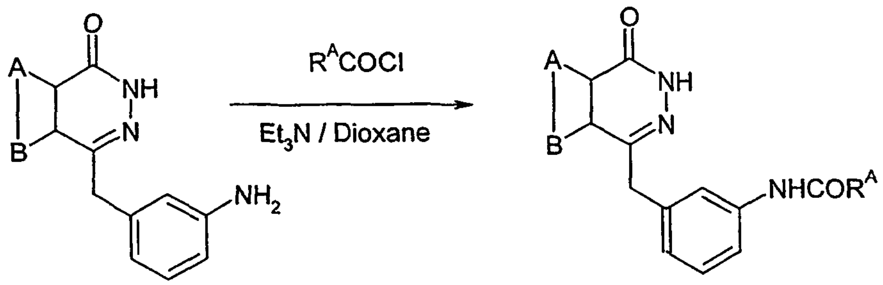


160

The appropriate acid chloride (0.2mmol) was added to a stirred solution of the aminobenzylphthalazinone 160 (0.05 g, 0.2 mmol) or equivalent and triethylamine (33 μl) in 1,4-dioxane (0.5 ml), the mixture was stirred at ambient temperature for 18 hours, then it was diluted with water (10 ml). The product was collected by filtration, washed with water (5 ml) and dried *in vacuo* to give the required amide.

(iv)

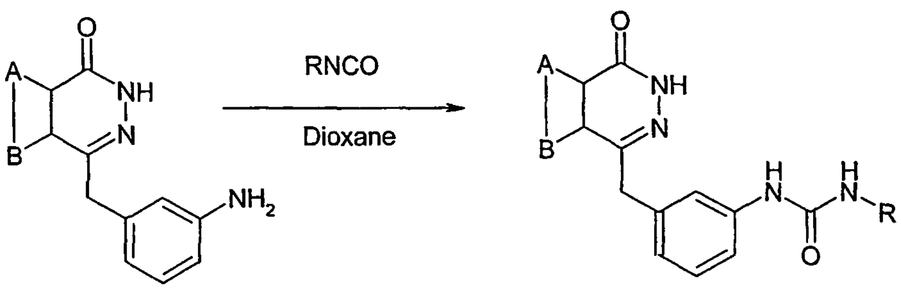


160

A mixture of the aminobenzylphthalazinone 160 (0.5 g, 2 mmol) or equivalent and 1,4-dioxane (15 ml) was stirred at ambient temperature until all of the solid had dissolved (5-15 minutes). The appropriate isocyanate (2mmol) was added, the mixture was stirred at ambient temperature for 2 hours, then it was allowed to stand at ambient temperature

for 18 hours. The resulting solid was collected by filtration, washed well with water and dried *in vacuo* to give the required ureido product.

(v) (a)

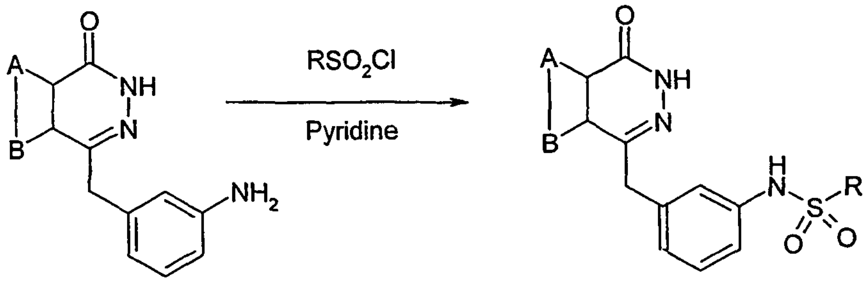


160

The appropriate sulphonyl chloride (1 mmol) was added to a solution of the aminobenzylphthalazinone 160 (0.25 g, 1 mmol) or equivalent in pyridine (10 ml), the stirred mixture was heated under reflux for 2 hours, then it was diluted with water (200 ml). The resulting solid was collected by filtration, washed well with water and dried *in vacuo* to give the required sulphonamido product.

(v) (b)

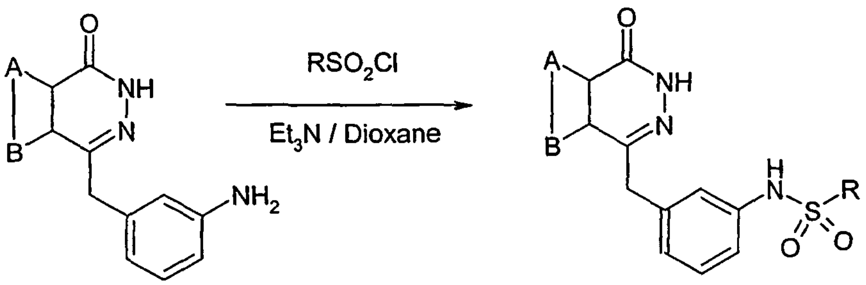


160

The appropriate sulphonyl chloride (1 mmol) was added to a solution of the aminobenzylphthalazinone 160 (0.25 g, 1 mmol) or equivalent and triethylamine (0.1 g, 1 mmol) in 1,4-dioxane (10 ml), the stirred mixture was heated under reflux for 2 hours, then it was diluted with water (200 ml). The resulting solid was collected by filtration, washed well with water and dried *in vacuo* to give the required sulphonamido product.

(e) *Synthesis of 227*

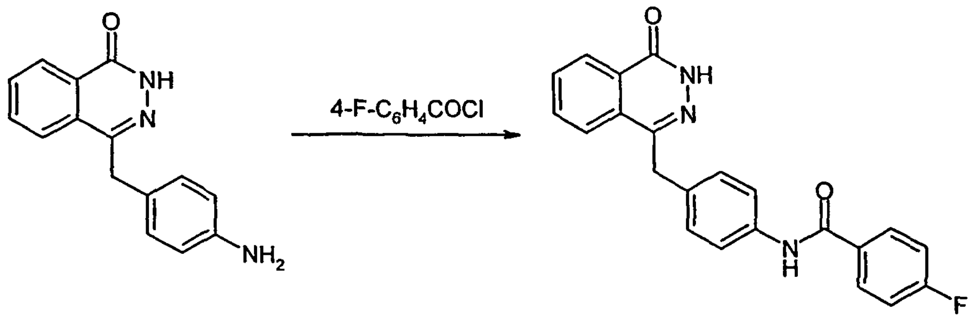


To a stirred solution of 4-fluorobenzoyl chloride (159) (0.95 g; 5.97 mmol) in dry 1,4-dioxan (50 ml) was added triethylamine (0.60 g; 5.97 mmol) and the aminobenzylphthalazinone (1.50 g; 5.97 mmol) or equivalent. The mixture was heated to reflux, with the exclusion of moisture ($CaCl_2$), for 2 h and cooled to room temperature. The reaction mixture was then poured onto water (250 ml) and the solid filtered off.

(f) *Synthesis of 239*

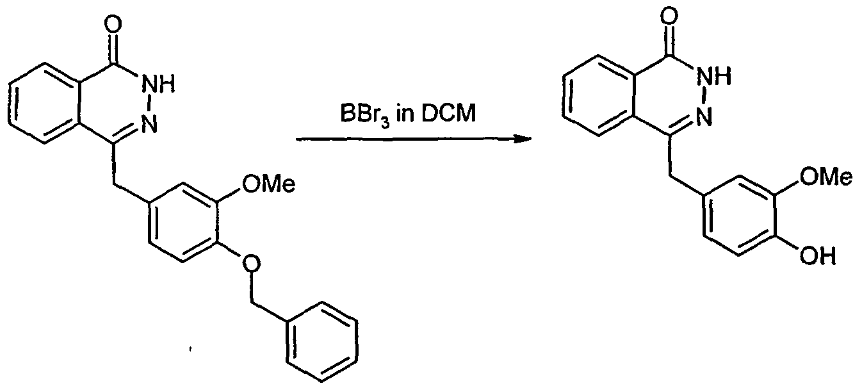


To a stirred suspension of the benzyloxymethoxybenzylphthalazinone (187)(5.00 g; 13.0 mmol) in dichloromethane (11.5 ml) was added, under nitrogen, a solution of boron tribromide in dichloromethane (1.0 M solution; 4.4 ml; 4.40 mmol). The reaction mixture was then heated under reflux for 24 h, cooled to room temperature and poured into ice/water (250 ml). The mixture was then basified by the addition of solid sodium hydroxide and the organic layer removed. The aqueous layer was then washed with dichloromethane (3 x 50 ml) and acidified with concentrated HCl. The solid was filtered off, washed with water and air-dried.

*(g) Synthesis of 247*

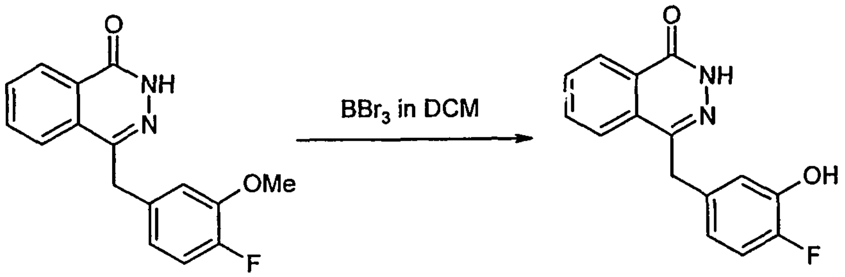


A stirred suspension of the starting methoxy-compound (241) (1.50 g; 5.27 mmol) in a solution of boron tribromide in dichloromethane (1.0 M solution; 12 ml; 12.00 mmol) was heated to reflux for 8 h under nitrogen. The reaction mixture was then cooled to room temperature and poured into ice/water (250 ml). The mixture was then basified by the addition of solid sodium hydroxide and the organic layer removed. The aqueous layer was then washed with dichloromethane (3 x 50 ml) and acidified with concentrated HCl. The solid was filtered off, washed with water and air-dried.

*(h) Synthesis of 277*

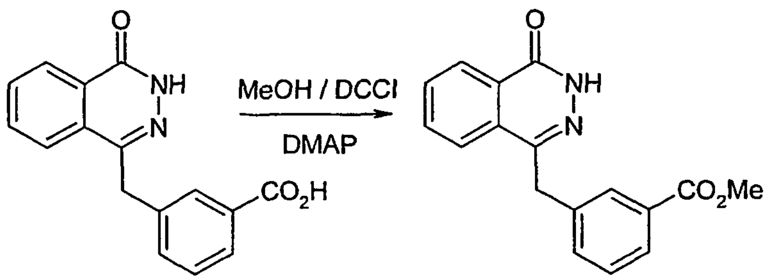


A solution of the carboxylic acid (276) (8g, 0.028mol) in dichloromethane (240ml) was added dropwise at ambient temperature to a stirred mixture of dicyclohexylcarbodiimide (5.8g, 0.028mol), 4-(dimethylamino)pyridine (0.2g, 0.0014mol), methanol (0.92g, 0.028mol) and dichloromethane

(40ml), then the mixture was stirred at ambient temperature for 18 hours and filtered. The filter cake was washed with dichloromethane (280ml), the filtrate and washings were combined and the solvent was removed *in vacuo*. The residue was diluted with ether (1000ml), the resulting precipitate was removed by filtration, then the ethereal filtrate was washed with saturated aqueous sodium hydrogencarbonate solution (400ml), 1.5M hydrochloric acid (400ml), water (400ml) and saturated aqueous sodium chloride solution (400ml). The solution was dried ($MgSO_4$) and the solvent was removed *in vacuo* to give 277.

*(i) Synthesis of 278*

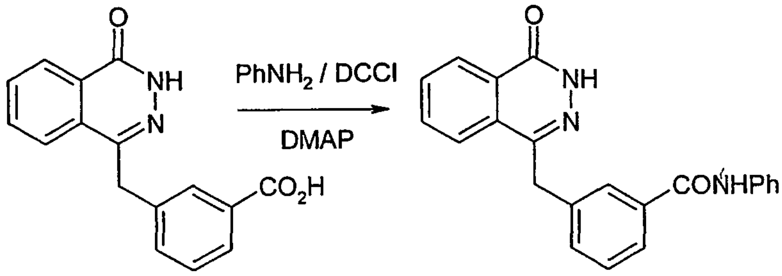


A solution of the carboxylic acid (276) (3g, 0.01mol) in dichloromethane (90ml) was added dropwise at ambient temperature to a stirred mixture of dicyclohexylcarbodiimide (2.2g, 0.01mol), 4-(dimethylamino)pyridine (0.08g, 0.5mmol), aniline (0.9g, 0.01mol) and dichloromethane (15ml), then the mixture was stirred at ambient temperature for 18 hours and filtered. The filter cake was washed with dichloromethane (105ml), the filtrate and washings were combined and the solvent was removed *in vacuo.* The residue was diluted with ether (375ml), the resulting precipitate was removed by filtration, then the ethereal filtrate was washed with saturated aqueous sodium hydrogencarbonate solution (150ml), 1.5M hydrochloric acid (150ml), water (150ml) and saturated aqueous sodium chloride solution (150ml). The solution was dried ($MgSO_4$) and the solvent was removed *in vacuo* to give 278.

*(j) Derivitisation of 197*

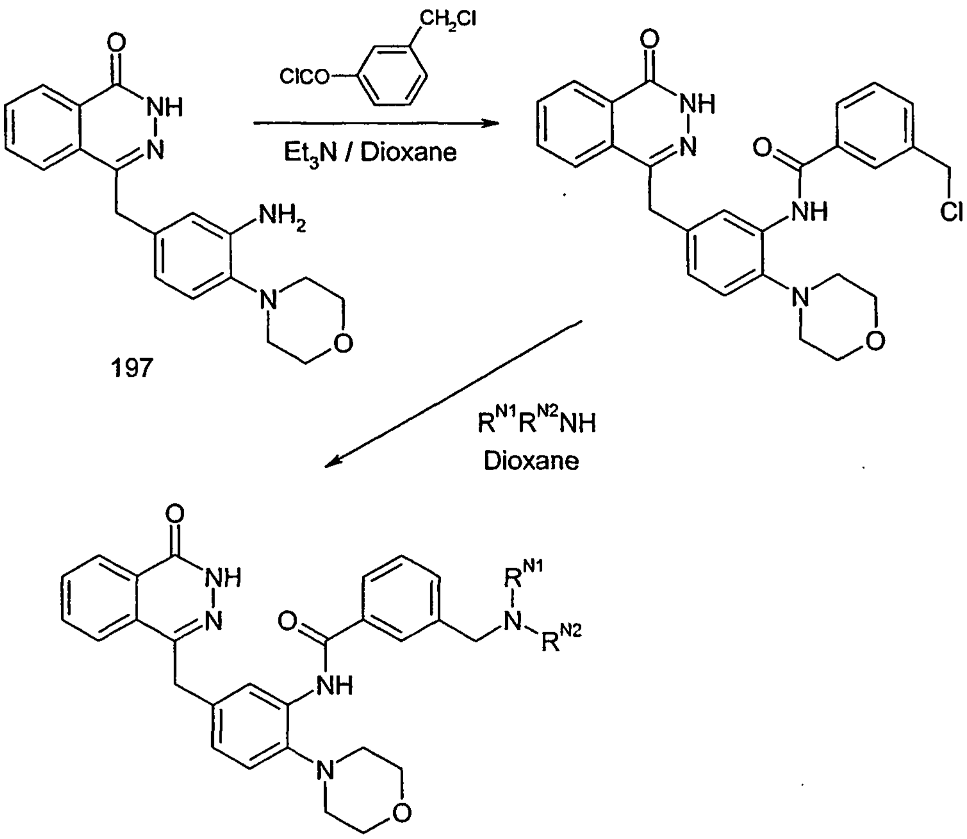


3-Chloromethylbenzoyl chloride (0.56g, 3mmol) was added to a stirred solution of 197 (1g, 3mmol) and triethylamine (0.4ml) in 1,4-dioxane (50ml), the mixture was stirred at ambient temperature for 2 hours, then it was diluted with water (100ml). The product was extracted into ethyl acetate (3 x 50ml), the combined extracts were dried ($MgSO_4$) and

the solvent was removed *in vacuo.* The residue was triturated with hot toluene (50 ml), the hot solution was filtered (Celite), cyclohexane (50 ml) was added, and the mixture was allowed to cool to ambient temperature. The resulting solid was collected by filtration and dried *in vacuo* to give 3-(chloromethyl)-*N*-[2-morpholino-5-(1-oxophthalazin-4-ylmethyl)phenyl]benzamide (1.32g, 90%) as a solid, m.pt. 117-122°C.

A mixture of 3-(chloromethyl)-N-[2-morpholino-5-(1-oxophthalazin-4-ylmethyl)phenyl]benzamide (0.66g, 1.35mmol), the appropriate amine(27mmol) and 1,4-dioxane (50ml) was heated under reflux for 2 hours, cooled to ambient temperature and diluted with water (100ml) to precipitate a sticky solid. The aqueous layer was removed by decantation and the residual solid was extracted into ethyl acetate (3 x 50ml). The combined extracts were washed with water (50ml), dried ($MgSO_4$), then the solvent was removed *in vacuo.* The residue was triturated with hot toluene (50 ml), the hot solution was separated by decantation from insoluble material, cyclohexane (50 ml) was added, and the mixture was allowed to cool to ambient temperature. The resulting solid was collected by filtration, washed with cyclohexane (30ml) and dried *in vacuo* to give the desired compound.

*(k) Synthesis of 265*

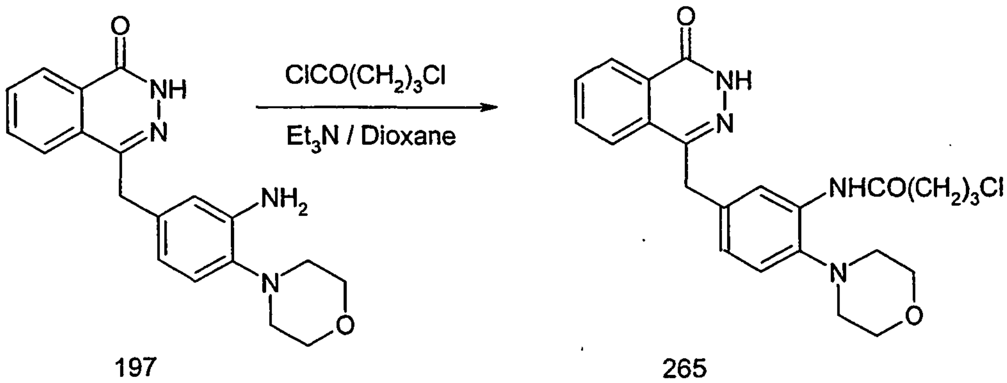


197 265

4-Chlorobutyryl chloride (1.26g, 8.9mmol) was added to a stirred solution of 197 (3g, 8.9mmol) and triethylamine (0.9g, 8.9mmol) in 1,4-dioxane (50ml), the mixture was stirred at ambient temperature for 2 hours, then it was diluted with water (100ml), causing a sticky semisolid to precipitate. The aqueous phase was removed by decantation, and the residue was triturated with hot ethyl acetate (100ml). The hot solution was separated from an insoluble residue, and the solvent was removed *in vacuo.* The residue was triturated with cyclohexane (50ml) and the resulting solid was collected by filtration and dried *in vacuo* to give 4-chloro-N-[2-morpholino-5-(1-oxophthalazin-4-ylmethyl)phenyl]butyramide.

Use

The present invention provides active compounds, specifically, active in inhibiting the activity of PARP.

The term "active," as used herein, pertains to compounds which are capable of inhibiting PARP activity, and specifically includes both compounds with intrinsic activity (drugs) as well as prodrugs of such compounds, which prodrugs may themselves exhibit little or no intrinsic activity.

One assay which may conveniently be used in order to assess the PARP inhibition offered by a particular compound is described in the examples below.

The present invention further provides a method of inhibiting the activity of PARP in a cell, comprising contacting said cell with an effective amount of an active compound, preferably in the form of a pharmaceutically acceptable composition. Such a method may be practised *in vitro* or *in vivo.*

For example, a sample of cells may be grown *in vitro* and an active compound brought into contact with said cells, and the effect of the compound on those cells observed. As examples of "effect," the amount of DNA repair effected in a certain time may be determined. Where the active compound is found to exert an influence on the cells, this may be used as a prognostic or diagnostic marker of the efficacy of the compound in methods of treating a patient carrying cells of the same cellular type.

The term "treatment," as used herein in the context of treating a condition, pertains generally to treatment and therapy, whether of a human or an animal (e.g. in veterinary applications), in which some desired therapeutic effect is achieved, for example, the inhibition of the progress of the condition, and includes a reduction in the rate of progress,

a halt in the rate of progress, amelioration of the condition, and cure of the condition. Treatment as a prophylactic measure (i.e. prophylaxis) is also included.

The term "adjunct" as used herein relates to the use of active compounds in conjunction with known therapeutic means. Such means include cytotoxic regimes of drugs and/or ionising radiation as used in the treatment of different cancer types.

Active compounds may also be used as cell culture additives to inhibit PARP, for example, in order to radio-sensitize cells to known chemo or ionising radiation treatments *in vitro.*

Active compounds may also be used as part of an *in vitro* assay, for example, in order to determine whether a candidate host is likely to benefit from treatment with the compound in question.

## Administration

The active compound or pharmaceutical composition comprising the active compound may be administered to a subject by any convenient route of administration, whether systemically/peripherally or at the site of desired action, including but not limited to, oral (e.g. by ingestion); topical (including e.g. transdermal, intranasal, ocular, buccal, and sublingual); pulmonary (e.g. by inhalation or insufflation therapy using, e.g. an aerosol, e.g. through mouth or nose); rectal; vaginal; parenteral, for example, by injection, including subcutaneous, intradermal, intramuscular, intravenous, intraarterial, intracardiac, intrathecal, intraspinal, intracapsular, subcapsular, intraorbital, intraperitoneal, intratracheal, subcuticular, intraarticular, subarachnoid, and intrasternal; by implant of a depot, for example, subcutaneously or intramuscularly.

The subject may be a eukaryote, an animal, a vertebrate animal, a mammal, a rodent (e.g. a guinea pig, a hamster, a rat, a mouse), murine (e.g. a mouse), canine (e.g. a dog), feline (e.g. a cat), equine (e.g. a horse), a primate, simian (e.g. a monkey or ape), a monkey (e.g. marmoset, baboon), an ape (e.g. gorilla, chimpanzee, orangutang, gibbon), or a human.

## Formulations

While it is possible for the active compound to be administered alone, it is preferable to present it as a pharmaceutical composition (e.g., formulation) comprising at least one active compound, as defined above, together with one or more pharmaceutically acceptable carriers, adjuvants, excipients, diluents, fillers, buffers, stabilisers, preservatives, lubricants, or other materials well known to those skilled in the art and optionally other therapeutic or prophylactic agents.

Thus, the present invention further provides pharmaceutical compositions, as defined above, and methods of making a pharmaceutical composition comprising admixing at least one active compound, as defined above, together with one or more pharmaceutically acceptable carriers, excipients, buffers, adjuvants, stabilisers, or other materials, as described herein.

The term "pharmaceutically acceptable" as used herein pertains to compounds, materials, compositions, and/or dosage forms which are, within the scope of sound medical judgement, suitable for use in contact with the tissues of a subject (e.g., human) without excessive toxicity, irritation, allergic response, or other problem or complication, commensurate with a reasonable benefit/risk ratio. Each carrier, excipient, etc. must also be "acceptable" in the sense of being compatible with the other ingredients of the formulation.

Suitable carriers, excipients, etc. can be found in standard pharmaceutical texts, for example, Remington's Pharmaceutical Sciences, 18th edition, Mack Publishing Company, Easton, Pa., 1990.

The formulations may conveniently be presented in unit dosage form and may be prepared by any methods well known in the art of pharmacy. Such methods include the step of bringing into association the active compound with the carrier which constitutes one or more accessory ingredients. In general, the formulations are prepared by uniformly and intimately bringing into association the active compound with liquid carriers or finely divided solid carriers or both, and then if necessary shaping the product.

Formulations may be in the form of liquids, solutions, suspensions, emulsions, elixirs, syrups, tablets, losenges, granules, powders, capsules, cachets, pills, ampoules, suppositories, pessaries, ointments, gels, pastes, creams, sprays, mists, foams, lotions, oils, boluses, electuaries, or aerosols.

Formulations suitable for oral administration (e.g., by ingestion) may be presented as discrete units such as capsules, cachets or tablets, each containing a predetermined amount of the active compound; as a powder or granules; as a solution or suspension in an aqueous or non-aqueous liquid; or as an oil-in-water liquid emulsion or a water-in-oil liquid emulsion; as a bolus; as an electuary; or as a paste.

A tablet may be made by conventional means, e.g., compression or molding, optionally with one or more accessory ingredients. Compressed tablets may be prepared by compressing in a suitable machine the active compound in a free-flowing form such as a powder or granules, optionally mixed with one or more binders (e.g., povidone, gelatin,

acacia, sorbitol, tragacanth, hydroxypropylmethyl cellulose); fillers or diluents (e.g., lactose, microcrystalline cellulose, calcium hydrogen phosphate); lubricants (e.g., magnesium stearate, talc, silica); disintegrants (e.g., sodium starch glycolate, cross-linked povidone, cross-linked sodium carboxymethyl cellulose); surface-active or dispersing or wetting agents (e.g., sodium lauryl sulfate); and preservatives (e.g., methyl p-hydroxybenzoate, propyl p-hydroxybenzoate, sorbic acid). Molded tablets may be made by molding in a suitable machine a mixture of the powdered compound moistened with an inert liquid diluent. The tablets may optionally be coated or scored and may be formulated so as to provide slow or controlled release of the active compound therein using, for example, hydroxypropylmethyl cellulose in varying proportions to provide the desired release profile. Tablets may optionally be provided with an enteric coating, to provide release in parts of the gut other than the stomach.

Formulations suitable for topical administration (e.g., transdermal, intranasal, ocular, buccal, and sublingual) may be formulated as an ointment, cream, suspension, lotion, powder, solution, past, gel, spray, aerosol, or oil. Alternatively, a formulation may comprise a patch or a dressing such as a bandage or adhesive plaster impregnated with active compounds and optionally one or more excipients or diluents.

Formulations suitable for topical administration in the mouth include losenges comprising the active compound in a flavored basis, usually sucrose and acacia or tragacanth; pastilles comprising the active compound in an inert basis such as gelatin and glycerin, or sucrose and acacia; and mouthwashes comprising the active compound in a suitable liquid carrier.

Formulations suitable for topical administration to the eye also include eye drops wherein the active compound is dissolved or suspended in a suitable carrier, especially an aqueous solvent for the active compound.

Formulations suitable for nasal administration, wherein the carrier is a solid, include a coarse powder having a particle size, for example, in the range of about 20 to about 500 microns which is administered in the manner in which snuff is taken, i.e., by rapid inhalation through the nasal passage from a container of the powder held close up to the nose. Suitable formulations wherein the carrier is a liquid for administration as, for example, nasal spray, nasal drops, or by aerosol administration by nebuliser, include aqueous or oily solutions of the active compound.

Formulations suitable for administration by inhalation include those presented as an aerosol spray from a pressurised pack, with the use of a suitable propellant, such as dichlorodifluoromethane, trichlorofluoromethane, dichorotetrafluoroethane, carbon dioxide, or other suitable gases.

Formulations suitable for topical administration via the skin include ointments, creams, and emulsions. When formulated in an ointment, the active compound may optionally be employed with either a paraffinic or a watermiscible ointment base. Alternatively, the active compounds may be formulated in a cream with an oil-in-water cream base. If desired, the aqueous phase of the cream base may include, for example, at least about 30% w/w of a polyhydric alcohol, i.e., an alcohol having two or more hydroxyl groups such as propylene glycol, butane-1,3-diol, mannitol, sorbitol, glycerol and polyethylene glycol and mixtures thereof. The topical formulations may desirably include a compound which enhances absorption or penetration of the active compound through the skin or other affected areas. Examples of such dermal penetration enhancers include dimethylsulfoxide and related analogues.

When formulated as a topical emulsion, the oily phase may optionally comprise merely an emulsifier (otherwise known as an emulgent), or it may comprises a mixture of at least one emulsifier with a fat or an oil or with both a fat and an oil. Preferably, a hydrophilic emulsifier is included together with a lipophilic emulsifier which acts as a stabiliser. It is also preferred to include both an oil and a fat. Together, the emulsifier(s) with or without stabiliser(s) make up the so-called emulsifying wax, and the wax together with the oil and/or fat make up the so-called emulsifying ointment base which forms the oily dispersed phase of the cream formulations.

Suitable emulgents and emulsion stabilisers include Tween 60, Span 80, cetostearyl alcohol, myristyl alcohol, glyceryl monostearate and sodium lauryl sulphate. The choice of suitable oils or fats for the formulation is based on achieving the desired cosmetic properties, since the solubility of the active compound in most oils likely to be used in pharmaceutical emulsion formulations may be very low. Thus the cream should preferably be a non-greasy, non-staining and washable product with suitable consistency to avoid leakage from tubes or other containers. Straight or branched chain, mono- or dibasic alkyl esters such as diisoadipate, isocetyl stearate, propylene glycol diester of coconut fatty acids, isopropyl myristate, decyl oleate, isopropyl palmitate, butyl stearate, 2-ethylhexyl palmitate or a blend of branched chain esters known as Crodamol CAP may be used, the last three being preferred esters. These may be used alone or in combination depending on the properties required. Alternatively, high melting point lipids such as white soft paraffin and/or liquid paraffin or other mineral oils can be used.

Formulations suitable for rectal administration may be presented as a suppository with a suitable base comprising, for example, cocoa butter or a salicylate.

Formulations suitable for vaginal administration may be presented as pessaries, tampons, creams, gels, pastes, foams or spray formulations containing in addition to the active compound, such carriers as are known in the art to be appropriate.

Formulations suitable for parenteral administration (e.g., by injection, including cutaneous, subcutaneous, intramuscular, intravenous and intradermal), include aqueous and non-aqueous isotonic, pyrogen-free, sterile injection

solutions which may contain anti-oxidants, buffers, preservatives, stabilisers, bacteriostats, and solutes which render the formulation isotonic with the blood of the intended recipient; and aqueous and non-aqueous sterile suspensions which may include suspending agents and thickening agents, and liposomes or other microparticulate systems which are designed to target the compound to blood components or one or more organs. Examples of suitable isotonic vehicles for use in such formulations include Sodium Chloride Injection, Ringer's Solution, or Lactated Ringer's Injection. Typically, the concentration of the active compound in the solution is from about 1 ng/ml to about 10 μg/ml, for example from about 10 ng/ml to about 1 μg/ml. The formulations may be presented in unit-dose or multi-dose sealed containers, for example, ampoules and vials, and may be stored in a freeze-dried (lyophilised) condition requiring only the addition of the sterile liquid carrier, for example water for injections, immediately prior to use. Extemporaneous injection solutions and suspensions may be prepared from sterile powders, granules, and tablets. Formulations may be in the form of liposomes or other microparticulate systems which are designed to target the active compound to blood components or one or more organs.

Dosage

It will be appreciated that appropriate dosages of the active compounds, and compositions comprising the active compounds, can vary from patient to patient. Determining the optimal dosage will generally involve the balancing of the level of therapeutic benefit against any risk or deleterious side effects of the treatments of the present invention. The selected dosage level will depend on a variety of factors including, but not limited to, the activity of the particular compound, the route of administration, the time of administration, the rate of excretion of the compound, the duration of the treatment, other drugs, compounds, and/or materials used in combination, and the age, sex, weight, condition, general health, and prior medical history of the patient. The amount of compound and route of administration will ultimately be at the discretion of the physician, although generally the dosage will be to achieve local concentrations at the site of action which achieve the desired effect without causing substantial harmful or deleterious side-effects.

Administration *in vivo* can be effected in one dose, continuously or intermittently (e.g., in divided doses at appropriate intervals) throughout the course of treatment. Methods of determining the most effective means and dosage of administration are well known to those of skill in the art and will vary with the formulation used for therapy, the purpose of the therapy, the target cell being treated, and the subject being treated. Single or multiple administrations can be carried out with the dose level and pattern being selected by the treating physician.

In general, a suitable dose of the active compound is in the range of about 100 μg to about 250 mg per kilogram body weight of the subject per day. Where the active compound is a salt, an ester, prodrug, or the like, the amount administered is calculated on the basis of the parent compound and so the actual weight to be used is increased proportionately.

Synthesis Data

The compounds of which the structures are shown in Table 1, with a number greater than 100 were synthesised according to the synthesised routes above - the characterisation data follows. The compounds in Table 1 with a number less than 100 are available from Maybridge plc, Cornwall, UK.

Route 1

(-A-B- = benzene ring)

**126;** Ar = 4-chlorophenyl Yield, 31%; mpt. 218-220°C; $\delta_H$ 4.30 (2H, s), 7.30 (4H, s), 7.75-8.00 (3H, s), 8.25-8.45 (1H, m), 12.40 (1H, br s); m/z $(M+H)^{+\cdot}$ 271 (100%), 273 (35%).

**129;** Ar = 4-bromophenyl

Yield, 59%; mpt. 232-235°C; $\delta_H$ 4.40 (2H, s), 7.30 (2H, d, *J* = 8.7 Hz), 7.45 (2H, d, *J* = 8.7 Hz), 7.60-7.95 (3H, m), 8.25-8.45 (1H, m), 12.40 (1H, br s); m/z $(M+H)^{+\cdot}$ 314 (100%), 316 (95%).

**131;** Ar = 1-naphthyl

Yield, 58%; mpt. 228-231°C; $\delta_H$ 4.80 (2H, s), 7.25-8.50 (11H, m), 12.50 (1H, br s) ; m/z $(M+H)^{+\cdot}$ 287 (100%).

**132;** Ar = 4-fluorophenyl

Yield, 54%; mpt. 194-197°C; $\delta_H$ 4.30 (2H, s), 7.10 (2H, d, *J* = 8.5 Hz), 7.25 (2H, d, *J* = 8.5 Hz), 7.25-7.50 (1H, m), 7.75-8.00 (2H, m), 8.25-8.45 (1H, m), 12.55 (1H, br s); m/z $(M+H)^{+\cdot}$ 255 (100%).

**138;** Ar = 4-methoxyphenyl

Yield, 66%; mpt. 194-196°C; $\delta_H$ 3.70 (3H, s), 4.50 (2H, s), 6.85 (2H, d, *J* = 8.5 Hz), 7.30 (2H, d, *J* = 8.5 Hz), 7.70-8.00 (3H, m), 8.25-8.45 (1H, m), 12.50 (1H, br s); m/z $(M+H)^{+\cdot}$ 267 (100%).

**139;** Ar = 4-methylphenyl

Yield, 80%; mpt. 205-207°C; $\delta_H$ 2.15 (3H, s), 4.25 (2H, s), 7.00-7.30 (4H, m), 7.60-7.95 (3H, m,), 8.25-8.45 (1H, m),

12.60 (1H, br s); m/z $(M+H)^{+\cdot}$ 251 (100%).

**141;** Ar = 2-fluorophenyl

Yield, 85%; mpt. 235-238°C; $\delta_H$ 4.40 (2H, s), 7.10-7.45 (4H, m), 7.70-8.05 (3H, m), 8.25-8.45 (1H, m), 12.40 (1H, br s); m/z $(M+H)^{+\cdot}$ 255 (100%).

**142;** Ar = 2-methoxyphenyl

Yield, 74%; mpt. 158-160°C; $\delta_H$ 3.70 (3H, s), 4.25 (2H, s), 6.70-6.95 (3H, m), 7.10-7.35 (1H, m), 7.60-7.95 (3H, m), 8.45-8.55 (1H, m), 11.15 (1H, br s) ; m/z $(M+H)^{+\cdot}$ 281 (100%).

**145;** Ar = phenyl

Yield, 85%; mpt. 201-204°C; $\delta_H$ 4.45 (2H, s), 7.20-7.45 (5H, m), 7.75-8.00 (3H, m), 8.25-8.45 (1H, m), 12.40 (1H, br s); m/z $(M+H)^{+\cdot}$ 237 (100%).

**151;** Ar = 4-iodophenyl

Yield, 86%; mpt. 233-236°C; $\delta_H$ 4.20 (2H, s), 7.15 (2H, d, *J* = 8.2 Hz), 7.60 (2H, d, *J* = 8.2 Hz), 7.75-7.95 (3H, m), 8.25-8.45 (1H, m), 12.15 (1H, br s); m/z $(M+H)^{+\cdot}$ 362 (100%).

**159;** Ar = 4-aminophenyl

Yield, 28%; mpt. 233-236°C; $\delta_H$ 4.15 (2H, s), 4.85 (2H, s), 6.50 (2H, d, *J* = 7.1 Hz), 7.00 (2H, d, *J* = 7.1 Hz), 7.75-7.95 (3H, m), 8.25-8.45 (1H, m), 12.50 (1H, br s); m/z $(M+H)^{+\cdot}$ 252 (100%).

**160;** Ar = 3-aminophenyl

Yield, 95%; mpt. 178-180°C; $\delta_H$ 4.15 (2H, s), 5.00 (2H, br s), 6.35-6.55 (3H, m), 6.80-7.05 (1H, m), 7.75-7.90 (3H, m), 8.25-8.40 (1H, m); m/z $(M+H)^{+\cdot}$ 252 (100%).

**163;** Ar = 2-methylphenyl

Yield, 72%; mpt. 201-204°C; $\delta_H$ 2.15 (3H, s), 4.10 (2H, s), 6.95-7.25 (4H, m), 7.80-7.95 (3H, m), 8.25-8.45 (1H, m), 12.25 (1H, br s).

**177;** Ar = 4-pyridyl

Yield, 40%; mpt. 214-216°C; $\delta_H$ 4.25 (2H, s), 7.45 (2H, d, *J* = 5.7 Hz), 7.75-7.95 (3H, m), 8.25-8.45 (1H, m), 8.55 (2H, d, *J* = 5.7 Hz), 12.00 (1H, br s); m/z $(M+H)^{+\cdot}$ 238 (100%).

**178;** Ar = 3-pyridyl

Yield, 62%; mpt. 196-199°C; $\delta_H$ 4.30 (2H, s), 7.25-7.45 (1H, m), 7.60-7.95 (4H, m), 8.25-8.45 (2H, m), 8.55 (1H, s), 12.15 (1H, br s); m/z $(M+H)^{+\cdot}$ 238 (100%).

**180;** Ar = 3,4-methylenedioxyphenyl

Yield, 59%; mpt. 225-228°C; $\delta_H$ 4.25 (2H, s), 6.00 (2H, s), 6.85-7.00 (3H, m), 7.70-7.95 (3H, m), 8.25-8.45 (1H, m), 12.25 (1H, br s); m/z $(M+H)^{+\cdot}$ 281 (100%).

**186;** Ar = 3-chlorophenyl

Yield, 69%; mpt. 192-194°C; $\delta_H$ 4.30 (2H, s), 7.30-7.50 (3H, s), 7.75-8.00 (3H, m), 8.25-8.45 (1H, m), 12.60 (1H, br s); m/z $(M+H)^{+\cdot}$ 271 (100%), 273 (35%).

**187;** Ar = 3-benzyloxy-4-methoxyphenyl

Yield, 51%; mpt. 150-152°C; $\delta_H$ 3.60 (3H, s), 4.20 (2H, s), 5.05 (2H, s), 7.30-7.50 (3H, s), 7.55 (5H, s), 7.75-8.00 (3H, m), 8.25-8.45 (1H, m), 12.50 (1H, br s); m/z $(M-H)^{+\cdot}$ 371(100%).

**191;** Ar = 3-(trifluoromethyl)phenyl

Yield, 71%; mpt. 195-198°C; $\delta_H$ 4.30 (2H, s), 7.50- 8.00 (7H, m), 8.25-8.45 (1H, m), 11.60 (1H, br s); m/z $(M+H)^{+\cdot}$ 305 (100%).

**192;** Ar = 3-fluorophenyl

Yield, 70%; mpt. 187-190°C; $\delta_H$ 4.30 (2H, s), 6.90-7.45 (4H, m), 7.75-8.00 (3H, m), 8.25-8.45 (1H, m), 12.30 (1H, br s); m/z $(M+H)^{+\cdot}$ 255 (65%).

**193;** Ar = 3-phenoxyphenyl

Yield, 52%; mpt. 146-148°C; $\delta_H$ 4.20 (2H, s), 6.80-7.50 (9H, m), 7.75-8.00 (3H, m), 8.25-8.45 (1H, m), 12.00 (1H, br s); m/z $(M+H)^{+\cdot}$ 329 (45%).

**194;** Ar = 3-benzyloxyphenyl

Yield, 83%; mpt. 177-180°C; $\delta_H$ 4.20 (2H, s), 5.00 (2H, s), 6.80-7.50 (9H, m), 7.75-8.00 (3H, m), 8.25-8.45 (1H, m), 12.00 (1H, br s).

**198;** Ar = 3-amino-4-thiomorpholinophenyl

Yield, 6%; mpt. 235-238°C; $\delta_H$ 2.75 (4H, br s), 2.95 (4H, br s), 4.10 (2H, s), 4.65 (2H, s), 6.50-6.85 (3H, m), 7.75-8.00 (3H, m), 8.25-8.45 (1H, m), 12.50 (1H, s); m/z $(M+H)^{+\cdot}$ 353 (100%).

**202;** Ar = 3,4-difluorophenyl

Yield, 70%; mpt. 186-191°C; $\delta_H$ 4.25 (2H, br s), 7.00-7.55 (3H, m), 7.75-8.00 (3H, m), 8.25-8.45 (1H, m), 12.50 (1H, s); m/z $(M-H)^{+\cdot}$ 271 (100%).

**204;** Ar = 3-nitro-4-pyrrolidinophenyl

Yield, 1%; mpt. 268-270°C; $\delta_H$ 2.75 (4H, br s), 3.10 (4H, br s), 4.10 (2H, s), 6.85 (1H, d, *J* = 8.15 Hz), 7.45 (1H, dd, *J* = <2 Hz and 8.15 Hz), 7.75-8.00 (3H, m), 8.25-8.45 (1H, m), 12.50 (1H, s); m/z $(M+H)^{+\cdot}$ 351 (100%).

**211;** Ar = 3-bromophenyl
Yield, 80%; mpt. 199-202°C; $\delta_H$ 4.35 (2H, s), 7.20-7.60 (4H, m), 7.75-8.00 (3H, m), 8.25-8.45 (1H, m), 12.40 (1H, s); m/z $(M+H)^{+\cdot}$ 316 (95%) and 318 (100%).

**222;** Ar = 4-benzyloxy-3-methoxyphenyl
Yield, 29%; mpt. 173-175°C; $\delta_H$ 3.60 (3H, s), 4.10 (2H, s), 5.00 (2H, s), 6.60-6.95 (3H, m), 7.40 (5H, s), 7.75-8.00 (3H, m), 8.25-8.45 (1H, m), 12.50 (1H, s); m/z $(M+H)^{+\cdot}$ 373 (100%).

**241;** Ar = 4-fluoro-3-methoxyphenyl Yield, 55%; mpt. 211-214°C; $\delta_H$ 3.80 (3H, s), 4.25 (2H, s), 6.75-7.25 (3H, m), 7.75-8.00 (3H, m), 8.25-8.45 (1H, m), 12.50 (1H, s); m/z $(M+H)^{+\cdot}$ 285 (100%).

**248;** Ar = 2-fluorophenyl
Yield, 86%; mpt. 239-236°C; $\delta_H$ 4.30 (2H, s), 4.65 (2H, s), 7.00-7.45 (4H, m), 7.75-8.00 (3H, m), 8.25-8.45 (1H, m), 12.50 (1H, s); m/z $(M+H)^{+\cdot}$ 255 (100%).

**249;** Ar = 2-pyridyl
Yield, 84%; mpt. 180-184°C; $\delta_H$ 4.45 (2H, s), 7.10-7.45 (2H, m), 7.50-8.00 (4H, m), 8.25-8.45 (1H, m), 8.45-8.55 (1H, m), 12.50 (1H, s); m/z $(M+H)^{+\cdot}$ 238 (100%).

**276;** Ar = 3-carboxyphenyl
Yield, 59%; m.pt. 277-280°C; $\delta_H$ 4.40 (2H, s), 7.3-8.0 (7H, m), 8.1-8.4 (1H, m) and 12.60 (1H, s); m/z $(M+H)^{+\cdot}$ 281 (88% purity).

**<u>Route 2</u>**

**279;** R" = 4-methyl
Yield, 5% over 7 stages; m.pt. 215-217°C; $\delta_H$ 2.20 (3H, s), 3.80 (3H, s), 4.40 (2H, s), 7.10 (4H, s), 7.40 (1H, dd), 7.70-7.90 (2H, m) and 12.60 (1H, br s); m/z $(M+H)^{+\cdot}$ 281 (100% purity).

<u>Further derivatisation</u>

(a)

**164;** 4-hydroxy
Yield, 99%; mpt. 231-234°C; $\delta_H$ 4.15 (2H, s), 6.60 (2H, d, *J* = 8.0 Hz), 7.10 (2H, d, *J* = 8.0 Hz), 7.80-7.95 (3H, m), 8.25-8.45 (1H, m), 12.50 (1H, s); m/z $(M+H)^{+\cdot}$ 253 (100%).

**165;** 3-hydroxy
Yield, 68%; mpt. 198-201°C; $\delta_H$ 4.15 (2H, s), 6.50-6.80 (3H, m,), 6.95-7.10 (1H, m), 7.80-7.95 (3H, m), 8.25-8.45 (1H, m), 12.50 (1H, s); m/z $(M+H)^{+\cdot}$ 253 (100%).

(b)

**166;** R" = 4-NHC(=O)$CH_3$
Yield, 57%; mpt. 267-271°C; $\delta_H$ 2.00 (3H, s), 4.25 (2H, s), 7.25 (2H, d, *J* = 7.7 Hz), 7.55 (2H, d, *J* = 7.7 Hz), 7.75-7.95 (3H, m), 8.25-8.45 (1H, m), 9.80 (1H, s), 12.50 (1H, br s); m/z $(M+H)^{+\cdot}$ 294 (100%).

**167;** R" = 4-NHC(=O)Ph
Yield, 87%; mpt. 293-296°C; $\delta_H$ 4.25 (2H, s), 7.20-8.00 (12H, m), 8.25-8.45 (1H, m), 10.15 (1H, s), 12.50 (1H, s); m/z $(M+H)^{+\cdot}$ 356 (100%).

**169;** R" = 3-NHC(=O)-2-thienyl
Yield, 72%; mpt. 232-235°C; $\delta_H$ 4.25 (2H, s), 7.00-7.45 (3H, m), 7.55-7.65 (2H, m), 7.75-7.95 (5H, m), 8.25-8.45 (1H, m), 10.10 (1H, s), 12.50 (1H, br s); m/z $(M+H)^{+\cdot}$ 362 (100%).

**170;** R" = 3-NHC(=O)-4-fluoropheny
Yield, 68%; mpt. 257-261°C; $\delta_H$ 4.25 (2H, s), 7.00-7.50 (4H, m), 7.55-8.25 (8H, m), 10.15 (1H, s), 12.50 (1H, br s); m/z $(M+H)^{+\cdot}$ 374 (100%).

**171;** R" = 3-NHC(=O)Ph
Yield, 78%; mpt. 261-264°C; $\delta_H$ 4.25 (2H, s), 7.05-7.95 (12H, m), 10.05 (1H, s), 12.50 (1H, s); m/z $(M+H)^{+\cdot}$ 356 (100%).

**172;** R" = 3-NHC(=O)$CH_3$
Yield, 55%; mpt. 270-272°C; $\delta_H$ 2.00 (3H, s), 4.25 (2H, s), 7.00-7.50 (4H, m), 7.75 (3H, s), 8.25-8.45 (1H, m), 9.80 (1H, s), 12.50 (1H, br s); m/z $(M+H)^{+\cdot}$ 294 (100%).

**233;** R" = 3-NHC(=O)CH(Et)Ph
Yield, 82%; m.pt. 150-154°C; $\delta_H$ 0.90 (3H, t), 1.50-2.25 (2H, m), 3.20-3.55 (1H, m), 4.25 (2H, s), 7.0-7.90 (12H, m), 8.25-8.45 (1H, m), 9.95 (1H, br s) and 12.50 (1H, br s); m/z $(M+H)^{+}$. 398 (88% purity).

(c) (i)

**179;** Yield, 45%; mpt. 161-164°C; $\delta_H$ 2.15 (3H, s), 4.25 (2H, s), 6.90-7.35 (4H, m), 7.75-7.95 (3H, s), 8.25-8.45 (1H, m), 12.50 (1H, br s); m/z $(M+H)^{+\cdot}$ 295 (100%).

(c) (ii)

**212;** Yield, 55%; mpt. 184-187°C; $\delta_H$ 3.55 (2H, s), 7.10-7.50 (6H, m), 7.75-8.05 (3H, m), 8.10-8.45 (3H, m), 12.55 (1H, s).

(c) (iii)

**213;** Yield, 12%; mpt. 193-196°C; $\delta_H$ 2.20 (3H, s), 3.55 (2H, s), 7.10 (4H, dd, *J* = 8.2 Hz), 7.75-8.00 (3H, m), 8.25-8.45 (1H, m), 12.55 (1H, s).

(c) (iv)

**289;** $R^E$ = n-$C_9H_{19}$; m/z $(M+H)^{+\cdot}$ 407 (purity >95%)
**290;** $R^E$ = 1-phenylsulphonylindol-3-yl
**291;** $R^E$ = 4-(N,N-dipropylsulphamoyl)phenyl; m/z $(M+H)^{+\cdot}$ 520 (purity >95%)
**292;** $R^E$ = 2-(4-methoxyphenoxy)-5-nitrophenyl; m/z $(M+H)^{+\cdot}$ 524 (purity >95%)
**293;** $R^E$ = 4-(4-chlorophenylsulphonyl)-3-methyl-2-thienyl; m/z $(M+H)^{+\cdot}$ 551 (purity >95%)
**294;** $R^E$ = 5-(2,3-dihydrobenzo[b]furan-5-yl)-4-methylthiazol-5-yl; m/z $(M+H)^{+\cdot}$ 496 (purity >95%)
**295;** $R^E$ = 2-(2-thienyl)thiazol-4-yl; m/z $(M+H)^{+\cdot}$ 446 (purity >95%)
**296;** $R^E$ = 3-methyl-5-(5-methylisoxazol-3-yl)isoxazol-4-yl; m/z $(M+H)^{+\cdot}$ 443 (purity >95%)
**298;** $R^E$ = 5-(4-chlorophenyl)-2-(trifluoromethyl)-3-furyl; m/z $(M+H)^{+\cdot}$ 525 (purity >95%)
**299;** $R^E$ = 2-(4-methylphenylthio)-3-pyridyl
**300;** $R^E$ = quinoxalin-6-yl; m/z $(M+H)^{+\cdot}$ 409 (purity >85%)
**601;** $R^E$ = 2-chloro-3,4-dimethoxystyryl; m/z $(M+H)^{+\cdot}$ 477 (purity >90%)
**602;** $R^E$ = 5-phenyloxazol-4-yl; m/z $(M+H)^{+\cdot}$ 424 (purity >85%)
**603;** $R^E$ = 1-benzyloxycarbonylpiperid-4-yl; m/z $(M+H)^{+\cdot}$ 498 (purity >95%)
**604;** $R^E$ = 1-(4-methoxyphenyl)-5-methylpyrazol-4-yl; m/z $(M+H)^{+\cdot}$ 467 (purity >95%)
**605;** $R^E$ = 4-n-hexylphenyl; m/z $(M+H)^{+\cdot}$ 441 (purity >95%)
**607;** $R^E$ = 4-n-propylphenyl; m/z $(M+H)^{+\cdot}$ 399 (purity >95%)
**608;** $R^E$ = 6-fluoro-1,3-benzodioxan-8-yl; m/z $(M+H)^{+\cdot}$ 433 purity >95%)
**609;** $R^E$ = 2,4,5-trifluoro-3-methoxyphenyl; m/z $(M+H)^{+\cdot}$ 441 (purity >95%)
**610;** $R^E$ = cyclohexyl; m/z $(M+H)^{+\cdot}$ 363 (purity >95%)
**611;** $R^E$ = 4-bromo-1-ethyl-3-methylpyrazol-5-yl
**612;** $R^E$ = 2-chloro-4-pyridyl; m/z $(M+H)^{+\cdot}$ 392/394 (purity >95%)
**613;** $R^E$ = cyclopropyl; m/z $(M+H)^{+\cdot}$ 321 (purity >95%)
**614;** $R^E$ = 5-methyl-2-(trifluoromethyl)-3-furyl; m/z $(M+H)^{+\cdot}$ 429 (purity >95%)
**615;** $R^E$ = cyclobutyl; m/z $(M+H)^{+\cdot}$ 335 (purity >95%)
**616;** $R^E$ = 2-chloro-6-methyl-4-pyridyl
**617;** $R^E$ = 1-(4-chlorophenoxy)-1-methylethyl; m/z $(M+H)^{+\cdot}$ 446/451 (purity >95%)
**618;** $R^E$ = 2-thienyl; m/z $(M+H)^{+\cdot}$ 363 (purity >90%)
**619;** $R^E$ = 3,4-methylenedioxyphenyl; m/z $(M+H)^{+\cdot}$ 401 (purity >95%)
**620;** $R^E$ = *n*-heptyl; m/z $(M+H)^{+\cdot}$ 379 (purity >95%)
**621;** $R^E$ = 3-chloropropyl; m/z $(M+H)^{+\cdot}$ 357/359 (purity >95%)
**622;** $R^E$ = 5-methylisoxazol-3-yl; m/z $(M+H)^{+\cdot}$ 362 (purity >95%)
**623;** $R^E$ = 1-*t*-butyl-5-methylpyrazol-3-yl; m/z $(M+H)^{+\cdot}$ 417 (purity >90%)
**624;** $R^E$ = 3-phenylthiazol-4-yl; m/z $(M+H)^{+\cdot}$ 440 (purity >95%)
**625;** $R^E$ = 3-*t*-butyl-1-(2,4-dichlorobenzyl)pyrazol-5-yl
**626;** $R^E$ = 1-chloroethyl
**627;** $R^E$ = 3,4-dihydro-2*H*-1,5-benzodioxepin-7-yl
**628;** $R^E$ = 1-ethylpentyl; m/z $(M+H)^{+\cdot}$ 379 (purity >90%)
**629;** $R^E$ = 1-benzyloxycarbonyl-2,3-diydroindol-2-yl; m/z $(M+H)^{+\cdot}$ 532 (purity >90%)
**630;** $R^E$ = 2-chloro-1,1-dimethylethyl; m/z $(M+H)^{+\cdot}$ 371/373 (purity >95%)
**631;** $R^E$ = 1-propenyl; m/z $(M+H)^{+\cdot}$ 321 (purity >95%)

(d) (i)

**215;** Ar = phenyl
Yield, 31%; mpt. 254-257°C; $\delta_H$ 3.55 (2H, s), 6.80-7.50 (13H, m), 7.85 (1H, s), 9.55 (1H, s); m/z $(M+H)^{+\cdot}$ 371 (100%).
**216;** Ar = 4-fluorophenyl
Yield, 79%; mpt. 240-244°C; $\delta_H$ 3.55 (2H, s), 6.80-7.50 (12H, m), 8.50 (1H, s), 9.55 (1H, s); m/z $(M+H)^{+\cdot}$ 389 (100%).

(d) (ii)

**206;** Yield, 12%; mpt. 125-126.5°C; $\delta_H$ 2.65 (4H, br s), 3.70 (4H, br s), 4.20 (2H, s), 4.30 (4H, s), 7.00-7.15 (2H, m), 7.65-8.00 (3H, m), 8.25-8.45 (2H, m), 9.60 (1H, s), 12.55 (1H, s).

(d) (iii)

**640;** $R^A$ = cyclobutyl
Yield, 77%; m/z $(M+H)^{+\cdot}$ 334 (96% purity).
**641;** $R^A$ = 5-methyl-2-(trifluoromethyl)-3-furyl Yield, 50%; m/z $(M+H)^{+\cdot}$ 428 (97% purity).
**642;** $R^A$ = 4-bromo-1-ethyl-3-methylpyrazol-5-yl
Yield, 97%; m/z $(M+H)^{+\cdot}$ 466/468 (100% purity).
**643;** $R^A$ = 2-thienyl
Yield, 100%; m/z $(M+H)^{+\cdot}$ 362 (93% purity).
**644;** $R^A$ = 5-methylisoxazol-3-yl
Yield, 99%; m/z $(M+H)^{+\cdot}$ 361 (100% purity).
**645;** $R^A$ = 3,4-methylenedioxyphenyl
Yield, 100%; m/z $(M+H)^{+\cdot}$ 400 (94% purity).
**646;** $R^A$ = 1-*t*-butyl-5-methylpyrazol-3-yl
Yield, 96%; m/z $(M+H)^{+\cdot}$ 416 (97% purity).
**647;** $R^A$ = 1-ethylpentyl
Yield, 80%; m/z $(M+H)^{+\cdot}$ 378 (100% purity).
**648;** $R^A$ = *n*-heptyl
Yield, 76%; m/z $(M+H)^{+\cdot}$ 378 (100% purity).
**649;** $R^A$ = 1-chloroethyl
Yield, 65%; m/z $(M+H)^{+\cdot}$ 342/344 (100% purity).
**650;** $R^A$ = 1-propenyl
Yield, 91%; m/z $(M+H)^{+\cdot}$ 320 (97% purity).
**651;** $R^A$ = 3,4-dihydro-2*H*-1,5-benzodioxepin-7-yl
Yield, 93%; m/z $(M+H)^{+\cdot}$ 428 (97% purity).
**652;** $R^A$ = 2-chloro-6-methyl-4-pyridyl
Yield, 77%; m/z $(M+H)^{+\cdot}$ 405/407 (100% purity).
**653;** $R^A$ = 2-chloro-4-pyridyl
Yield, 87%; m/z $(M+H)^{+\cdot}$ 391/393 (100% purity).
**654;** $R^A$ = 1-(4-chlorophenoxy)-1-methylethyl
Yield, 89%; m/z $(M+H)^{+\cdot}$ 448/450 (100% purity).
**655;** $R^A$ = 4-(trifluoromethoxy)phenyl
Yield, 100%; m/z $(M+H)^{+\cdot}$ 440 (100% purity).
**656;** $R^A$ = cyclohexyl
Yield, 75%; m/z $(M+H)^{+\cdot}$ 362 (97% purity).
**657;** $R^A$ = 6-fluoro-1,3-benzodioxan-8-yl
Yield, 87%; m/z $(M+H)^{+\cdot}$ 432 (97% purity).
**658;** $R^A$ = 4-propylphenyl
Yield, 79%; m/z $(M+H)^{+\cdot}$ 398 (100% purity).
**659;** $R^A$ = 2,4,5-trifluoro-3-methoxyphenyl
Yield, 83%; m/z $(M+H)^{+\cdot}$ 440 (100% purity).
**667;** $R^A$ = 2-chloro-3,4-dimethoxystyryl
Yield, 76%; m/z $(M+H)^{+\cdot}$ 476/478 (100% purity).
**668;** $R^A$ = 4-hexylphenyl
Yield, 63%; m/z $(M+H)^{+\cdot}$ 440 (100% purity).
**669;** $R^A$ = 2-(4-methylphenoxy)-3-pyridyl

Yield, 41%; m/z $(M+H)^{+\cdot}$ 163 (97% purity).

**670;** $R^A$ = 2-(4-methylphenylthio)-3-pyridyl
Yield, 100%; m/z $(M+H)^{+\cdot}$ 479 (88% purity).

**671;** $R^A$ = quinoxalin-6-yl
Yield, 86%; m/z $(M+H)^{+\cdot}$ 408 (100% purity).

**672;** $R^A$ = 1-benzyloxycarbonylpiperid-4-yl
Yield, 84%; m/z $(M+H)^{+\cdot}$ 497 (95% purity).

**673;** $R^A$ = 1-(4-methoxyphenyl)-5-methylpyrazol-4-yl
Yield, 76%; m/z $(M+H)^{+\cdot}$ 466 (97% purity).

**674;** $R^A$ = 5-(2-pyridyl)-2-thienyl
Yield, 66%; m/z $(M+H)^{+\cdot}$ 439 (100% purity).

**675;** $R^A$ = 5-phenyloxazol-4-yl
Yield, 63%; m/z $(M+H)^{+\cdot}$ 423 (100% purity).

**676;** $R^A$ = 3-methyl-5-(5-methylisoxazol-3-yl)isoxazol-4-yl
Yield, 49%; m/z $(M+H)^{+\cdot}$ 442 (100% purity).

**677;** $R^A$ = 2 (2-thienyl)isothiazol-4-yl
Yield, 70%; m/z $(M+H)^{+\cdot}$ 445 (100% purity).

**678;** $R^A$ = 2-(2,3-dihydrobenzo[*b*]furan-5-yl)-4-methylthiazol-5-yl
Yield, 96%; m/z $(M+H)^{+\cdot}$ 495 (100% purity).

**679;** $R^A$ = 5-(4-chlorophenyl)-2-(trifluoromethyl)-3-furyl Yield, 95%; m/z $(M+H)^{+\cdot}$ 524/526 (98% purity).

**680;** $R^A$ = 3,5-*bis*-(trifluorcmethyl)phenyl
Yield, 69%; m/z $(M+H)^{+\cdot}$ 492 (100% purity).

**681;** $R^A$ = 4-(4-chlorophenylsulphonyl)-3-methyl-2-thienyl
Yield, 45%; m/z $(M+H)^{+\cdot}$ 550/552 (100% purity).

**682;** $R^A$ = 1-benzyl-3-*t*-butylpyrazol-5-yl
Yield, 94%; m/z $(M+H)^{+\cdot}$ 492 (98% purity).

**683;** $R^A$ = 1-phenylsulphonylindol-3-yl
Yield, 90%; m/z $(M+H)^{+\cdot}$ 535 (100% purity).

**684;** $R^A$ = *n*-nonyl
Yield, 75%; m/z $(M+H)^{+\cdot}$ 406 (94% purity).

**685;** $R^A$ = 2-(4-methoxyphenoxy)-5-nitrophenyl
Yield, 98%; m/z $(M+H)^{+\cdot}$ 523 (100% purity).

**686;** $R^A$ = propyl
Yield, 56%; m/z $(M+H)^{+\cdot}$ 322 (92% purity).

**687;** $R^A$ = ethyl
Yield, 68%; m/z $(M+H)^{+\cdot}$ 308 (97% purity).

**688;** $R^A$ = 1-methylethyl
Yield, 100%; m/z $(M+H)^{+\cdot}$ 322 (100% purity).

(d) (iv)

**691;** R = 6-fluoro-1,3-benzodioxan-8-yl
Yield, 100%; m.pt. 250-254°C (shrinks 142-146°C) ; m/z $(M+H)^{+\cdot}$ 447 (100% purity). **692;** R = 3,4-dihydro-2*H*-1,5-benzodioxepin-7-yl
Yield, 71%; m.pt. 205-208°C; m/z $(M+H)^{+\cdot}$ 443 (100% purity).

**693;** R = 1-benzyloxycarbonylpiperid-4-yl
Yield, 78%; m.pt. 216-219°C; m/z $(M+H)^{+\cdot}$ 512 (100% purity).

**694;** R = propyl
Yield, 75%; m.pt. 205-208°C; $\delta_H$ 0.80 (3H, t), 1.2-1.7 (2H, m), 3.0 (2H, q), 4.20 (2H, s), 6.0 (1H, br s), 6.8-7.3 (4H, m), 7.7-7.95 (3H, m), 8.3-8.45 (2H, m) and 12.55 (1H, s); m/z $(M+H)^{+\cdot}$ 337 (100% purity).

**698;** R = 2,3-dihydrobenzo[*b*]furan-5-yl
Yield, 88%; m.pt. 251-254°C; $\delta_H$ 3.1 (2H, t), 4.25 (2H, s), 4.5 (2H, t), 6,6 (1H, d), 6.8-6.4 (6H, m), 7.7-7.9 (3H, m), 8.2-8.4 (2H, m), 9.0 (1H, s) and 12.55 (1H, s); m/z $(M+H)^{+\cdot}$ 413 (94% purity).

**699;** R = 3-methoxyphenyl
Yield, 67%; m.pt. 195-200°C; $\delta_H$ 3.7 (3H, s), 4.25 (2H, s), 6.4-6.6 (1H, m), 6.8-7.3 (7H, m), 9.7-9.9 (3H, m), 8.2-8.4 (1H, m), 8.6-8.7 (2H, m) and 12.25 (1H, s); m/z $(M+H)^{+\cdot}$ 401 (100% purity).

**700;** R = 2-(trifluoromethoxy)phenyl
Yield, 84%; m.pt. 229-231°C; $\delta_H$ 4.25 (2H, s), 6.9-7.3 (7H, m), 9.7-9.9 (3H, m), 8.2-8.4 (3H, m) 11.25 (1H, s) and 12.25

(1H, s); m/z $(M+H)^{+\cdot}$ 455 (100% purity).

**701;** R = 5-methyl-3-phenylisoxazol-4-yl
Yield, 39%; m.pt. 256-258°C; m/z $(M+1)^{+\cdot}$ 452 (97% purity).

**704;** R = 2-ethoxyphenyl
Yield, 77%; m.pt. 174-178°C; m/z $(M+H)^{+\cdot}$ 415 (100% purity).

**705;** R = 4-butylphenyl
Yield, 78%; m.pt. 201-205°C; $\delta_H$ 0.80 (3H, t), 1.0-1.8 (4H, m), 2.5 (2H, m), 4.25 (2H, s), 6.9-7.4 (8H, m), 9.7-9.9 (3H, m), 8.2-8.6 (3H, m) and 12.25 (1H, s); m/z $(M+H)^{+\cdot}$ 427 (100% purity).

**706;** R = butyl
Yield, 65%; m.pt. 225-227°C; m/z $(M+H)^{+\cdot}$ 351 (96% purity).

(d) (v) (a)

**697;** R = methyl
Yield, 78%; m.pt. 192-194°C; m/z $(M+H)^{+\cdot}$ 330 (100% purity).

(d) (v) (b)

**702;** R = 4-acetamidophenyl
Yield, 67%; m.pt. 263-265°C; m/z $(M+H)^{+\cdot}$ 449 (97% purity).

**703;** R = 5-(2-pyridyl)-2-thienyl
Yield, 80%; m.pt. 258-261°C; m/z $(M+H)^{+\cdot}$ 475 (100% purity).

(e)

**227;** Yield, 31%; mpt. 124-125. 5°C; $\delta_H$ 4.25 (2H, s), 7.20-7.50 (4H, m), 7.60-8.45 (8H, m), 10.20 (1H, m), 12.55 (1H, m); m/z $(M-H)^{+\cdot}$ 372 (20%).

(f)

**239;** Yield, 68%; mpt. 230-232°C; $\delta_H$ 3.65 (2H, s), 4.10 (3H, s), 6.50-7.00 (3H, m), 7.75-8.00 (3H, m), 8.25-8.45 (1H, m), 8.50-9.00 (1H, br s), 12.50 (1H, br s); m/z $(M+H)^{+\cdot}$ 283 (100%).

(g)

**247;** Yield, 89%; mpt. 228-231°C; $\delta_H$ 4.25 (2H, s), 6.60-7.05 (3H, m), 7.80-8.00 (3H, m), 8.25-8.45 (1H, m), 9.80 (1H, s), 12.55 (1H, s); m/z $(M+H)^{+\cdot}$ 271 (65%).

(h)

**277;** Yield, 36%; m.pt. 157-162°C; $\delta_H$ 3.80 (3H, s), 4.40 (2H, s), 7.3-8.0 (7H, m), 8.2-8.4 (1H, m) and 12.60 (1H, s).

(i)

**278;** Yield, 6%; m.pt. 131-139°C; $\delta_H$ 4.40 (2H, s), 7.3-7.9 (12H, m), 8.2-8.4 (1H, m), 10.20 (1H, s) and 12.60 (1H, s); m/z $(M+H)^{+\cdot}$ 356 (79% purity).

(j)

**253;** $R^{N1}R^{N2}NH$ = morpholine
Yield, 19% over 2 stages; m.pt. 118-120°C; $\delta_H$ 2.3-2.6 (4H, m), 2.7-2.9 (4H, m), 3.4-3.9 (10H, m) 4.35 (2H, s), 7.0-8.3 (11H, m) 9.70 (1H, s) and 12.30 (1H, s) ; m/z $(M+H)^{+\cdot}$ 540 (95% purity).

**254;** $R^{N1}R^{N2}NH$ = pyrrolidine
Yield, 42% over 2 stages; m.pt. 110-113°C; $\delta_H$ 1.6-1.8 (4H, m), 2.3-2.6 (4H, m), 2.7-2.9 (4H, m), 3.6-3.9 (6H, m), 4.35 (2H, s), 7.2-8.3 (11H, m) 9.70 (1H, s) and 12.60 (1H, s).

(k)

**265;**

Yield, 46%; m.pt. decomposes >75°C; $\delta_H$ 1.9-2.2 (2H, m), 2.7-2.85 (4H, m), 3.5-3.9 (8H, m), 4.20 (2H, s), 7.0 (1H, s), 7.7-8.2 (6H, m), 8.80 (1H, s) and 12.50 (1H, s).

Biological Testing

In order to assess the inhibitory action of the compounds, the following assay was used to determine $IC_{50}$ values.

Mammalian PARP, isolated from Hela cell nuclear extract, was incubated with Z-buffer (25mM Hepes (Sigma); 12.5 mM $MgCl_2$ (Sigma); 50mM KCl (Sigma); 1 mM DTT (Sigma); 10% Glycerol (Sigma) 0.001% NP-40 (Sigma); pH 7.4) in 96 well FlashPlates (TRADE MARK) (NEN, UK) and varying concentrations of said inhibitors added. All compounds were diluted in DMSO and gave final assay concentrations of between 10 and 0.01 μM, with the DMSO being at a final concentration of 1% per well. The total assay volume per well was 40 μl.

After 10 minutes incubation at 30°C the reactions were initiated by the addition of a 10 μl reaction mixture, containing NAD (5μM), $^3$H-NAD and 30mer double stranded DNA-oligos. Designated positive and negative reaction wells were done in combination with compound wells (unknowns) in order to calculate % enzyme activities. The plates were then shaken for 2 minutes and incubated at 30°C for 45 minutes.

Following the incubation, the reactions were quenched by the addition of 50 μl 30% acetic acid to each well. The plates were then shaken for 1 hour at room temperature.

The plates were transferred to a TopCount NXT (TRADE MARK) (Packard, UK) for scintillation counting. Values recorded are counts per minute (cpm) following a 30 second counting of each well.

The % enzyme activity for each compound is then calculated using the following equation:

$$\%\ \text{Inhibition} = 100 - \left(100\text{x}\frac{(\text{cpm of unknowns} - \text{mean negative cpm})}{(\text{mean positive cpm} - \text{mean neagative cpm})}\right)$$

Some results are detailed below in Table 1 as $IC_{50}$ values (the concentration at which 50% of the enzyme activity is inhibited), which are determined over a range of different concentrations, normally from 10 μM down to 0.01 μM. Such $IC_{50}$ values are used as comparative values to identify increased compound potencies.

For comparison, the $IC_{50}$ of 100 (1(2H)-phthalazinone was determined using the above test to be 7.2 μM.

Table 1

| Compound | $IC_{50}$ (μM) |
|---|---|
| 126 | 1.8 |
| 129 | 1.6 |
| 132 | 0.7 |
| 141 | 1.4 |
| 151 | 1.8 |
| 186 | 1.1 |
| 191 | 1.3 |
| 211 | 0.4 |
| 248 | 1.0 |
| 139 | 1.7 |
| 163 | 1.8 |
| 192 | 1.4 |

(continued)

| Compound | $IC_{50}$ (μM) |
|---|---|
| 138 | 1.2 |
| 142 | 0.7 |
| 193 | 1.0 |
| 194 | 1.4 |
| 164 | 1.8 |
| 165 | 0.3 |
| 276 | 2.5 |
| 277 | 0.6 |
| 159 | 3.5 |
| 160 | 1.3 |
| 166 | 4.1 |
| 167 | 1.6 |
| 227 | 0.4 |
| 169 | 0.6 |
| 170 | 0.4 |
| 171 | 0.6 |
| 172 | 0.09 |
| 215 | 4.0 |
| 216 | 0.3 |
| 206 | 1.2 |
| 179 | 0.04 |
| 212 | 0.9 |
| 213 | 4.4 |
| 239 | 0.6 |
| 180 | 1.3 |
| 222 | 2.2 |
| 247 | 0.5 |
| 241 | 0.9 |
| 198 | 3.8 |
| 204 | 0.7 |
| 202 | 0.07 |
| 131 | 4.4 |
| 177 | 0.8 |
| 178 | 0.2 |
| 249 | 0.7 |
| 145 | 0.8 |

The following compounds were tested and had $IC_{50}$s of less than, or equal to, 1 μM: 233, 278, 279, 294, 295, 601, 604, 624, 640-659, 667-678 and 680-706.

The following compounds were tested and had $IC_{50}$s of less than, or equal to, 3 $\mu$M: 253, 254, 265 and 619.

The Dose Enhancing Factor (DEF) is a ratio of the enhancement of cell growth inhibition elicited by the test compound in the presence of bleomycin compared to bleomycin alone. The test compounds were used at a fixed concentration of 25 $\mu$M. Bleomycin was used at a concentration of 0.5 $\mu$g/ml. The DEF was calculated from the formula:

$$\frac{Growth_{TC}}{Growth_{Control}} \times \frac{Growth_{bleo}}{Growth_{(bleo+TC)}}$$

where $Growth_{TC}$ is cell growth in presence of the test compound;
$Growth_{Control}$ is cell growth of control cells;
$Growth_{bleo}$ is cell growth in presence of bleomycin; and $Growth_{(bleo+TC)}$ is cell growth in presence of bleomycin and the test compound.

Cell growth was assessed using the sulforhodamine B (SRB) assay (Skehan, P., et al., 1990, J. Natl. Cancer Inst., 82, 1107-1112). 2,000 HeLa cells were seeded into each well of a flat-bottomed 96-well microtiter plate in a volume of 100 $\mu$l and incubated for 6 hours at 37°C. Cells were either replaced with media alone or with media containing the test compound at a final concentration of 25 $\mu$M. Cells were allowed to grow for a further 1 hour before the addition of bleomycin to either untreated cells or test compound treated cells. Cells untreated with either bleomycin or test compound were used as a control. Cells treated with test compound alone were used to assess the growth inhibition by the test compound.

Cells were left for a further 16 hours before replacing the media and allowing the cells to grow for a further 72 hours at 37°C. The media was then removed and the cells fixed with 100$\mu$l of ice cold 10% (w/v) trichloroacetic acid. The plates were incubated at 4°C for 20 minutes and then washed four times with water. Each well of cells was then stained with 100$\mu$l of 0.4% (w/v) SRB in 1% acetic acid for 20 minutes before washing four times with 1% acetic acid. Plates were then dried for 2 hours at room temperature. The dye from the stained cells was solubilized by the addition of 100$\mu$l of 10mM Tris Base into each well. Plates were gently shaken and left at room temperature for 30 minutes before measuring the optical density at 564nM on a Microquant microtiter plate reader.

Some results are shown in Table 2.

Table 2

| Compound | DEF |
|---|---|
| 126 | 1.9 |
| 129 | 1.3 |
| 132 | 2.3 |
| 141 | 1.6 |
| 151 | 1.9 |
| 186 | 1.6 |
| 191 | 1.5 |
| 211 | 1.4 |
| 248 | 1.2 |
| 139 | 1.4 |
| 163 | 1.3 |
| 192 | 1.4 |
| 138 | 1.3 |
| 142 | 1.6 |
| 193 | 2.2 |
| 194 | 1.6 |

(continued)

| Compound | DEF |
|---|---|
| 164 | 1.3 |
| 165 | 1.3 |
| 160 | 1.5 |
| 166 | 1.4 |
| 227 | 2.6 |
| 169 | 1.5 |
| 170 | 2.6 |
| 171 | 1.8 |
| 172 | 1.4 |
| 215 | 1.4 |
| 216 | 1.3 |
| 206 | 1.2 |
| 179 | 1.4 |
| 212 | 2.3 |
| 213 | 1.3 |
| 180 | 1.2 |
| 222 | 1.2 |
| 198 | 2.3 |
| 204 | 1.6 |
| 131 | 1.3 |
| 177 | 1.2 |
| 178 | 1.9 |
| 145 | 1.8 |

The following compounds were tested and had DEFs of greater than 1: 233, 249, 254, 265, 278, 279, 640, 645, 648-654, 655-658, 667, 671, 672, 678, 680, 683, 684 and 686-688.

## Claims

**1.** The use of a compound of the formula:

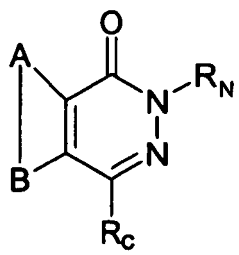

or isomers i.e. geometric, optical, entianomeric, diasteriomeric, epimeric, stereoisomeric, tautomeric, conformational, or anomeric forms, salts, solvates and chemically protected forms thereof, in the preparation of a medicament for

inhibiting the activity of PARP, wherein:

A and B together represent a fused aromatic ring, optionally substituted with one or more substituent groups selected from halo, nitro, hydroxy, ether, thiol, thioether, amino, $C_{1-7}$ alkyl, $C_{3-20}$ heterocyclyl and $C_{5-20}$ aryl;
$R_C$ is $-CH_2-R_L$, where $R_L$ is a $C_{5-20}$ aryl group, optionally substituted with one or more substituent groups selected from $C_{1-7}$ alkyl, $C_{5-20}$ aryl, $C_{3-20}$ heterocyclyl, halo, hydroxy, ether, nitro, cyano, acyl, carboxy, ester, amido, amino, sulfonamido, acylamido, ureido, acyloxy, thiol, thioether, sulfoxide and sulfone; and
$R_N$ is hydrogen.

**2.** The use of a compound of the formula:

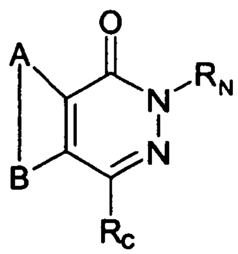

or isomers i.e. geometric, optical, entianomeric, diasteriomeric, epimeric, stereoisomeric, tautomeric, conformational, or anomeric forms, salts, solvates and chemically protected forms thereof, in the preparation of a medicament for use as an adjunct in cancer therapy, wherein:

A and B together represent a fused aromatic ring, optionally substituted with one or more substituent groups selected from halo, nitro, hydroxy, ether, thiol, thioether, amino, $C_{1-7}$ alkyl, $C_{3-20}$ heterocyclyl and $C_{5-20}$ aryl;
$R_c$ is $-CH_2-R_L$, where $R_L$ is a $C_{5-20}$ aryl group, optionally substituted with one or more substituent groups selected from $C_{1-7}$ alkyl, $C_{5-20}$ aryl, $C_{3-20}$ heterocyclyl, halo, hydroxy, ether, nitro, cyano, acyl, carboxy, ester, amido, amino, sulfonamido, acylamido, ureido, acyloxy, thiol, thioether, sulfoxide and sulfone; and
$R_N$ is hydrogen.

**3.** The use according to claim 2, wherein the adjunct is for use in combination with ionizing radiation.

**4.** The use according to claim 2, wherein the adjunct is for use in combination with chemotherapeutic agents.

**5.** The use according to any one of claims 1 to 4, wherein the fused aromatic ring(s) represented by -A-B- consists of solely carbon ring atoms.

**6.** The use according to claim 5, wherein the fused aromatic ring represented by -A-B- is benzene.

**7.** The use according to either claim 5 or claim 6, wherein said rings are unsubstituted.

**8.** The use according to any one of the preceding claims, wherein $R_L$ is a benzene ring.

**9.** The use according to claim 8, wherein $R_L$ is substituted by one or more substituents selected from the group consisting of: $C_{1-7}$ alkyl; $C_{5-20}$ aryl; $C_{3-20}$ heterocyclyl; halo; hydroxy; ether; nitro; cyano; carbonyl groups; amino; sulfonamido; acylamido; ureido; acyloxy; thiol; thioether; sulfoxide; and sulfone.

**10.** The use according to claim 9, wherein $R_L$ is substituted by a substituent selected from the group consisting of:

acylamido, ureido, sulfonamido, and acyloxy.

**11.** A pharmaceutical composition comprising a compound of the formula:

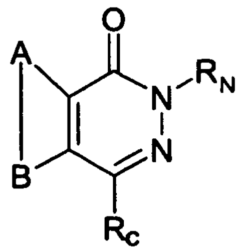

or isomers i.e. geometric, optical, enantiomeric, diasteriomeric, epimeric, stereoisomeric, tautomeric, conformational, or anomeric forms, salts, solvates and chemically protected forms thereof, wherein:

A and B together represent a fused aromatic ring, optionally substituted with one or more substituent groups selected from halo, nitro, hydroxy, ether, thiol, thioether, amino, $C_{1-7}$ alkyl, $C_{3-20}$ heterocyclyl and $C_{5-20}$ aryl;
$R_C$ is $-CH_2-R_L$;
$R_L$ is phenyl optionally substituted with one or more substituent groups selected from $C_{1-7}$ alkyl, $C_{5-20}$ aryl, $C_{3-20}$ heterocyclyl, halo, hydroxy, ether, nitro, cyano, carboxy, ester, amido, amino, sulfonamido, acylamido, ureido, acyloxy, thiol, thioether, sulfoxide and sulfone; and
$R_N$ is hydrogen,
and a pharmaceutically acceptable carrier or diluent.

**12.** A pharmaceutical composition according to claim 11, wherein the fused aromatic ring(s) represented by -A-B- consists of solely carbon ring atoms.

**13.** A pharmaceutical composition according to claim 12, wherein the fused aromatic ring represented by -A-B- is benzene.

**14.** A pharmaceutical composition according to either claim 12 or claim 13, wherein said rings are unsubstituted.

**15.** A pharmaceutical composition according to any one of claims claim 11 to 14, wherein $R_L$ is substituted by one or more substituents selected from the group consisting of:

$C_{1-7}$ alkyl; $C_{5-20}$ aryl; $C_{3-20}$ heterocyclyl; halo; hydroxy; ether; nitro; cyano; carbonyl groups; amino; sulfonamido; acylamido; ureido; acyloxy; thiol; thioether; sulfoxide; and sulfone .

**16.** A pharmaceutical composition according to claim 15, wherein $R_L$ is substituted by a substituent selected from the group consisting of: acylamido, ureido, sulfonamido, and acyloxy.

**17.** A compound of the formula:

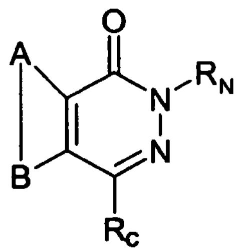

or isomers i.e. geometric, optical, enantiomeric, diasteriomeric, epimeric, stereoisomeric, tautomeric, conformational, or anomeric forms, salts, solvates and chemically protected forms thereof, wherein:

A and B together represent a fused aromatic ring, optionally substituted with one or more substituent groups selected from halo, nitro, hydroxy, ether, thiol, thioether, amino, $C_{1-7}$ alkyl, $C_{3-20}$ heterocyclyl and $C_{5-20}$ aryl;
$R_C$ is $-CH_2-R_L$;
$R_L$ is phenyl optionally substituted with one or more substituent groups selected from $C_{1-7}$ alkyl, $C_{5-20}$ aryl, $C_{3-20}$ heterocyclyl, halo, hydroxy, ether, nitro, cyano, carboxy, ester, amido, amino, sulfonamido, acylamido, ureido, acyloxy, thiol, thioether, sulfoxide and sulfone; and
$R_N$ is hydrogen,
for use in a method of treatment of the human or animal body.

**18.** A compound according to claim 17, wherein
the fused aromatic ring(s) represented by -A-B- consists of solely carbon ring atoms.

**19.** A compound according to claim 18, wherein the fused aromatic ring represented by -A-B- is benzene.

**20.** A compound according to either claim 18 or claim 19, wherein said rings are unsubstituted.

**21.** A compound according to any one of claims 17 to 20, wherein $R_L$ is substituted by one or more substituents selected from the group consisting of: $C_{1-7}$, alkyl; $C_{5-20}$ aryl; $C_{3-20}$ heterocyclyl; halo; hydroxy; ether; nitro; cyano; carbonyl groups; amino; sulfonamido; acylamido; ureido; acyloxy; thiol; thioether; sulfoxide; and sulfone.

**22.** A compound according to claim 21, wherein $R_L$ is substituted by a substituent selected from the group consisting of: acylamido, ureido, sulfonamido, and acyloxy.

**23.** A compound of the formula:

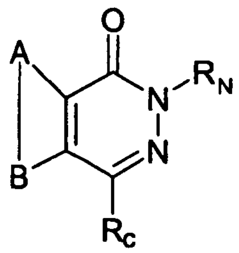

or an isomer i.e. geometric, optical, enatiomeric, diasteriomeric, epimeric, stereoisomeric, tautomeric, conformational, or anomeric form, salt, solvate and chemically protected forms thereof, wherein:

A and B together represent a fused aromatic ring, optionally substituted with one substituent group selected from halo, nitro, hydroxy, ether, thiol, thioether, amino, $C_{1-7}$ alkyl, $C_{3-20}$ heterocyclyl and $C_{5-20}$ aryl;
$R_C$ is $-CH_2-R_L$;
$R_L$ is phenyl substituted by a substituent selected from the group consisting of: $C_{3-20}$ heterocyclyl; amido; ureido; sulfonamido; and acyloxy and optionally further substituted by a group selected from $C_{1-7}$ alkyl, $C_{5-20}$ aryl, $C_{3-20}$ heterocyclyl, halo, hydroxy, ether, nitro, cyano, carboxy, ester, amido, amino, sulfonamide, acylamido, ureido, acyloxy, thiol, thioether, sulfoxide and sulfone; and
$R_N$ is hydrogen.

## Patentansprüche

**1.** Verwendung einer Verbindung der Formel:

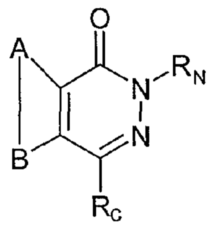

oder von Isomeren (d.h. geometrischen, optischen, enantiomeren, diastereoisomeren, epimeren, stereoisomeren, tautomeren, konformeren oder anomeren Formen), Salzen, Solvaten und chemisch geschützten Formen davon zur Herstellung eines Medikaments, das die Aktivität voin PARP hemmt, worin:

A und B gemeinsam für einen kondensierten aromatischen Ring stehen, der gegebenenfalls mit einer oder mehreren, aus Halogen, Nitro, Hydroxy, Ether, Thiol, Thioether, Amino, $C_{1-7}$-Alkyl, $C_{3-20}$-Heterocyclyl und $C_{5-20}$-Aryl ausgewählten Substituentengruppen substituiert ist;
$R_C$ -$CH_2$-$R_L$ ist, worin $R_L$ eine $C_{5-20}$-Arylgruppe ist, die gegebenenfalls mit einer oder mehreren aus $C_{1-7}$-Alkyl, $C_{5-20}$-Aryl, $C_{3-20}$-Heterocyclyl, Halogen, Hydroxy, Ether, Nitro, Cyano, Acyl, Carboxy, Ester, Amido, Amino, Sulfonamido, Acylamido, Ureido, Acyloxy, Thiol, Thioether, Sulfoxid und Sulfon ausgewählten Substituentengruppen substituiert ist; und
$R_N$ Wasserstoff ist.

**2.** Verwendung einer Verbindung der Formel:

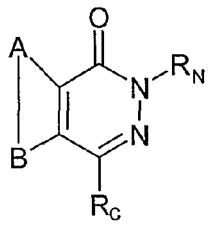

oder von Isomeren (d.h. geometrischen, optischen, enantiomeren, diastereoisomeren, epimeren, stereoisomeren, tautomeren, konformeren oder anomeren Formen), Salzen, Solvaten und chemisch geschützten Formen davon zur Herstellung eines Medikaments zur Verwendung als Zusatz in der Krebstherapie, worin:

A und B gemeinsam für einen kondensierten aromatischen Ring stehen, der gegebenenfalls mit einer oder mehreren aus Halogen, Nitro, Hydroxy, Ether, Thiol, Thioether, Amino, $C_{1-7}$-Alkyl, $C_{3-20}$-Heterocyclyl und $C_{5-20}$-Aryl ausgewählten Substituentengruppen substituiert ist;
$R_C$ -$CH_2$-$R_L$ ist, worin $R_L$ eine $C_{5-20}$-Arylgruppe ist, die gegebenenfalls mit einer oder mehreren aus $C_{1-7}$-Alkyl, $C_{5-20}$-Aryl, $C_{3-20}$-Heterocyclyl, Halogen, Hydroxy, Ether, Nitro, Cyano, Acyl, Carboxy, Ester, Amido, Amino, Sulfonamido, Acylamido, Ureido, Acyloxy, Thiol, Thioether, Sulfoxid und Sulfon ausgewählten Substituentengruppen substituiert ist; und
$R_N$ Wasserstoff ist.

**3.** Verwendung nach Anspruch 2, worin der Zusatz zur Verwendung in Kombination mit ionisierender Strahlung dient.

**4.** Verwendung nach Anspruch 2, worin der Zusatz zur Verwendung in Kombination mit chemotherapeutischen Mitteln dient.

**5.** Verwendung nach einem der Ansprüche 1 bis 4, worin der kondensierte aromatische Ring/die kondensierten aromatischen Ringe, der/die durch -A-B- dargestellt ist/sind, nur aus Kohlenstoff-Ringatomen besteht/bestehen.

**6.** Verwendung nach Anspruch 5, worin der durch -A-B- dargestellte kondensierte aromatische Ring Benzol ist.

**7.** Verwendung nach Anspruch 5 oder 6, worin die Ringe unsubstituiert sind.

**8.** Verwendung nach einem der vorangegangenen Ansprüche, worin $R_L$ ein Benzolring ist.

**9.** Verwendung nach Anspruch 8, worin $R_L$ mit einem oder mehreren Substituenten substituiert ist, die aus der aus Folgendem bestehenden Gruppe ausgewählt sind: $C_{1-7}$-Alkyl, $C_{5-20}$-Aryl, $C_{3-20}$-Heterocyclyl, Halogen, Hydroxy, Ether, Nitro, Cyano, Carbonylgruppen, Amino, Sulfonamido, Acylamido, Ureido, Acyloxy, Thiol, Thioether, Sulfoxid und Sulfon.

**10.** Verwendung nach Anspruch 9, worin $R_L$ mit einem aus der aus Acylamido, Ureido, Sulfonamido und Acyloxy bestehenden Gruppe ausgewählten Substituenten substituiert ist.

**11.** Pharmazeutische Zusammensetzung, die eine Verbindung der Formel:

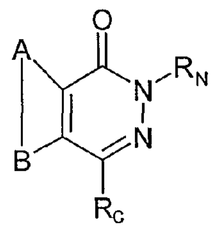

oder Isomere (d.h. geometrische, optische, enantiomere, diastereoisomere, epimere, stereoisomere, tautomere, konformere oder anomere Formen), Salze, Solvate und chemisch geschützte Formen davon, worin:

A und B gemeinsam für einen kondensierten aromatischen Ring stehen, der gegebenenfalls mit einer oder mehreren aus Halogen, Nitro, Hydroxy, Ether, Thiol, Thioether, Amino, $C_{1-7}$-Alkyl, $C_{3-20}$-Heterocyclyl und $C_{5-20}$-Aryl ausgewählten Substituentengruppen substituiert ist;
$R_C$ $-CH_2-R_L$ ist, worin $R_L$ eine $C_{5-20}$-Arylgruppe ist, die gegebenenfalls mit einer oder mehreren aus $C_{1-7}$-Alkyl, $C_{5-20}$-Aryl, $C_{3-20}$-Heterocyclyl, Halogen, Hydroxy, Ether, Nitro, Cyano, Acyl, Carboxy, Ester, Amido, Amino, Sulfonamido, Acylamido, Ureido, Acyloxy, Thiol, Thioether, Sulfoxid und Sulfon ausgewählten Substituentengruppen substituiert ist; und
$R_N$ Wasserstoff ist;

und einen pharmazeutischen annehmbaren Träger oder ein pharmazeutisch annehmbares Verdünnungsmittel umfasst.

**12.** Pharmazeutische Zusammensetzung nach Anspruch 11, worin der kondensierte aromatische Ring/die kondensierten aromatischen Ringe, der/die durch -A-B-dargestellt ist/sind, nur aus Kohlenstoff-Ringatomen besteht/bestehen.

**13.** Pharmazeutische Zusammensetzung nach Anspruch 12, worin der durch -A-B- dargestellte kondensierte aromatische Ring Benzol ist.

**14.** Pharmazeutische Zusammensetzung nach Anspruch 12 oder 13, worin die Ringe unsubstituiert sind.

**15.** Pharmazeutische Zusammensetzung nach einem der Ansprüche 11 bis 14, worin $R_L$ mit einem oder mehreren Substituenten substituiert ist, die aus der aus Folgendem bestehenden Gruppe ausgewählt sind: $C_{1-7}$-Alkyl, $C_{5-20}$-Aryl, $C_{3-20}$-Heterocyclyl, Halogen, Hydroxy, Ether, Nitro, Cyano, Carbonylgruppen, Amino, Sulfonamido, Acylamido, Ureido, Acyloxy, Thiol, Thioether, Sulfoxid und Sulfon.

**16.** Pharmazeutische Zusammensetzung nach Anspruch 15, worin $R_L$ mit einem aus der aus Acylamido, Ureido, Sulfonamido und Acyloxy bestehenden Gruppe ausgewählten Substituenten substituiert ist.

**17.** Verbindung der Formel:

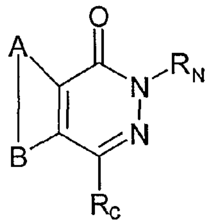

oder Isomere (d.h. geometrische, optische, enantiomere, diastereoisomere, epimere, stereoisomere, tautomere, konformere oder anomere Formen), Salze, Solvate und chemisch geschützte Formen davon, worin:

A und B gemeinsam für einen kondensierten aromatischen Ring stehen, der gegebenenfalls mit einer oder mehreren aus Halogen, Nitro, Hydroxy, Ether, Thiol, Thioether, Amino, $C_{1-7}$-Alkyl, $C_{3-20}$-Heterocyclyl und $C_{5-20}$-Aryl ausgewählten Substituentengruppen substituiert ist;
$R_C$ -$CH_2$-$R_L$ ist;
$R_L$ Phenyl ist, das gegebenenfalls mit einer oder mehreren aus $C_{1-7}$-Alkyl, $C_{5-20}$-Aryl, $C_{3-20}$-Heterocyclyl, Halogen, Hydroxy, Ether, Nitro, Cyano, Acyl, Carboxy, Ester, Amido, Amino, Sulfonamido, Acylamido, Ureido, Acyloxy, Thiol, Thioether, Sulfoxid und Sulfon ausgewählten Substituentengruppen substituiert ist; und
$R_N$ Wasserstoff ist;

zur Verwendung in einem Verfahren zur Behandlung des menschlichen oder tierischen Körpers.

**18.** Verbindung nach Anspruch 17, worin der kondensierte aromatische Ring/die kondensierten aromatischen Ringe, der/die durch -A-B- dargestellt ist/sind, nur aus Kohlenstoff-Ringatomen besteht/bestehen.

**19.** Verbindung nach Anspruch 18, worin der durch -A-B- dargestellte kondensierte aromatische Ring Benzol ist.

**20.** Verbindung nach Anspruch 18 oder 19, worin die Ringe unsubstituiert sind.

**21.** Verbindung nach einem der Ansprüche 17 bis 20, worin $R_L$ mit einem oder mehreren Substituenten substituiert ist, die aus der aus Folgendem bestehenden Gruppe ausgewählt sind: $C_{1-7}$-Alkyl, $C_{5-20}$-Aryl, $C_{3-20}$-Heterocyclyl, Halogen, Hydroxy, Ether, Nitro, Cyano, Carbonylgruppen, Amino, Sulfonamido, Acylamido, Ureido, Acyloxy, Thiol, Thioether, Sulfoxid und Sulfon.

**22.** Verbindung nach Anspruch 21, worin $R_L$ mit einem aus der aus Acylamido, Ureido, Sulfonamido und Acyloxy bestehenden Gruppe ausgewählten Substituenten substituiert ist.

**23.** Verbindung der Formel:

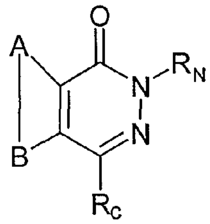

oder ein Isomer (d.h. eine geometrische, optische, enantiomere, diastereoisomere, epimere, stereoisomere, tautomere, konformere oder anomere Form), ein Salz, ein Solvat oder eine chemisch geschützte Form davon, worin:

A und B gemeinsam für einen kondensierten aromatischen Ring stehen, der gegebenenfalls mit einer aus Halogen, Nitro, Hydroxy, Ether, Thiol, Thioether, Amino, $C_{1-7}$-Alkyl, $C_{3-20}$-Heterocyclyl und $C_{5-20}$-Aryl ausgewählten Substituentengruppe substituiert ist;

$R_C$ -$CH_2$-$R_L$ ist;
$R_L$ Phenyl ist, das gegebenenfalls mit einem aus der aus $C_{3-20}$-Heterocyclyl, Amido, Ureido, Acyloxy, Sulfonamido und Acyloxy bestehenden Gruppe ausgewählten Substituenten substituiert ist und gegebenenfalls mit einer aus $C_{1-7}$-Alkyl, $C_{5-20}$-Aryl, $C_{3-20}$-Heterocyclyl, Halogen, Hydroxy, Ether, Nitro, Cyano, Acyl, Carboxy, Ester, Amido, Amino, Sulfonamido, Acylamido, Ureido, Acyloxy, Thiol, Thioether, Sulfoxid und Sulfon ausgewählten Gruppe weiter substituiert ist; und
$R_N$ Wasserstoff ist.

## Revendications

1. Utilisation d'un composé de la formule:

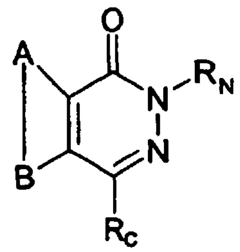

ou d'isomères, (c'est-à-dire des formes géométriques, optiques, énantiomères, diastéréomères, épimères, stéréoisomères, tautomères, conformationnelles ou anomères), sels, solvates et des formes chimiquement protégées de celui-ci, dans la préparation d'un médicament pour inhiber l'activité de PARP, où:

A et B ensemble représentent un cycle aromatique fusionné, optionnellement substitué par un ou plusieurs groupes substituants sélectionnés parmi halo, nitro, hydroxy, éther, thiol, thioéther, amino, alkyle$C_{1-7}$, hétérocyclyle $C_{3-20}$ et aryle$C_{5-20}$;
Rc est -$CH_2$-$R_L$, où $R_L$ est un groupe aryle$C_{5-20}$, optionnellement substitué par un ou plusieurs groupes substituants sélectionnés parmi alkyle$C_{1-7}$, aryle$C_{5-20}$, hétérocyclyle$C_{3-20}$, halo, hydroxy, éther, nitro, cyano, acyle, carboxy, ester, amido, amino, sulfonamido, acylamido, uréido, acyloxy, thiol, thioéther, sulfoxyde et sulfone; et
$R_N$ est hydrogène.

2. Utilisation d'un composé de la formule:

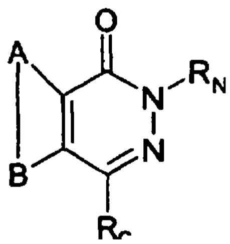

ou d'isomères, c'est-à-dire formes géométriques, optiques, énantiomères, diastéréomères, épimères, stéréoisomères, tautomères, conformationnelles ou anomères, sels, solvates et de formes chimiquement protégées de celuici, dans la préparation d'un médicament pour utilisation comme un adjuvant dans la thérapie du cancer, où:
A et B ensemble représentent un cycle aromatique fusionné, optionnellement substitué par un ou plusieurs groupes substituants sélectionnés parmi halo, nitro, hydroxy, éther, thiol, thioéther, amino, alkyle$C_{1-7}$, hétérocyclyle$C_{3-20}$ et aryle$C_{5-20}$;
$R_C$ est -$CH_2$-$R_L$, où $R_L$ est un groupe aryle$C_{5-20}$, optionnnellement substitué par un ou plusieurs groupes substituants sélectionnés parmi alkyle$C_{1-7}$, aryle$C_{5-20}$, hétérocyclyle$C_{3-20}$, halo, hydroxy, éther, nitro, cyano, acyle, carboxy, ester, amido, amino, sulfonamido, acylamido, uréido, acyloxy, thiol, thioéther, sulfoxyde et sulfone; et

$R_N$ est hydrogène.

3. Utilisation selon la revendication 2, où l'adjuvant est pour utilisation en combinaison avec un rayonnement ionisant.

4. Utilisation selon la revendication 2, où l'adjuvant est pour utilisation en combinaison avec des agents chimiothérapeutiques.

5. Utilisation selon l'une quelconque des revendications 1 à 4, où le ou les cycles aromatiques fusionnés représentés par -A-B- consistent en atomes de cycle de carbone uniquement.

6. Utilisation selon la revendication 5, où le cycle aromatique fusionné représenté par -A-B- est benzène.

7. Utilisation selon la revendication 5 ou la revendication 6, où lesdits cycles ne sont pas substitués.

8. Utilisation selon l'une quelconque des revendications précédentes, où $R_L$ est un cycle de benzène.

9. Utilisation selon la revendication 8, où $R_L$ est substitué par un ou plusieurs substituants sélectionnés dans le groupe consistant en: alkyle$C_{1-7}$; aryle$C_{5-20}$; hétérocyclyle$C_{3-20}$; halo; hydroxy; éther; nitro; cyano; groupes carbonyles; amino; sulfonamido; acylamido; uréido; acyloxy; thiol; thioéther; sulfoxyde et sulfone.

10. Utilisation selon la revendication 9, où $R_L$ est substitué par un substituant sélectionné dans le groupe consistant en: acylamido, uréido, sulfonamido et acyloxy.

11. Composition pharmaceutique comprenant un composé de la formule:

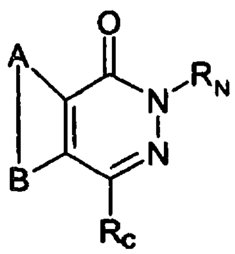

ou d'isomères, c'est-à-dire des formes géométriques, optiques, énantiomères, diastéréomères, épimères, stéréoisomères, tautomères, conformationnelles ou anomères, sels, solvates et des formes chimiquement protégées de celui-ci où:
A et B ensemble représentent un cycle aromatique fusionné, optionnellement substitué par un ou plusieurs groupes substituants sélectionnés parmi halo, nitro, hydroxy, éther, thiol, thioéther, amino, alkyle$C_{1-7}$, hétérocyclyle$C_{3-20}$ et aryle$C_{5-20}$;
$R_C$ est $-CH_2-R_L$;
$R_L$ est phényle optionnellement substitué par un ou plusieurs groupes substituants sélectionnés parmi alkyle$C_{1-7}$, aryle $C_{5-20}$, hétérocyclyle$C_{3-20}$, halo, hydroxy, éther, nitro, cyano, carboxy, ester, amido, amino, sulfonamido, acylamido, uréido, acyloxy, thiol, thioéther, sulfoxyde et sulfone; et $R_N$ est hydrogène,
et un support ou diluant pharmaceutiquement acceptable.

12. Composition pharmaceutique selon la revendication 11, dans laquelle le ou les cycles aromatiques fusionnés représentés par -A-B- consistent en atomes de cycle de carbone uniquement.

13. Composition pharmaceutique selon la revendication 12, dans laquelle le cycle aromatique fusionné représenté par -A-B- est benzène.

14. Composition pharmaceutique selon l'une quelconque de la revendication 12 ou de la revendication 13, où lesdits cycles ne sont pas substitués.

15. Composition pharmaceutique selon l'une quelconque des revendications 11 à 14, dans laquelle $R_L$ est substitué

par un ou plusieurs substituants sélectionnés dans le groupe consistant en: alkyle$C_{1-7}$; aryle$C_{5-20}$; hétérocyclyle $C_{3-20}$; halo; hydroxy; éther; nitro; cyano; groupes carbonyles; amino; sulfonamido; acylamido; uréido; acyloxy; thiol; thioéther; sulfoxyde et sulfone.

**16.** Composition pharmaceutique selon la revendication 15, où $R_L$ est substitué par un substituant sélectionné dans le groupe consistant en: acylamido, uréido, sulfonamido et acyloxy.

**17.** Composé selon la formule:

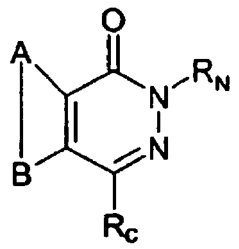

où isomères (c'est-à-dire des formes géométriques, optiques, énantiomères, diastéréomères, épimères, stéréoisomères, tautomères, conformationnelles ou anomères, sels, solvates et des formes chimiquement protégées de celui-ci où:

A et B ensemble représentent un cycle aromatique fusionné, optionnellement substitué par un ou plusieurs groupes substituants sélectionnés parmi halo, nitro, hydroxy, éther, thiol, thioéther, amino, alkyle$C_{1-7}$, hétérocyclyle$C_{3-20}$ et aryle$C_{5-20}$;
$R_C$ est $-CH_2-R_L$;
$R_L$ est phényle optionnellement substitué par un ou plusieurs groupes substituants sélectionnés parmi alkyle$C_{1-7}$, aryle $C_{5-20}$, hétérocyclyle$C_{3-20}$, halo, hydroxy, éther, nitro, cyano, carboxy, ester, amido, amino, sulfonamido, acylamido, uréido, acyloxy, thiol, thioéther, sulfoxyde et sulfone; et $R_N$ est hydrogène,
pour utilisation dans une méthode de traitement du corps humain ou animal.

**18.** Composé selon la revendication 17, où le ou les cycles aromatiques fusionnés représentés par -A-B-consistent en atomes de cycle de carbone uniquement.

**19.** Composé selon la revendication 18, où le cycle aromatique fusionné représenté par -A-B- est benzène.

**20.** Composé selon l'une quelconque des revendications 18 ou 19, où lesdits cycles ne sont pas substitués.

**21.** Composé selon l'une quelconque des revendications 17 à 20, où $R_L$ est substitué par un ou plusieurs substituants sélectionnés dans le groupe consistant en:

alkyle$C_{1-7}$; aryle$C_{5-20}$; hétérocyclyle$C_{3-20}$; halo; hydroxy; éther; nitro; cyano; groupes carbonyles; amino; sulfonamido; acylamido; uréido; acyloxy; thiol; thioéther, sulfoxyde et sulfone.

**22.** Composé selon la revendication 21, où $R_L$ est substitué par un substituant sélectionné dans le groupe consistant en: acylamido, uréido, sulfonamido et acyloxy.

**23.** Composé de la formule:

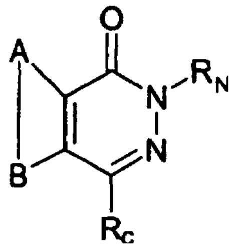

ou un isomère, c'est-à-dire forme géométrique, optique, énantiomère, diastéréomère, épimère, stéréoisomère, tautomère, conformationnelle ou anomère, sel, solvate et des formes chimiquement protégées de celui-ci, où:
A et B ensemble représentent un cycle aromatique fusionné, optionnellement substitué par un groupe substituant sélectionné parmi halo, nitro, hydroxy, éther, thiol, thioéther, amino, alkyle$C_{1-7}$, hétérocyclyle$C_{3-20}$ et aryle$C_{5-20}$;
$R_C$ est $-CH_2-R_L$;
$R_L$ est phényle substitué par un substituant sélectionné dans le groupe consistant en: hétérocyclyle$C_{3-20}$; amido; uréido; sulfonamido; et acyloxy et optionnellement substitué en outre par un groupe sélectionné parmi alkyle $C_{1-7}$, aryle$C_{5-20}$, hétérocyclyle$C_{3-20}$, halo, hydroxy, éther, nitro, cyano, carboxy, ester, amido, amino, sulfonamide, acylamido, uréido, acyloxy, thiol, thioéther, sulfoxyde et sulfone; et $R_N$ est hydrogène.

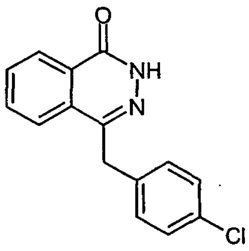

126

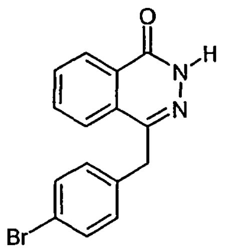

129

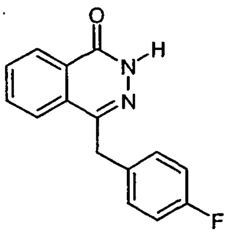

132

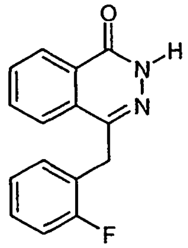

141

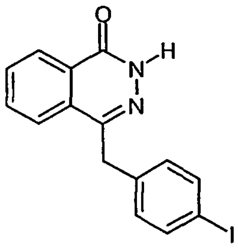

151

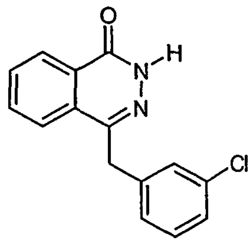

186

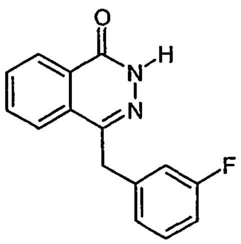

191

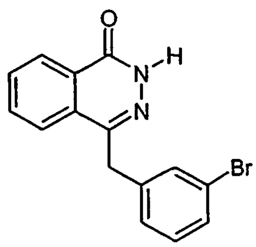

211

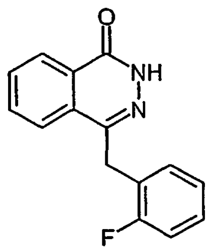

248

Figure 1

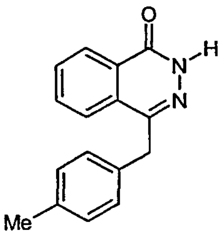

139

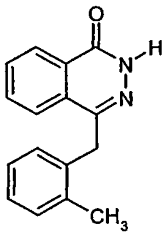

163

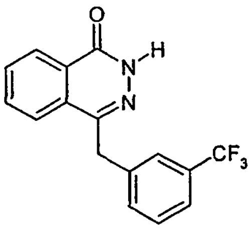

192

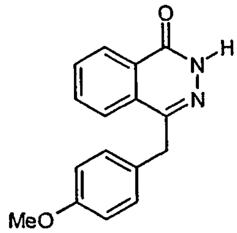

138

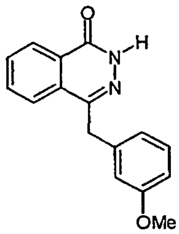

142

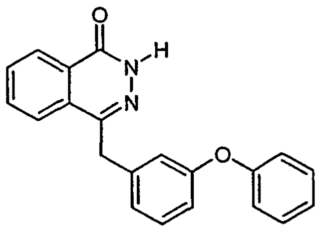

193

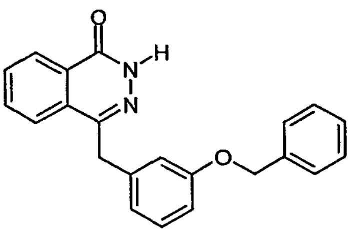

194

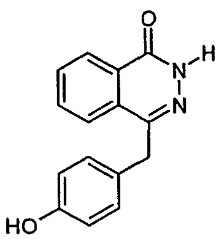

164

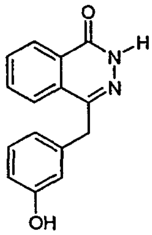

165

Figure 2

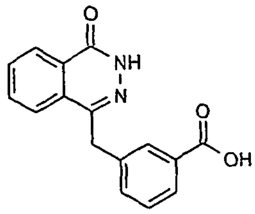

276

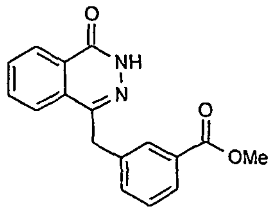

277

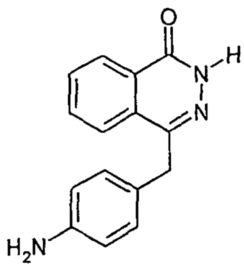

159

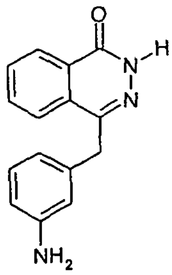

160

Figure 3

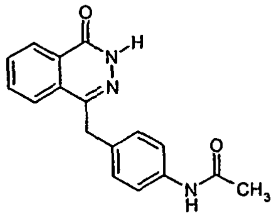

166

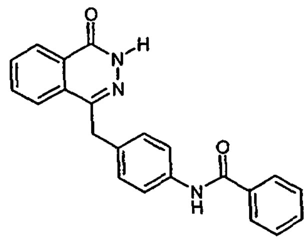

167

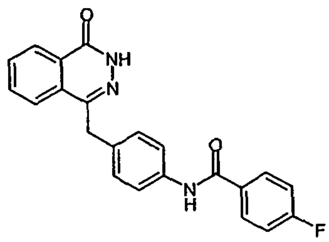

227

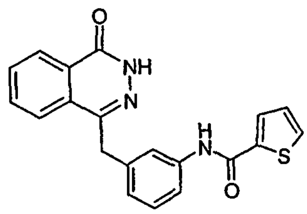

169

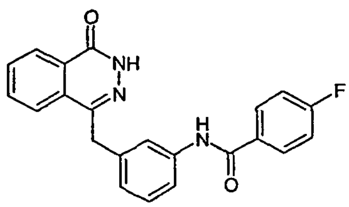

170

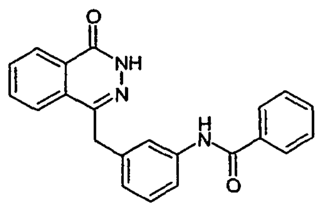

171

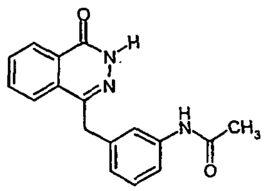

172

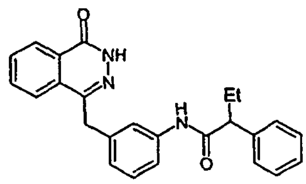

233

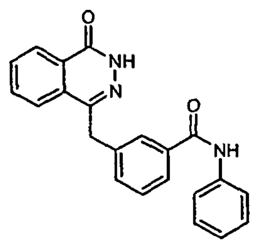

278

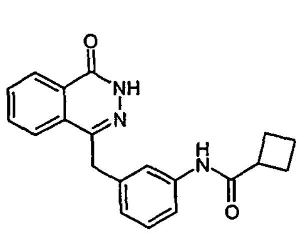

640

Figure 4

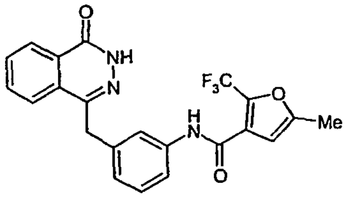

641

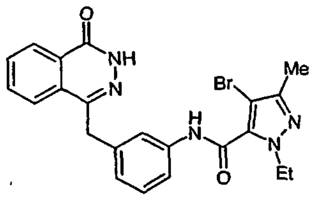

642

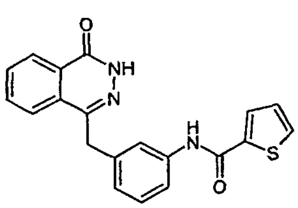

643

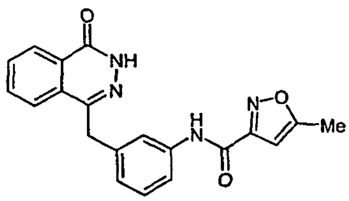

644

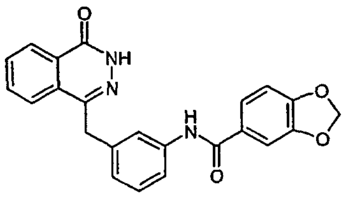

645

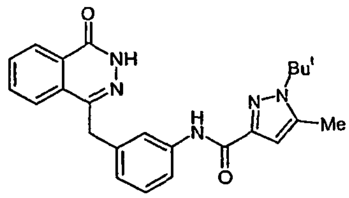

646

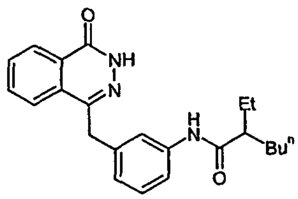

647

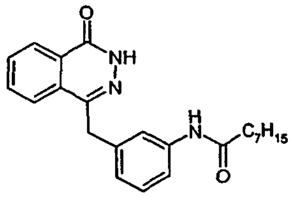

648

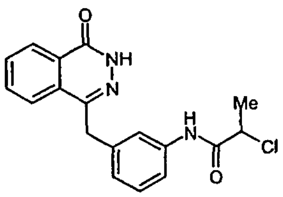

649

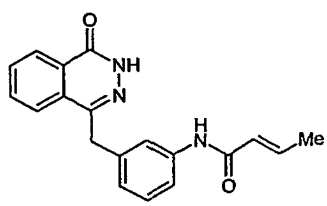

650

Figure 5

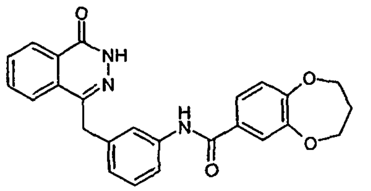

651

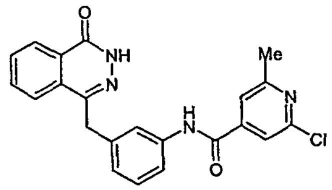

652

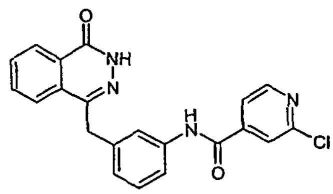

653

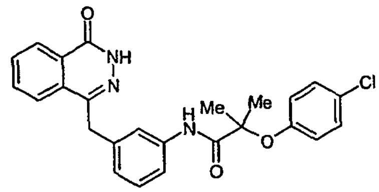

654

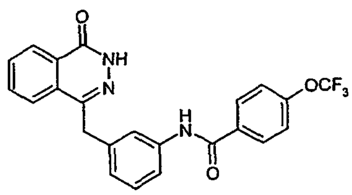

655

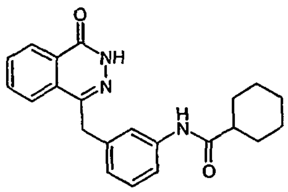

656

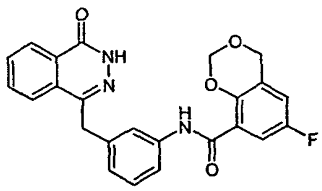

657

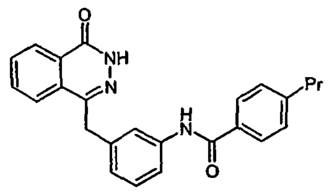

658

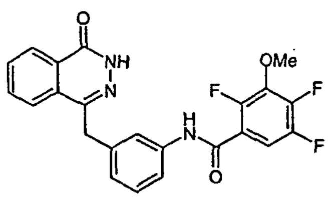

659

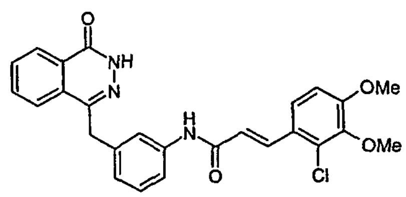

667

Figure 6

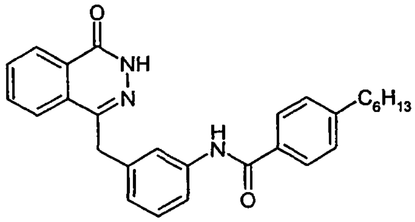

668

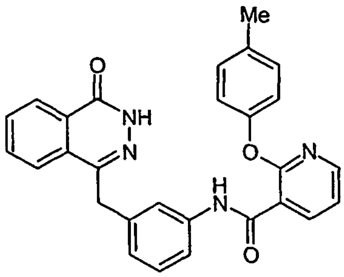

669

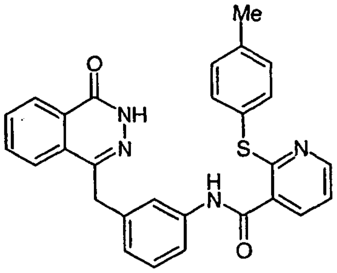

670

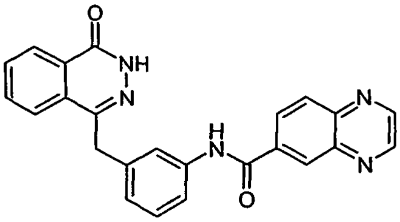

671

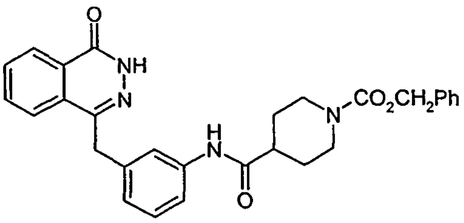

672

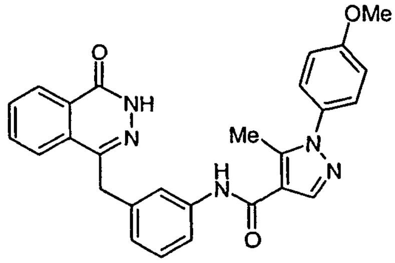

673

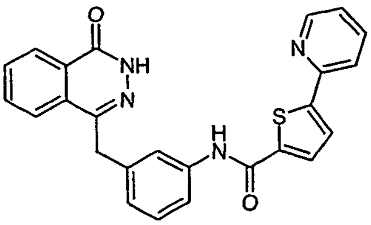

674

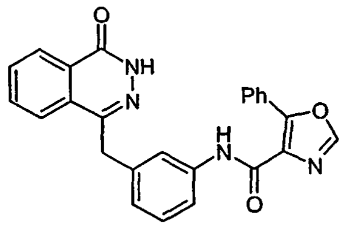

675

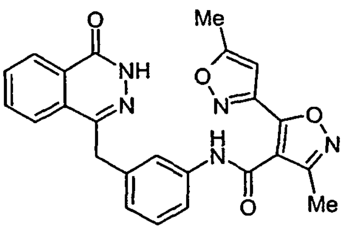

676

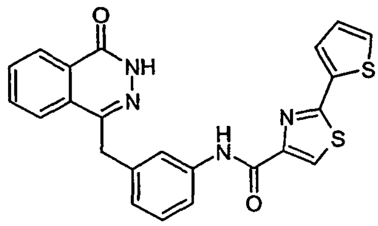

677

Figure 7

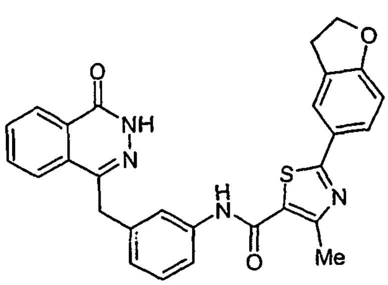

678

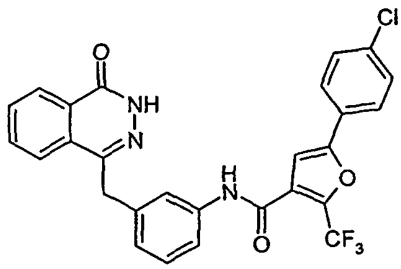

679

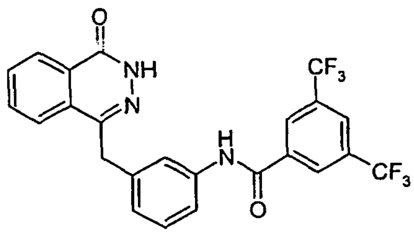

680

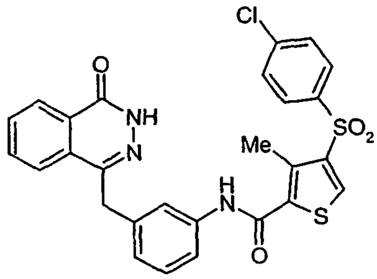

681

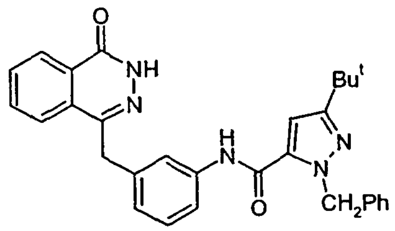

682

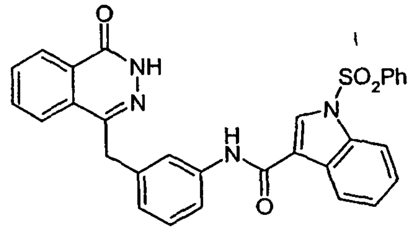

683

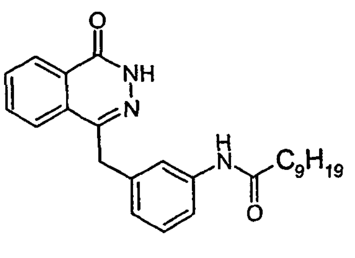

684

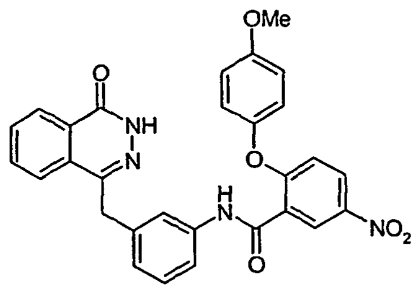

685

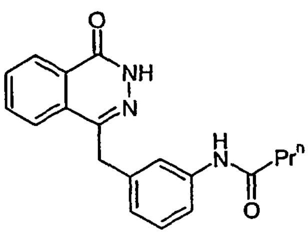

686

Figure 8

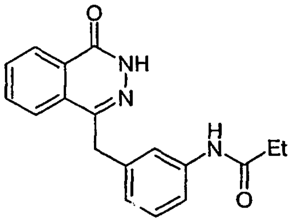

687

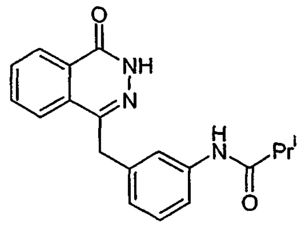

688

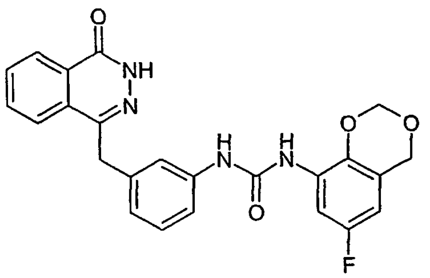

691

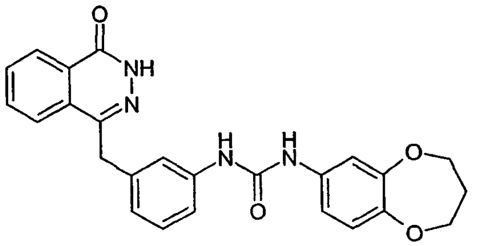

692

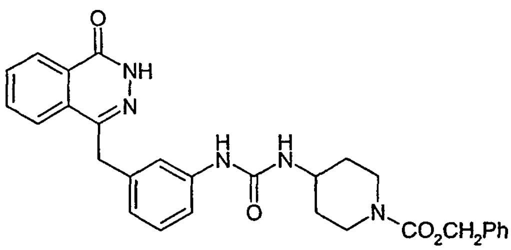

693

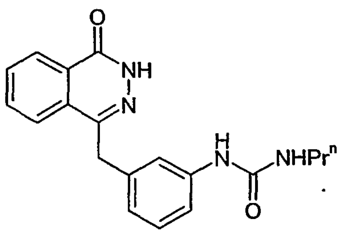

694

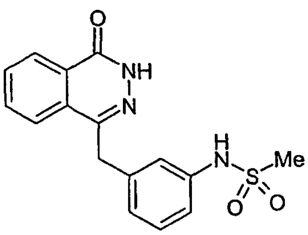

697

Figure 9

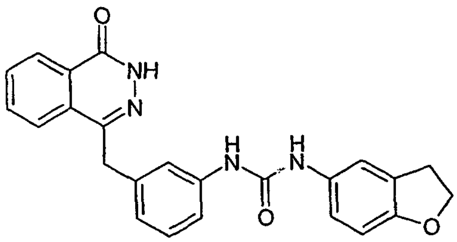

698

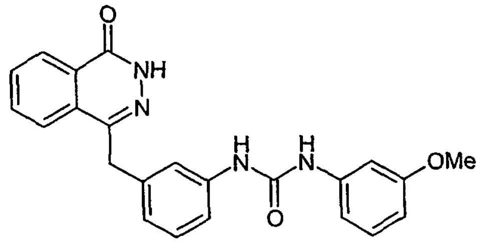

699

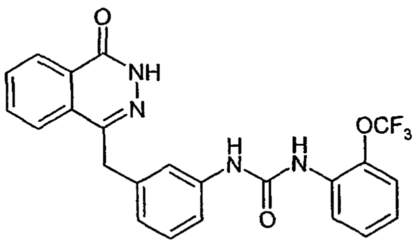

700

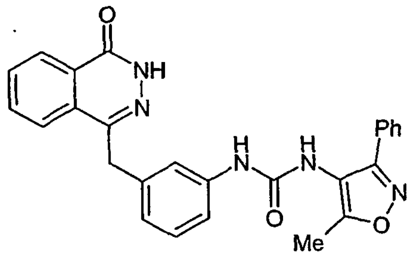

701

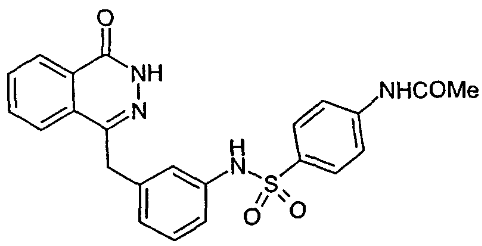

702

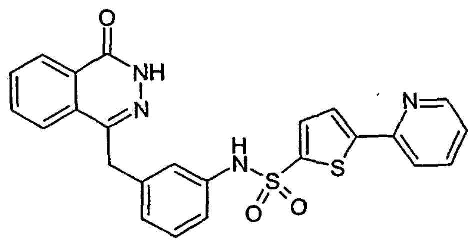

703

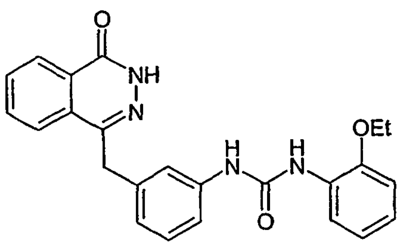

704

Figure 10

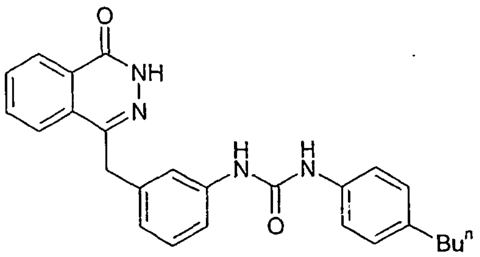

705

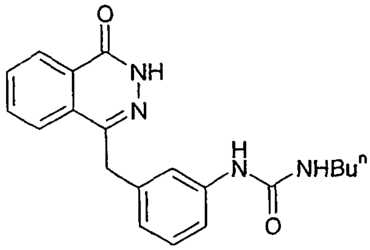

706

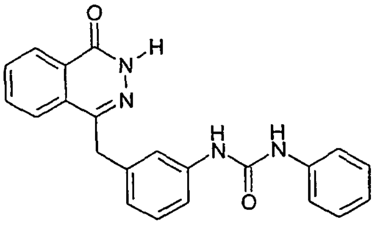

215

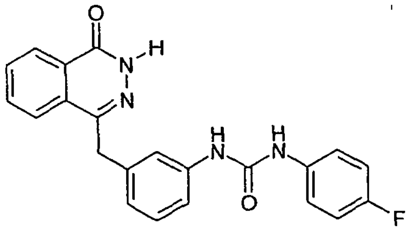

216

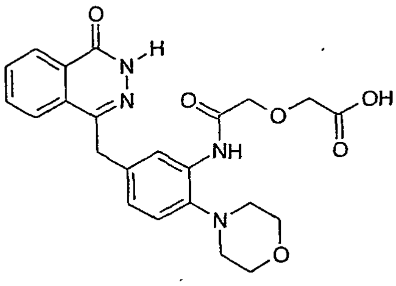

206

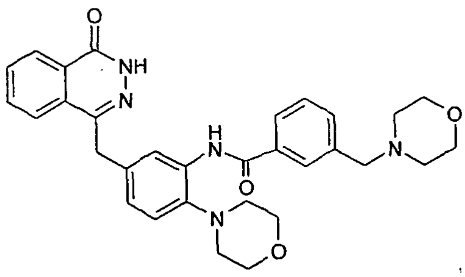

253

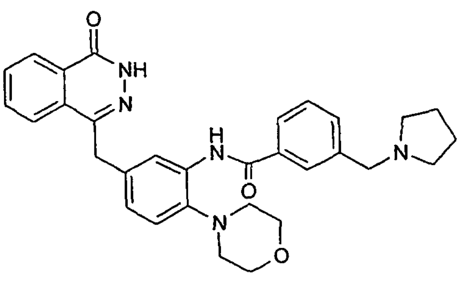

254

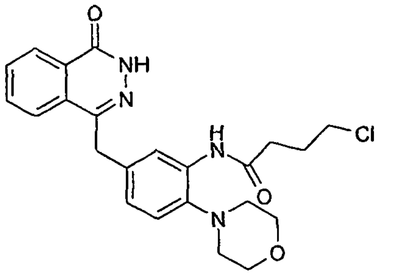

265

Figure 11

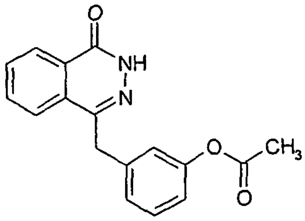

179

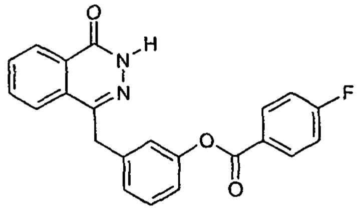

212

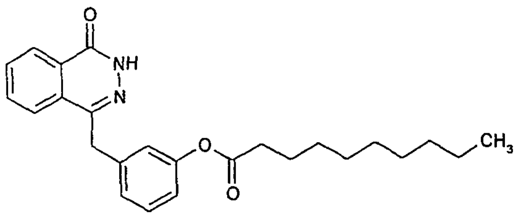

289

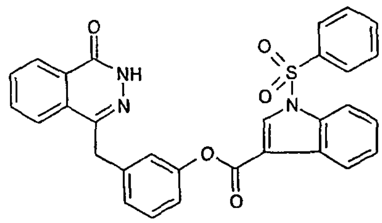

290

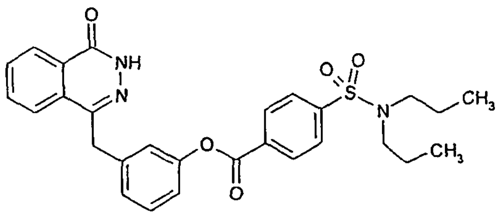

291

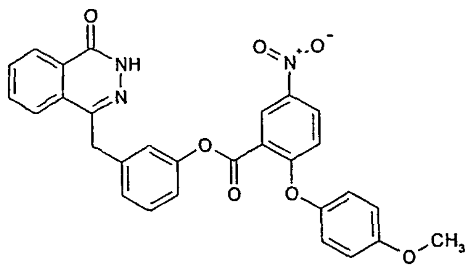

292

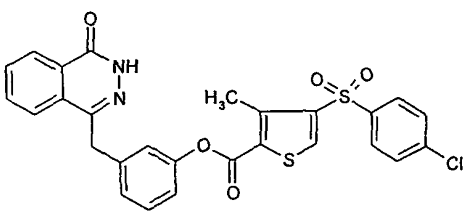

293

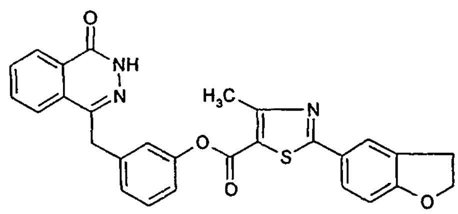

294

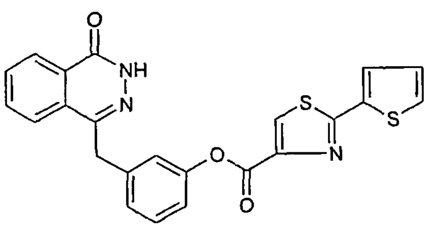

295

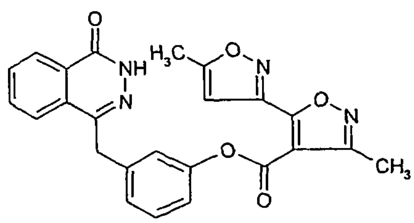

296

Figure 12

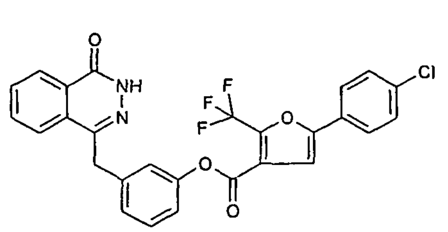

298

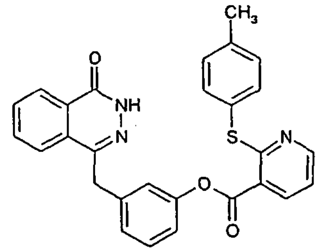

299

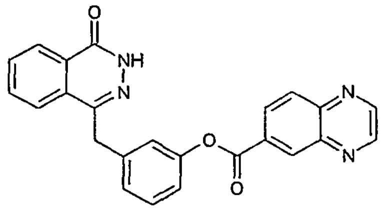

300

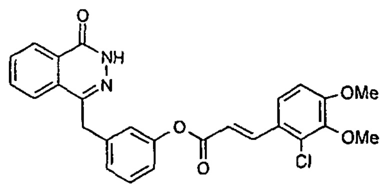

601

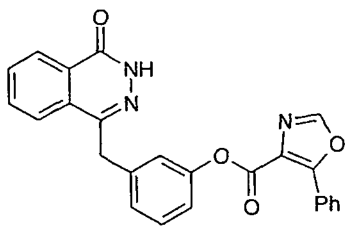

602

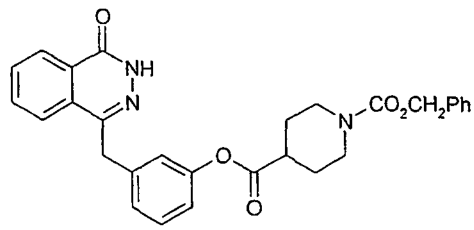

603

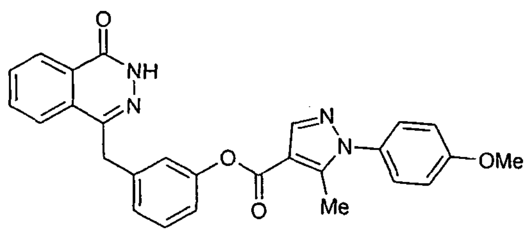

604

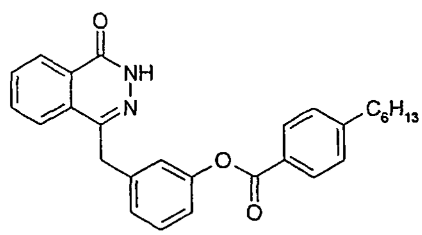

605

Figure 13

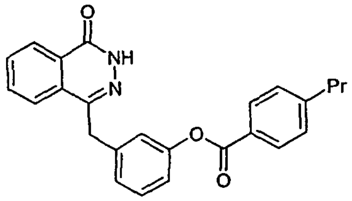

607

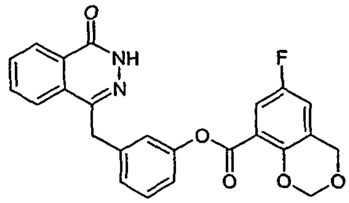

608

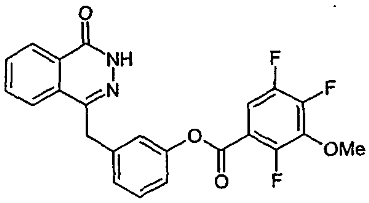

609

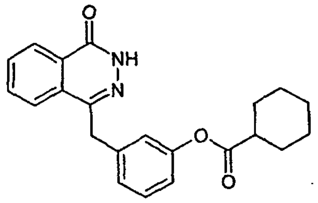

610

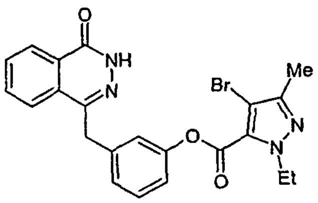

611

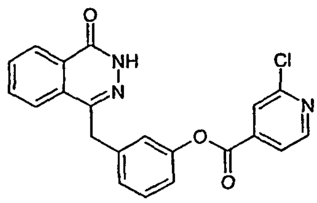

612

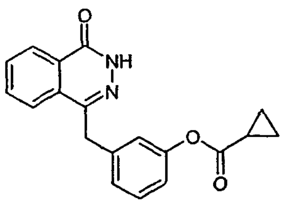

613

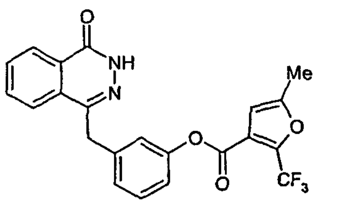

614

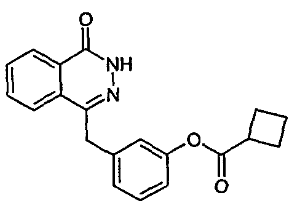

615

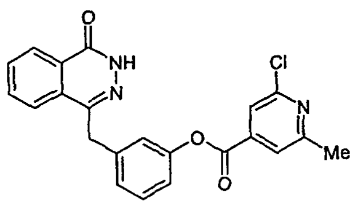

616

Figure 14

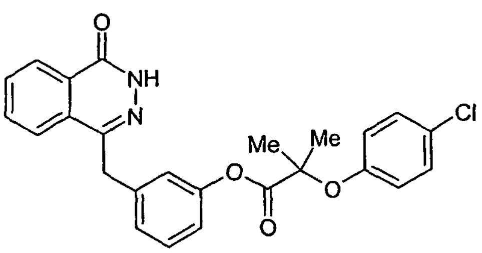

617

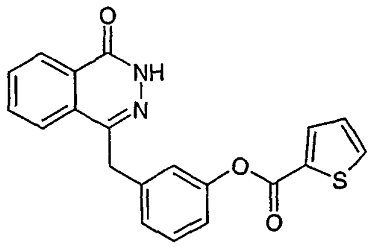

618

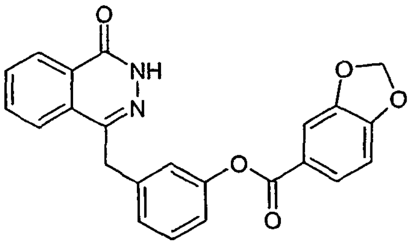

619

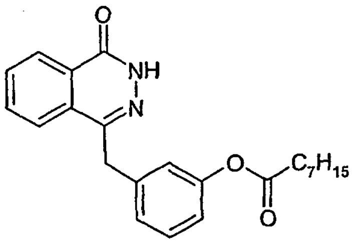

620

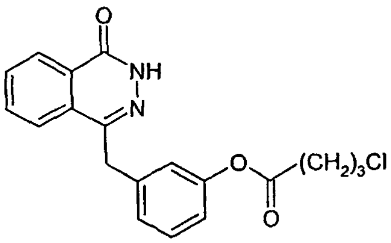

621

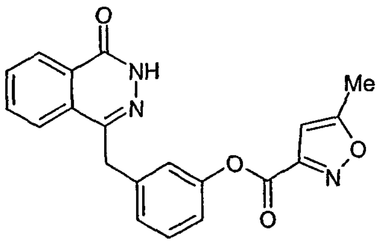

622

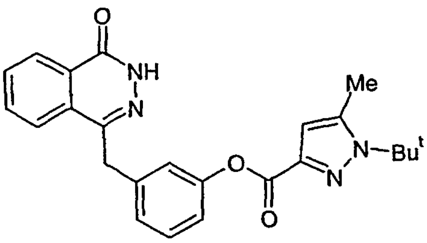

623

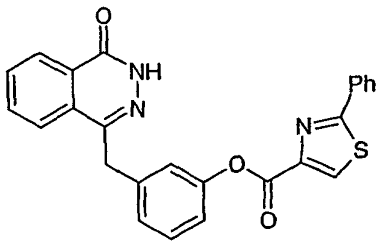

624

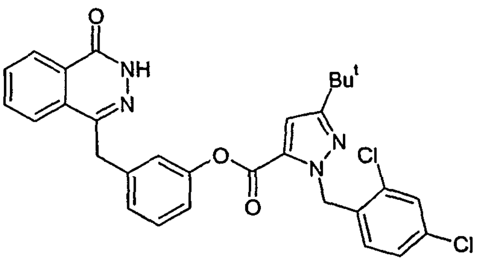

625

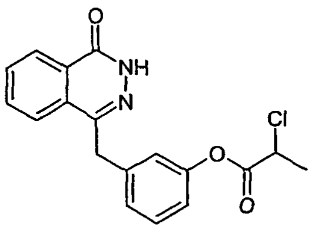

626

Figure 15

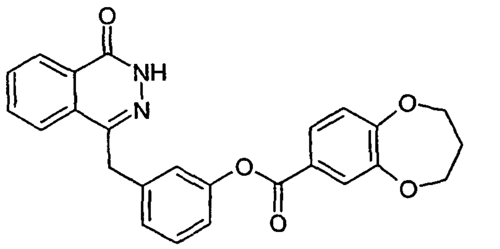

627

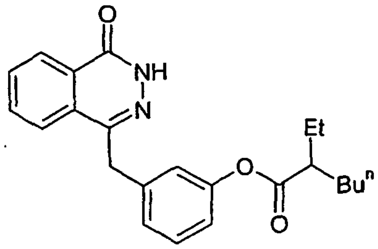

628

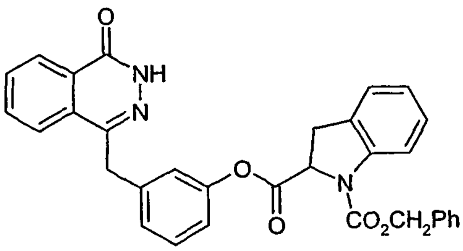

629

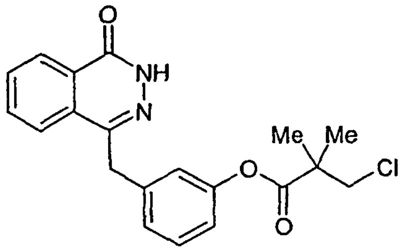

630

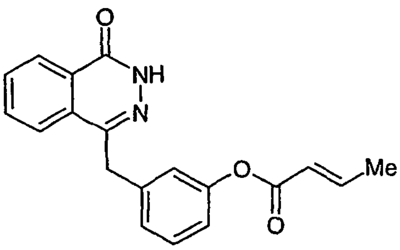

631

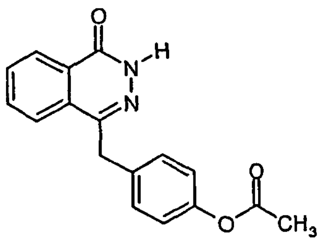

213

Figure 16

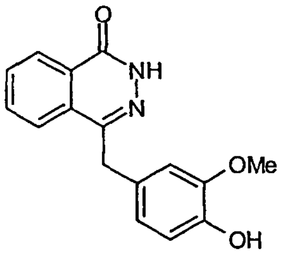

239

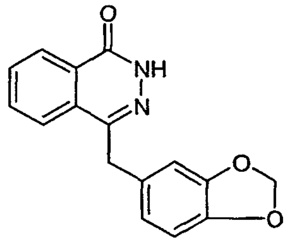

180

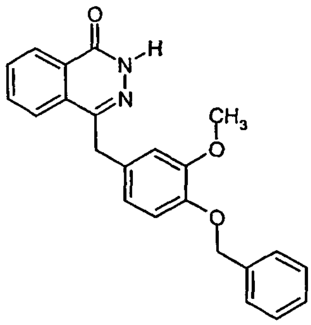

222

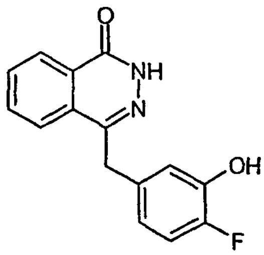

247

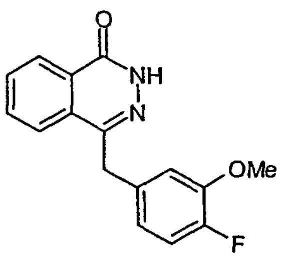

241

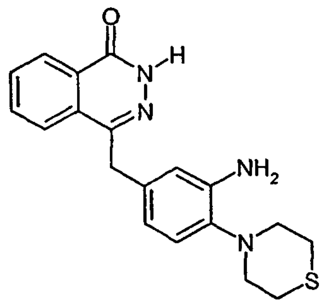

198

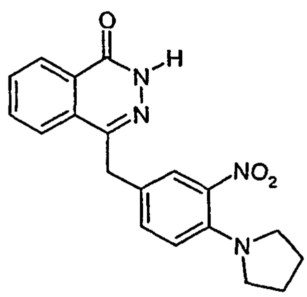

204

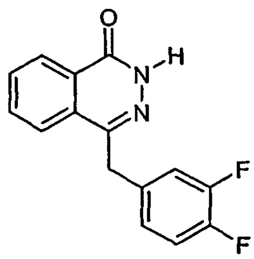

202

Figure 17

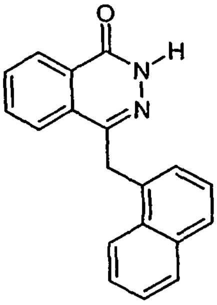

131

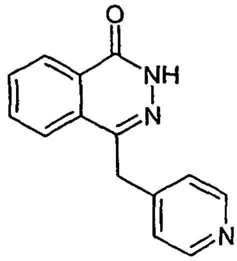

177

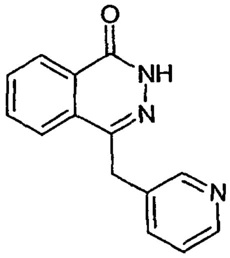

178

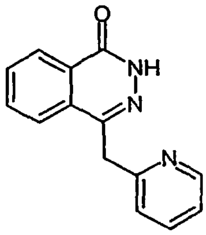

249

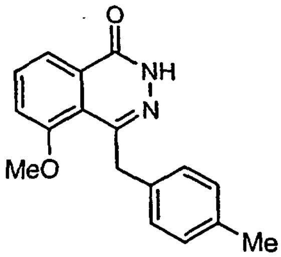

279

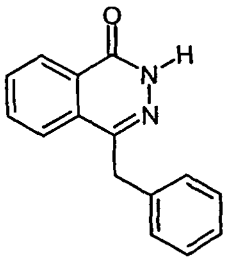

145

Figure 18

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5032617 A
- US 5215738 A
- US 5041653 A
- WO 9118591 A
- US 5874444 A


### Non-patent literature cited in the description


- **D'Amours et al.** *Biochem. J.,* 1999, vol. 342, 249-268
- **d'Adda di Fagagna et al.** *Nature Gen.,* 1999, vol. 23 (1), 76-80
- **Althaus, F.R. ; Richter, C.** ADP-Ribosylation of Proteins: Enzymology and Biological Significance. Springer-Verlag, 1987
- **Rhun et al.** *Biochem. Biophys. Res. Commun.,* 1998, vol. 245, 1-10
- **Miwa et al.** *Arch. Biochem. Biophys.,* 1977, vol. 181, 313-321
- **Burzio et al.** *Proc. Soc. Exp. Bioi. Med.,* 1975, vol. 149, 933-938
- **Hirai et al.** *Cancer Res.,* 1983, vol. 43, 3441-3446
- **Durkacz et al.** *Nature,* 1980, vol. 283, 593-596
- **Berger, N.A.** *Radiation Research,* 1985, vol. 101, 4-14
- **Ben-Hur et al.** *British Journal of Cancer,* 1984, vol. 49 (VI), 34-42
- **Schlicker et al.** *Int. J. Radiat. Bioi.,* 1999, vol. 75, 91-100
- **Wang et al.** *Genes Dev.,* 1995, vol. 9, 509-520
- **Menissier de Murcia et al.** *Proc. Natl. Acad. Sci. USA,* 1997, vol. 94, 7303-7307
- **Cantoni et al.** *Biochim. Biophys. Acta,* 1989, vol. 1014, 1-7
- **Szabo et al.** *J. Clin.Invest.,* 1997, vol. 100, 723-735
- **Cosi et al.** *J. Neurosci. Res.,* 1994, vol. 39, 38-46
- **Said et al.** *Proc. Natl. Acad. Sci. U.S.A.,* 1996, vol. 93, 4688-4692
- **Liaudet et al.** *Proc. Natl. Acad. Sci. U.S.A.,* 2000, vol. 97 (3), 10203-10208
- **Gaken et al.** *J. Virology,* 1996, vol. 70 (6), 3992-4000
- **Rattan ; Clark.** *Biochem. Biophys. Res. Comm.,* 1994, vol. 201 (2), 665-672
- **Berge et al.** Pharmaceutically Acceptable Salts. *J. Pharm. Sci.,* 1977, vol. 66, 1-19
- **T. Green ; P. Wuts.** Protective Groups in Organic Synthesis. Wiley, 1991
- **Yamaguchi et al.** *J. Med. Chem.,* 1993, vol. 36, 4502-4068
- Remington's Pharmaceutical Sciences. Mack Publishing Company, 1990
- **Skehan, P. et al.** *J. Natl. Cancer Inst.,* 1990, vol. 82, 1107-1112